(19) Europäisches Patentamt
European Patent Office
Office européen des brevets

(11) EP 3 703 756 B1

(12) # EUROPEAN PATENT SPECIFICATION

(45) Date of publication and mention of the grant of the patent:
**20.04.2022 Bulletin 2022/16**

(21) Application number: **18800066.5**

(22) Date of filing: **02.11.2018**

(51) International Patent Classification (IPC):
***A61K 31/43*** (2006.01) ***A61K 31/431*** (2006.01)
***A61K 31/366*** (2006.01) ***A61K 31/22*** (2006.01)
***A61K 45/06*** (2006.01) ***A61P 31/04*** (2006.01)

(52) Cooperative Patent Classification (CPC):
(C-Sets available)
**A61K 45/06; A61K 31/22; A61K 31/366; A61K 31/43; A61K 31/431; A61P 31/04;**
Y02A 50/30 (Cont.)

(86) International application number:
**PCT/EP2018/080057**

(87) International publication number:
**WO 2019/086633 (09.05.2019 Gazette 2019/19)**

(54) **USE OF STATINS IN OVERCOMING RESISTANCE TO BETA-LACTAM ANTIBIOTICS IN BACTERIAL SPECIES SYNTHETIZING ISOPRENOIDS USING THE MEVALONATE SYNTHETIC PATHWAY**

VERWENDUNG VON STATINEN BEI DER ÜBERWINDUNG DER RESISTENZ GEGENÜBER BETA-LAKTAM-ANTIBIOTIKA IN BAKTERIENSPEZIES, DIE MITHILFE DES MEVALONATSYNTHESEWEGS ISOPRENOIDE SYNTHETISIEREN

UTILISATION DE STATINES POUR SURMONTER LA RÉSISTANCE AUX ANTIBIOTIQUES BÊTALACTAMINES DANS DES ESPÈCES BACTÉRIENNES SYNTHÉTISANT LES ISOPRÉNOÏDES À L'AIDE DE LA VOIE DE SYNTHÈSE DU MÉVALONATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.11.2017 EP 17382734**

(43) Date of publication of application:
**09.09.2020 Bulletin 2020/37**

(73) Proprietors:
- **Consejo Superior de Investigaciones Científicas (CSIC)**
  **28006 Madrid (ES)**
- **University of Würzburg**
  **97070 Würzburg (DE)**

(72) Inventors:
- **LÓPEZ SERRANO, Daniel**
  **28049 Madrid (ES)**
- **KOCH, Gudrun**
  **97080 Würzburg (DE)**

(74) Representative: **Hoffmann Eitle**
**Hoffmann Eitle S.L.U.**
**Paseo de la Castellana 140, 3a planta**
**Edificio LIMA**
**28046 Madrid (ES)**

(56) References cited:
- **AISLING R. CAFFREY ET AL: "Evidence To Support Continuation of Statin Therapy in Patients with Staphylococcus aureus Bacteremia", ANTIMICROBIAL AGENTS AND CHEMOTHERAPY, vol. 61, no. 3, 1 March 2017 (2017-03-01), XP55545080, US ISSN: 0066-4804, DOI: 10.1128/AAC.02228-16**
- **EMMA HENNESSY ET AL: "Is There Potential for Repurposing Statins as Novel Antimicrobials?", ANTIMICROBIAL AGENTS AND CHEMOTHERAPY, vol. 60, no. 9, 20 June 2016 (2016-06-20), pages 5111-5121, XP055465158, ISSN: 0066-4804, DOI: 10.1128/AAC.00192-16**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SHANKAR THANGAMANI ET AL: "Exploring simvastatin, an antihyperlipidemic drug, as a potential topical antibacterial agent", SCIENTIFIC REPORTS, vol. 5, no. 1, 10 November 2015 (2015-11-10), XP055465162, DOI: 10.1038/srep16407**
- **CATHERINE LIU ET AL: "Clinical Practice Guidelines by the Infectious Diseases Society of America for the Treatment of Methicillin-Resistant Staphylococcus aureus Infections in Adults and Children", CLINICAL INFECTIOUS DISEASES, vol. 52, no. 3, 1 February 2011 (2011-02-01), pages e18-e55, XP055465163, US ISSN: 1058-4838, DOI: 10.1093/cid/ciq146**


(52) Cooperative Patent Classification (CPC): (Cont.)

C-Sets
**A61K 31/22;**
**A61K 31/366;**
**A61K 31/43;**
**A61K 31/431**

**Description**

## FIELD OF THE INVENTION

The present invention relates to the fields of pharmacy and microbiology, more specifically to the field of bacterial infections and antibiotic therapy, especially to the field of antibiotic resistance.

In particular, the invention refers to a beta-lactamase resistant penicillin for use in a method of treating a bacterial infection in a subject in need thereof wherein said bacterial infection is caused by a bacterial population, i.e. Enterococci, Pneumococci or Staphylococci, resistant to said beta-lactamase resistant penicillin. In a particular aspect, the invention relates to a beta-lactamase resistant penicillin for use in a method of treating a bacterial infection in a subject in need thereof wherein said bacterial infection is caused by a bacterial population i.e. Enterococci, Pneumococci, or Staphylococci, resistant to said beta-lactamase resistant penicillin, wherein said subject is further treated before or simultaneously to said beta-lactam antibiotic, with a statin of formula (I) or (Ia), wherein said statin is for use in reducing, mitigating or reversing resistance induced by low affinity penicillin-binding proteins (PBPs) to said beta-lactamase resistant penicillin in said resistant bacterial population.

## BACKGROUND OF THE INVENTION

Antibiotics are medicines used to prevent and treat bacterial infections. The incidence of the multiple antibiotic resistance of bacteria which cause infections in hospitals/intensive care units is increasing, with many multiple resistent strains having been described, including methicillin-resistant and methicillin-vancomycin-resistant *Staphylococcus aureus;* vancomycin-resistant enterococci, such as *Enterococcus faecalis and Enterococcus faecium;* penicillin-resistant *Streptococcus pneumoniae,* and cephalosporin and quinolone resistant gram-negative rods (coliforms), such as E. *coli, Salmonella* species, *Klebsiella pneumoniae,* Pseudomonas species and Enterobacter species. Several international reports have highlighted the potential problems associated with the emergence of resistances in many areas of medicine and also outlined the difficulties in the management of patients with infections caused by these microorganisms.

Inhibition of resistance mechanisms can restore the activities of anti-infective agents that are substrates for these mechanisms. Accordingly, there is an urgent need for drugs that can inhibit or circumvent the resistance mechanisms and improve the effectiveness of the currently available anti-infective agents.

*Staphylococcus aureus* attracts considerable attention of the scientific community, as it causes hard-to-treat hospital-associated infections due to its capacity to overcome antibiotic treatments. S. *aureus* acquires resistance to β-lactam antibiotics such as methicillin (methicillin-resistant S. *aureus;* MRSA) (Kreiswirth et al., 1993) through expression of a low-affinity penicillin-binding protein (PBP2a) that acts cooperatively with the general penicillin-binding protein PBP2 (Fishovitz et al., 2014; Pinho et al., 2001a).

Prokaryotic membranes have been reported to compartmentalize diverse cell processes in raft-like regions termed functional membrane microdomains (FMM), similar to their eukaryotic counterparts (LaRocca et al., 2013; Lopez and Kolter, 2010). FMM formation in bacteria involves the biosynthesis and aggregation of still-unknown isoprenoid membrane lipids (Feng et al., 2014; Lopez and Kolter, 2010) and their co-localization with flotillin-homolog proteins (Donovan and Bramkamp, 2009; Lopez and Kolter, 2010). Bacterial flotillins probably recruit protein cargo to FMM to facilitate protein interaction and oligomerization (Bach and Bramkamp, 2013; Koch et al., 2017; Schneider et al., 2015), similar to eukaryotic flotillins. Flotillin-deficient strains have defects in biofilm formation in *Bacillus subtilis and Staphylococcus aureus* (Bach and Bramkamp, 2013; Koch et al., 2017; Schneider et al., 2015), virulence in *B. anthracis* (Somani et al., 2016) and *Campylobacter jejuni* (Heimesaat et al., 2014; Tareen et al., 2013), or thylakoid integrity in cyanobacteria (Bryan et al., 2011).

Despite this importance, the organization and biological significance of FMM are largely unknown. In contrast to traditional bacterial models, S. *aureus* expresses a single flotillin, FloA, and the biosynthesis pathway for isoprenoid membrane lipids is fairly well known (Marshall and Wilmoth, 1981; Pelz et al., 2005; Wieland et al., 1994), rendering a realistic model in which to undertake FMM organizational and functional studies.

Statins are anti-cholesterol drugs which have been described to exert their effect by inhibiting the enzyme class I 3-hydroxy-3-methyl-glutaryl-CoenzymeA(HMG-CoA) reductase in the mevalonate synthetic route leading to decreased synthesis of cholesterol and increased removal of low-density lipoprotein (LDL) circulating in the body.

There are several works exploring the use of statins as novel antimicrobials (Hennessy et al., 2016; Thangamani et al. 2015, Farmer et al., 2013). Some clinical studies detected a beneficial role of statins in microbial infections (Falagas et al., 2008; Liappis et al., 2001; Lopez-Cortes et al., 2013; Parihar et al., 2014). However, *in vitro* studies have also been published were the authors did not detect an antimicrobial effect of statins in specific conditions or bacterial species (Bergman et al., 2011; Wan et al., 2014).

SHANKAR THANGAMANI ET AL: "Exploring simvastatin, an antihyperlipidemic drug, as a potential topical antibacterial agent", SCIENTIFIC REPORTS, vol. 5, no. 1, 10 November 2015, discloses simvastatin which exhibits

broad spectrum antibacterial activity against Gram-positive bacteria, such as methicillin-resistant Staphylococcus aureus (MRSA), vancomycin-intermediate resistant Staphylococcus aureus (VISA), and vancomycin resistant Staphylococcus aureus (VRSA).

None of the prior art documents discloses the effect of a particular subgroup of statins which render susceptible to beta-lactamase resistant penicillins a bacterial population resistant thereto specifically in bacterial species, characterized by comprising isoprenoid lipids in its bacterial membrane and for synthetizing isoprenoids using the mevalonate synthetic pathway, such as MRSA which comprises staphyloxanthin and derivative isoprenoid lipids in its membrane. In particular, the inventors have shown that treatment with inhibitors of isoprenoid lipid synthesis, such as statins , reduces or reverses resistance to beta-lactamase resistant penicillins induced by low affinity penicillin-binding proteins (PBPs).

## SUMMARY OF THE INVENTION

The invention is defined by the claims. Any references in the description to methods of treatment refer to the compounds, pharmaceutical compositions and medicaments of the present invention for use in a method of treatment of the human or animal body by therapy.

The inventors have shown that FMM organization in MRSA requires lateral segregation of unphosphorylated carotenoids (staphyloxanthin and derivative isoprenoid lipids) in membrane microdomains. Flotillin preferentially binds to these lipids and oligomerizes in these domains, followed by attraction of membrane-associated multimeric complexes with which flotillin interacts and promotes efficient oligomerization. One of these proteins is PBP2a.

As above-mentioned, MRSA has acquired resistance to penicillins through expression of a low-affinity penicillin-binding protein (PBP2a) that acts cooperatively with the general penicillin-binding protein PBP2 (Fishovitz et al., 2014; Pinho et al., 2001a). More specifically, β-lactam antibiotics bind the PBP active site as substrate analogs (Zapun et al., 2008), to inhibit the PBP activity responsible for peptidoglycan synthesis during cell division. The PBP2a active site is located in a deep pocket inaccessible to β-lactam antibiotics (Otero et al., 2013) enabling MRSA strains to divide and proliferate in their presence (Kreiswirth et al., 1993).

Flotillin scaffold activity promotes PBP2a oligomerization; the inventors have now shown that perturbation of FMM assembly using a particular group of statins interferes with biosynthesis of FMM constituent lipids, which affects flotillin activity and ultimately, PBP2a oligomerization. This disables penicillin resistance in MRSA in a murine infection model, resulting in MRSA infections susceptible to penicillin antibiotic treatments. Accordingly, the inventors provide an innovative strategy to overcome resistance to beta-lactam antibiotics and in particular MRSA antibiotic resistance.

The inventors have identified a therapeutic effect only for a certain subgroup of statins (namely those of formula (I) or (Ia)). In particular, they have uncovered the ability of said statins of sensitizing to beta-lactamase resistant penicillins bacterial species resistant thereto, particularly by reducing or reversing resistance induced by low affinity penicillin-binding proteins (PBPs), wherein said bacterial species are characterized by synthetizing isoprenoids using the mevalonate synthetic pathway, i.e. Enterococci, Pneumococci or Staphylococci. This therapeutic effect provides for a medical application wherein only those patients infected with said bacterial species will be treated with a combination of a beta-lactamase resistant penicillin and one of said statins.

Accordingly, in a first aspect, the invention refers to a beta-lactamase resistant penicillin for use in a method of treating a bacterial infection in a subject in need thereof wherein said bacterial infection is caused by a bacterial population resistant to said beta-lactamase resistant penicillin i.e. Enterococci, Pnumococci or Staphylococci, wherein said subject is further administered, before or simultaneously to said beta-lactamase resistant penicillin, a statin of formula (I) or (Ia), wherein said statin is for use in reducing, mitigating or reversing resistance to said beta-lactamase resistant penicillin in resistant bacterial populations thereto of said bacterial species.

In an additional aspect, the present invention refers to a pharmaceutical kit comprising:

i. a pharmaceutical composition comprising a beta-lactamase resistant penicillin, and
ii. a pharmaceutical composition comprising a statin of formula (I) or (Ia), for use in a method of treatment as described herein.

## BRIEF DESCRIPTION OF THE DRAWINGS

Zaragozic acid is not a statin presently claimed. It is thus considered to be a reference compound in these figures.

**Figure 1. Identification of FMM constituent lipids. (A)** Ion chromatogram of FMM lipid markers in DRM (left) and DSM (right) fractions, labeled with RT and m/z ratios. Lipid abundance represented in absorbance units **(B)** Fragmentation pattern of FMM lipid markers at negative (top) and positive (bottom) ESI by product ion scan (MS/MS). Common fragments with respective MW and tentative formulas are shown. **(C)** Top, TLC detection of staphyloxanthin lipids in DRM and DSM fractions of WT and Δ*crt* mutant. Staphyloxanthin lipids are visualized as yellow-pigmented bands

(arrowheads). **(D)** UV-visible spectroscopy of purified staphyloxanthin and DRM/DSM samples (WT and Δ*crt* mutant). Arrowheads, characteristic 463 and 490 nm staphyloxanthin peaks. **(E)** Fluorescein-labeled lectin binding assay to WT and Δ*crt* DRM samples. WGA, wheat germ agglutinin; STL, *Solanum tuberosum* lectin; RCA, *Ricinus communis* agglutinin; DBA, *Dolichos biflorus* agglutinin; UEA, *Ulex europaeus* agglutinin; ConA, concanavalin A. **(F)** Relative abundance of FMM lipid markers in WT and Δ*crt* mutant using ion chromatography. Data shown as mean ± SD for three biological replicates (n = 3). **(G)** Tentative molecular structure and fragmentation pattern (blue, negative ESI; red, positive ESI) of staphyloxanthin-related FMM lipid markers. In another aspect, the present invention relates to a pharmaceutical composition comprising a beta-lactamase resistant penicillin, a statin of formula (I) or (Ia), and a pharmaceutically acceptable excipient or carrier, for use in a method of treatment according to the invention.

**Figure 2. FMM-constituent lipids and flotillin preferentially interact. (A)** Fluorescence micrographs of MRSA cells expressing FloA-YFP. Bottom, detail of flotillin focus localization and cell numbers. Two images show 3 foci in a dividing and a non-dividing cell. Dividing cells show foci at septal invaginations. **(B)** Quantification of focus number in WT and Δ*crt* mutant in exponential and stationary phase. Insets, quantification of septal focus number. We counted 700 random cells from each of three microscopic fields from independent experiments (n = 2100 cells/strain). **(C)** Scheme of WT, ΔMAR, ΔPHB and ΔEA4 flotillin variants. **(D)** Lipid-binding flotation assay. Left, flotation assay images using Nile Red (NR) for lipid staining. NR is fluorescent only in the presence of lipids. After ultracentrifugation, lipids migrate to the low-density sucrose fraction (0% sucrose; tube top). Right, FloA immunodetection in the lipid fraction (0% sucrose) after ultracentrifugation. C, control with no lipids; Stx, with staphyloxanthin lipids; pg, with phosphatidylglycerol (phospholipid). **(E)** Lipid-protein interaction of staphyloxanthin and phosphatidylglycerol with flotillin variants determined using BLI. Negative control (black line) is a cytoplasmic lactonase (YtnP) that does not interact with lipids (Schneider et al., 2012). Data shown as mean for three biological replicates (n = 3). Response measured in arbitrary units (a.u.).

**Figure 3. FMM-constituent lipids promote flotillin oligomerization. (A)** Size exclusion chromatography profiles of WT, ΔMAR, ΔPHB and ΔEA4 flotillin variants. Arrows show protein standards for calibration. **(B)** Scheme of the molecular process that organizes bacterial FMM. Top, flotillin N-terminal region preferentially binds FMM-constituent lipids via PHB domain interaction, whereas the C-terminal region is responsible for flotillin oligomerization. Bottom, a) constituent lipids aggregate in membrane microdomains based on similar physicochemical properties. b) Flotillin is confined to these microdomains via staphyloxanthin-PHB interaction. c) Flotillin oligomerizes via C-terminal interaction and accumulates to assemble FMM. **(C)** Left, TEM micrographs of purified flotillin oligomers alone or preincubated with staphyloxanthin, phosphatidylethanolamine (PE) or phosphatidylglycerol (PG). Staphyloxanthin-incubated oligomers generated larger protein assemblies. Bar, 50 nm. Right, BN-PAGE/immunoblot to detect FloA oligomers in WT and Δ*crt* mutant and SDS-PAGE of the same samples as a loading control. Bottom, immunoblot to detect FloA in the membrane fraction of WT (top) and Δ*crt* (bottom) strains resolved on a 5-40% sucrose gradient (fractions 1-12).

**Figure 4. FMM organization in S. *aureus* membranes. (A)** FloA immunodetection by whole-cell srAT (SIM + SEM) micrographs of 100-nm sections of MRSA cells. Each column shows all sections of a given cell. Yellow, FloA immunodetection; blue, Hoechst-stained micrograph. A 3D reconstruction of FloA signal organization is shown beneath each column. Bar, 1 μm. **(B)** Top, FloA immunodetection in 100-nm sections of dividing cells, overlaid on a SEM micrograph. Flotillin localizes near septal invaginations (a and b, in red). Bottom, TEM micrographs of septal invaginations to which flotillin localizes (a and b, above), showing light electron-dense membrane regions (arrowheads). Bar, 500 nm. **(C)** TEM micrographs showing immunogold detection of FloA in light electron-dense membrane areas. Inset, zoom of colocalizing region. Bar, 500 nm. **(D)** Statistical analysis of flotillin signal colocalization with light electron-dense membrane areas (LED) for 20 random cells from three independent experiments (n = 60). Data shown as mean ± SD. Significance was measured using an unpaired Student's t-test, ** p<0.01.

**Figure 5. FMM organization in S. *aureus* membranes. (A)** Left, Flotillin localization analyzed by super-resolution microscopy. dSTORM of S. *aureus* cells labeled with FloA-SNAP. Signal is detected in membrane foci (a, b and c) and in surrounding cytoplasm. Center, right, Mean flotillin cluster diameter and localizations/cluster for 20 random cells from three independent experiments (n = 60). **(B)** Virtual slice of an electron tomogram of a S. *aureus* cell showing FloA localization (a, b, c). **(C)** Magnified details of a, b and c, showing light electron-dense membrane areas. Small electron-dense particles show higher concentration in surrounding cytoplasm. Segmentation of cell structures shows dark electron-dense membrane areas (blue contour) and small electron-dense particles (yellow). Each yellow contour denotes four adjacent black pixels. **(D)** Top, 3D model of the tomogram in B, with (bottom) a detailed view of region a. Dark electron-dense membrane regions are shown in blue, light electron-dense nanodomains in red, and small electron-dense particles in yellow.

**Figure 6. MRSA proteins associated with the FMM-rich fraction. (A)** Workflow of membrane protein extraction and label-free quantification (LFQ) analyses of FMM-enriched associated proteins. **(B)** MS-based LFQ of the DRM fraction proteome of four growth conditions (LST, late stationary; EST, early stationary; EXP, exponential and NLC, nutrient-limiting conditions). Heatmap shows ratios of calculated protein abundance in DRM vs. total membrane proteome/DSM vs. total membrane proteome by unsupervised hierarchical clustering. Red denotes a DRM increase relative to total membrane proteome and blue, a decrease. Grey boxes indicate missing values. A number of proteins are highlighted. Clusters A (dark blue) and B (light blue) comprise proteins enriched in DRM in all growth conditions. Cluster C (dark green) shows DRM proteins in EXP; cluster D (red), in NLC; cluster E (light green), in EST; cluster F (pink), in LST. **(C)** Functional classification of DRM-associated proteins according to TIGRFAMM, in four growth conditions and a core of proteins detected in all conditions tested.

**Figure 7. Flotillin scaffolds PBP2a oligomerization. (A)** Bacterial two-hybrid analysis showing FloA-PBP2a interaction. - is empty plasmids (negative control). FloA-FloA interaction is positive control. Red line denotes 700 Miller unit threshold to define interaction (BACTH System; EuroMedex). Data shown as mean ± SD for three independent biological replicates (n = 3). **(B)** PBP2a immunodetection in protein samples pulled down with FloA-GFP (+/+ lane). Negative controls are FloA-GFP-labeled Δ*pbp2a* (or Δ*mecA)* strain (+/- lane) and unlabeled WT strain (-/+ lane). **(C)** srAT (SIM + SEM) to detect FloA-PBP2a co-localization in 100-nm sections. When detected in thin sections, PBP2a colocalized with flotillin. **(D)** BN-PAGE and immunoblot of PBP2a oligomeric states of various mutants. WT + ZA, membrane fraction of zaragozic acid-treated WT cells. Arrowheads, ligomeric species at 240 and 80 kDa. **(E)** Immunoblot of FloA and PBP2a oligomerization in membrane fractions from untreated or ZA-treated WT cells resolved on sucrose gradients. **(F)** Effect of MRSA resistance to several β-lactam antibiotics using WT, Δ*floA* and ZA-treated WT samples. Data shown as mean ± SD for three independent experiments (n = 3). Statistical analysis, one-way ANOVA with Tukey's test for multiple comparisons (*** p<0.001). **(G)** Survival curve of oxacillin-treated mice infected with WT, Δ*floA* mutant or ZA-treated WT ($3 \times 10^7$ cells; *n* = 10). Statistical analysis, one-way ANOVA with Tukey's comparison between WT vs. Δ*floA* or WT vs. WT+ZA (** p<0.01). Each point represents the mean of three independent experiments. **(H)** Bacterial load in lungs of oxacillin-treated infected mice in a pulmonary infection model. Mice were infected with $3 \times 10^8$ cells (n = 10). Two days after bacterial challenge, organs were collected aseptically and CFU counted. Statistical analysis, one-way ANOVA with Tukey's comparison (*** p<0.001). **(I)** Chemical structures of the beta-lactam antibiotics ampicillin, oxacillin, methicillin, flucoxacillin, nafcillin, and dicloxacillin.

**Figure 8 (related to Figure 1). Detection of FMM lipid markers in the DRM fraction. (A)** Scheme of the workflow to identify the most abundant DRM lipid markers using ultra-performance liquid chromatography coupled to mass spectrometry equipped with electrospray ionization source (UPLC-ESI-qTOF-MS). DRM lipid composition was compared to the control sample (buffer only). Of 2044 peaks, 39 were detected exclusively in the DRM fraction. The abundance of these peaks was determined in DSM samples; of the 39 peaks detected, 30 were not detected in the DSM fraction and were thus exclusive to the DRM fraction. Data for three independent biological replicates (n = 3) showed a consistently high concentration of 7 of the 30 peaks in the DRM vs. DSM fraction. Univariate statistical analysis (using three filters; infinite in FMM-enriched sample, signal-to-noise ratio of the most abundant peak >50 [area in progenesis: 10,000], and correlation variance <10%). These peaks were thus considered lipid markers for FMM. Detection of the peaks in different mutants and examination of the molecular fractionation pattern generated a tentative molecular formula. Experimental confirmation of the most significant features associated with the tentative molecular formula were confirmed by UV spectroscopy (to confirm that the molecule is a staphyloxanthin derivative) and lectin-probed sugar identification assay (to confirm that the molecule bears diverse sugars such as N-acetylglucosamine and N-acetylmuramic acid. **(B)** Total ion chromatograms of lipid species in the DRM fraction (top), DSM fraction (center) and buffer control (bottom) using UPLC-ESI-qTOF-MS. **(C)** Top, MS spectrum of the DRM fraction. The 7 FMM lipid markers are highlighted with their m/z values. Bottom, ionization and retention behavior of the 7 FMM lipid markers. MS spectra using negative ESI (i) and extracted chromatogram (ii) of the 7 FMM lipid markers. In (i), the Y axis represents normalized abundance and the X axis, mass-to-charge ratio (m/z). In (ii), the Y axis shows normalized abundance and the X axis, retention time (RT). Marker features were annotated as 4.0_1150, 4.2_1092, 4.5_1142, 4.6_1106, 4.7_1084, 4.7_1095, and 5.0_1098, according to their RT and nominal m/z. An RT between 4-5 min suggests polar characteristics of the markers (phospholipids elute at 6-8 min, triacylglycerols at 8-10 min). Mass spectra indicated a double-charged ion, since the mass difference of isotope peaks were 0.496-0.503 Da and the most abundant isotope was the second peak in all profiles.

**Figure 9 (related to Figures 1 and 2). Identification of FMM lipid markers. (A)** Lectin-probed blot analyses of FMM lipid samples from S. *aureus* wild type (WT) and the staphyloxanthin-deficient strain (Δ*crt* mutant). Carbohydrates bound to the FMM lipids were identified based on the specific carbohydrate-binding properties of various

lectins used in this assay. FMM lipids from WT and the Δ*crt* mutant were purified and immobilized on TLC membranes, which were blocked (10% non-fat milk) and incubated with distinct fluorescein-labeled lectins. After washing, a fluorescence signal is detected only if lectins are bound to the FMM lipids, through recognition of the sugars borne by the lipids. The lectins used and their recognition specificities are shown in the figure. Control sample (C) was incubated with no lectin, to detect sample autofluorescence. Positive signal was obtained with WGA and STL lectins in the WT sample but not the Δ*crt* mutant. **(B)** Quantitative determination of FMM lipid markers in DSM and DRM fractions in WT and Δ*crt* using UPLC-ESI-qTOF-MS. The Y axis shows normalized abundance; FMM lipid markers in the X axis are named according to RT and m/z (RT_m/z). FMM lipid markers were concentrated in the DRM fraction, and were not detected in Δ*crt* fractions. Data shown as mean ± SD of three independent biological replicates. **(C)** Fluorescence micrographs of MRSA cells expressing FloA-YFP at different integration sites in the chromosome. Bar, 5 μm. **(D)** Purification of staphyloxanthin lipids from exponential and stationary MRSA cultures. Samples were spotted on a TLC plate. Staphyloxanthin lipids are produced in cultures in exponential and stationary phases.

**Figure 10 (related to Figure 2). Flotillin preferentially binds staphyloxanthin. (A)** Top, biolayer interferometry (BLI) to assay lipid-protein interactions. This optical-analytical technique monitors the interference pattern of white light reflected from two surfaces, a layer of immobilized lipids on the biosensor tip, and an internal biocompatible surface (left). Any change in the number of proteins bound to the biosensor tip causes a shift in the interference pattern that can be detected and quantified (right). Interactions are measured in real time, providing the ability to monitor binding specificity with association/dissociation rates. Bottom, interaction between staphyloxanthin and the FloA variants (WT, ΔMAR, ΔPHB, ΔEA4) were measured using BLI. Purified staphyloxanthin was immobilized on aminopropylsilane biosensor tips. 0.5 μM protein solution was added and affinity constants ($K_D$) calculated using $K_a$ (association) and $K_d$ (dissociation) rate constants. Values are the mean of three independent experiments. Chi-squared $X^2$ and $R^2$ indicates goodness of fit. As control, interaction of flotillin variants with membrane phosphatidylethanolamine (PE) or phosphatidylglycerol (PG) was tested. The signal in these control assays did not fit association and dissociation kinetics thus their $X^2$ and $R^2$ showed poor fit and affinity constants $K_D$ could not be extracted. **(B)** Control experiments using BLI showing staphyloxanthin, PE or PG binding to the biosensor tip. These experiments tested two dissociation conditions with buffer containing 0.001% (solid line) or 0.02% (dashed line) DDM (docecylmatoside); 0.001% is the DDM concentration used to test FloA binding to lipids in BLI experiments (main figure 2E). Conditions using 0.02% DDM were 20-fold higher DDM concentration than normal resting conditions. Affinity constants ($K_D$) and goodness of fit $X^2$ and $R^2$ were calculated for both binding conditions. Values are the mean of three independent experiments. In both cases, lipid binding fit an association curve correctly, showing marked, predictable and reproducible staphyloxanthin and PE or PG association to the biosensor. The presence of DDM in the buffer at the concentrations used to test flotillin binding or higher did not cause marked lipid dissociation from the biosensor tip. Response measured in arbitrary units (a.u.). **(C)** Top, immunodetection of FloA-YFP (WT) and YFP-labeled flotillin variants (ΔMAR, ΔPHB, ΔEA4) in S. *aureus* cytoplasmic and membrane fractions, using polyclonal anti-YFP antibodies. Bottom, fluorescence microscopy analyses of subcellular localization of FloA-YFP (WT) and YFP-labeled flotillin variants (as above) in S. *aureus* cells. Bar, 5 μm.

**Figure 11 (related to Figures 3 and 4). Visualization of FMM in whole cells. (A)** Left, TEM micrographs of purified flotillin oligomers alone or preincubated with staphyloxanthin or PE/PG. Staphyloxanthin-incubated FloA oligomers generated larger protein assemblies. Fields corresponding to detailed micrographs (main figure 3C). Right, control TEM micrographs of purified lipids alone (staphyloxanthin or PE/PG). In the absence of flotillin, lipid samples do not organize large assemblies. Bar, 100 nm. **(B)** Super-resolution array tomography (srAT) using structural illumination microscopy (SIM) and scanning electron microscopy (SEM) (SIM +SEM) of S. *aureus* cells. Cells are embedded in a methacrylate matrix and thin-sectioned in 100-nm slices. Columns show each of the 100-nm sections from an entire cell. FloA was immunodetected with Alexa-488-conjugated secondary antibody (yellow signal, second row). DNA was stained using Hoechst 33258 dye (blue signal, third row) to determine cell contour in fluorescence microscopy images. The SEM image is used as background (first row). First right column shows a merge of all three channels. Bar, 1 μm. (C) Control TEM micrographs of staphyloxanthin-deficient cells (Δ*crt* mutant) collected at stationary phase. Left, single cells; right, dividing cells. Uranyl acetate staining shows cell membranes with more uniform electron contrast in the Δ*crt* mutant than WT cells. Bar, 300 nm. **(D)** Top, immunogold labeling of FloA in thin-sectioned cells visualized by TEM. Gold particles (10-nm diameter) localized in discrete membrane foci. FloA signal colocalizes with light electron-dense membrane areas. Bottom, differential electron density map of FloA immunogold-labeled TEM image. Gold particles labeled in yellow. This map corresponds to the image in main figure 4C. Bar, 300 nm.

**Figure 12 (related to Figure 6). Identification of proteins localized preferentially in DRM fractions using label-free proteomic quantification (LFQ). (A)** SDS-PAGE analysis of DRM and DSM fractions, which show comparable

protein concentrations in the four growth conditions tested (exponential phase, EXP; early stationary phase, ESP; late stationary phase, LST, and nutrient-limiting conditions, NLC). *Staphylococcus aureus* was grown in TSB medium (37°C, 200 rpm). For EXP, cells were collected after 3 h incubation, for ESP, after 12 h and for LST, after 24 h. For NLC, cells were grown in TSB medium supplemented with 0.5 mM dipyridyl (12 h, 37°C, 200 rpm). **(B)** Protein quantification using label-free liquid chromatography-mass spectrometry (LC-MS). Scatterblots of identified proteins are given as normalized log2 ratios. Each dot represents one protein. After normalization, DRM vs total membrane ratio was plotted (Y axis) and DSM vs. total membrane (X axis) for each growth condition. Imputed values (Imp.) indicate that, for log-ratio calculation, protein detected in DRM or DSM was not detected in the "total membrane fraction". This could be due to sample complexity, which renders some proteins below our detection limit. Although the value for these proteins would be 0, we used an imputed value of 1 to enable ratio calculation. These proteins are indicated by unfilled colored circles. Colored dots are proteins outside an interquartile range (IQR) of 1.5. IQR is a measure of statistical dispersion of the data, as it determines the difference between upper and lower quartiles. Statistically significant outliers (outside the IQR; these proteins show enrichment in one fraction vs the other) are colored dots. Non-significantly enriched proteins are shown in grey. Red dots are proteins whose DRM and DSM measurements are both outside IQR = 1.5; blue dots are those with only one value outside IQR = 1.5 and for yellow dots, only one value was available (proteins detected exclusively in DRM or DSM). The scheme shows various scatterblot zones representing proteins found exclusively or enriched in DSM or DRM.

**Figure 13 (related to Figure 7). Flotillin scaffold activity contributes to PBP2a oligomerization. (A)** SrAT (SIM+SEM) 100-nm thin section image of S. *aureus* cells. For FloA immunodetection, Cy3-conjugated secondary antibody was used (red) and for PBP2a, Cy5-secondary antibody (green). DNA was Hoechst 33258-stained (blue). Overlay shows the merge of all signals. PBP2a and FloA signals colocalized in discrete membrane foci. Bar, 300 nm. **(B)** Bacterial three-hybrid analysis showing interaction of PBP2a with the tentative interacting partners PBP2, PrsA and RodA, and the non-interacting control protein FtsZ at distinct FloA concentrations. Three pSEVA plasmids were used to express FloA at distinct concentrations. pSEVA 621, 631 and 641 plasmids maintain similar backbones and expressed *floA* under the control of a constitutive promoter. Plasmids bear different replication origins; pSEVA 621 carries the low-copy-number replication origin RK2, pSEVA 631, the medium-copy-number replication origin pBR1, and pSEVA 641, the high-copy-number replication origin pRO1600. The strains carrying each of these plasmids thus produce FloA at different concentrations as a direct function of the *floA* copy number expressed. Dashed red line indicates the threshold limit that defines a positive (≥700 Miller units) or negative interaction signal (<700 Miller units). Data shown as mean ± SD of three independent biological replicates. **(C)** Molecular structure of the statins tested in this study. **(D)** Scheme of the mevalonate pathway and its bifurcation to produce staphyloxanthin-related lipids in S. *aureus* (blue background) or cholesterol in humans (green). Zaragozic acid (ZA) is a competitive inhibitor in both routes, acting downstream of formation of farnesyl pyrophosphate (FPP). Statins such as simvastatin also inhibit both routes, as they inhibit the enzyme HMG-CoA reductase.

**Figure 14 (related** to **Figure 7). Synergistic antimicrobial effect of statin and β-lactam antibiotics. (A)** Growth curves of S. *aureus* cultures at different ZA concentrations. Cultures were grown in TSB medium and incubated (36 h, 37°C, 200 rpm). ZA addition to cultures did not affect S. *aureus* growth at the concentration used (50 μM). Data shown as mean ± SD of three independent biological replicates. **(B)** Immunodetection of the chaperonin GroEL in S. *aureus* cell extracts. Untreated (control) and treated samples (ZA) showed comparable GroEL levels, suggesting that S. *aureus* treatment with ZA at the concentration tested had no pronounced effects on major cell processes or weakened cell physiology. **(C)** Measure of the reduction in focus number in ZA-treated S. *aureus* cells. We counted 700 random cells from each of three independent microscopic fields in independent experiments (n = 2100 cells total/strain). **(D)** Bacterial count (colony-forming units/ml) of MRSA cultures treated with a combination of ZA or simvastatin and the β-lactam antibiotic oxacillin. MRSA growth was unaltered in the presence of ZA or simvastatin. Growth was inhibited by oxacillin if ZA or simvastatin were added to the cultures. Increasing ZA or simvastatin concentration in the cultures potentiated the antibiotic effect of oxacillin. **(E)** Bacterial count (CFU/ml) of MRSA cultures treated with a combination of statins and the β-lactam antibiotic oxacillin. L, lovastatin; M, mevastatin; P, pravastatin; S, simvastatin; A, atorvastatin; F, fluvastastin, ZA, zaragozic acid. Whereas some statins inhibited growth in response to oxacillin, others such as atorvastatin or fluvastatin did not alter the MRSA antibiotic-resistant phenotype. We attribute this "all-or-nothing" effect to the properties of certain molecules, which prevents their penetration of the cell envelope and thus, encounter with the target (the cytoplasmic enzyme CrtM). Data shown as mean ± SD of three independent biological replicates. Statistical analysis was carried out using an unpaired Student's t-test (***P<0.001). **(F)** Oxacillin minimal inhibitory concentration (MIC) antibiotic susceptibility test of different strains (MRSA and MSSA strains), mutants (the Δ*floA* MRSA mutant) and ZA- or simvastatin-treated MRSA strains. **(G)** Survival curve of oxacillin-treated mice infected with ZA-treated WT ($3 \times 10^7$ cells; *n* = 10). ZA was administered at two concentrations. Statistical analysis, one-way ANOVA with Tukey's comparison between WT vs. WT+ZA (*

p<0.05, ** p<0.01).

**Figure 15. Mevalonate and MEP isoprenoids synthetic route in bacteria.** It corresponds to Fig.1 in Heuston et al. 2012. Representation of the MEP and mevalonate pathways for the synthesis of the universal isoprenoids precursor isopentyl diphosphate (IPP). MEP genes are shown with their historical designation and current nomenclature (bold type). Statins inhibit HMGR, the rate-limiting enzyme of the mevalonate pathway. The penultimate compound of the MEP pathway is HMB-PP, a non-peptidic antigen which is a potent activator of human Vc9/ Vd2 T cells. GA3P, glyceraldehyde 3-phosphate.

**Figure 16. List of beta-lactam antibiotics** (source: Mededucation)

## DETAILED DESCRIPTION OF THE INVENTION

Definitions

The term "treatment" as used herein refers to prophylactic and/or therapeutic treatment.

The term "therapeutic treatment" as used herein refers to bringing a body from a pathological state or disease back to its normal, healthy state. Specifically, unless otherwise indicated, includes the amelioration, cure, and/or maintenance of a cure (i.e., the prevention or delay of relapse) of a disease or disorder. Treatment after a disorder has started aims to reduce, alleviate, ameliorate or altogether eliminate the disorder, and/or its associated symptoms, to prevent it from becoming worse, to slow the rate of progression, or to prevent the disorder from re-occurring once it has been initially eliminated (i.e., to prevent a relapse). It is noted that, this term as used herein is not understood to include the term "prophylactic treatment" as defined herein.

The term "prophylactic treatment" or "preventive treatment" as used herein refers to preventing a pathological state. It is noted that, this term as used herein is not understood to include the term "therapeutic treatment" as defined herein.

The term "effective amount" as used herein refers to an amount that is effective, upon single or multiple dose administration to a subject (such as a human patient) in the prophylactic or therapeutic treatment of a disease, disorder or pathological condition.

The term "subject" as used herein refers to a mammalian subject. Preferably, it is selected from a human, companion animal, non-domestic livestock or zoo animal. For example, the subject may be selected from a human, dog, cat, cow, pig, sheep, horse, bear, and so on. In a preferred embodiment, said mammalian subject is a human subject.

The term "combination" or "combination therapy" as used throughout the specification, is meant to comprise the administration of the referred therapeutic agents to a subject in need of such a treatment, in the same or separate pharmaceutical formulations, and at the same time or at different times. If the therapeutic agents are administered at different times they should be administered sufficiently close in time to provide for the combined effect (e.g. potentiating or synergistic response) to occur. The particular combination of therapies to employ in a combination regimen will take into account compatibility of the desired therapeutics and/or procedures and/or the desired therapeutic effect to be achieved.

The term "biological sample", as used herein, may refer to biological material isolated from a subject. The biological sample may contain any biological material suitable for detecting, isolating or analyzing the infectious bacterial species. The sample can be isolated from any suitable biological tissue or fluid such as, for example, blood, blood plasma, serum, cerebral spinal fluid (CSF), urine, amniotic fluid, lymph fluids, external secretions of the respiratory, intestinal, genitourinary tracts, tears, saliva, white blood cells. Preferably, said biological sample is a sample which can be obtained using minimally invasive procedures, such as intestinal samples.

The term "pharmaceutically acceptable salt" as used herein refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, trifluoroacetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, and ammonium. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate.

In the present context, an infectious agent (e.g. a bacterial strain or population) is said to be "resistant" or "drug resistant" if the infectious agent has undergone a change which reduces or eliminates the effectiveness of an anti-infective agent which is normally used to cure infections caused by the infectious agent. Analogously, the term "drug resistance" means a circumstance when a disease, e.g. an infectious disease, does not respond to a therapeutic agent, such as an anti-infective agent. Drug resistance can be intrinsic, which means that the disease has never been responsive to the therapeutic agent, or acquired, which means that the disease ceases responding to the therapeutic agent to which the disease had previously been responsive.

## Detailed description of the invention

In a first aspect, the invention refers to a beta-lactamase resistant penicillin for use in a method of treating a bacterial infection in a subject in need thereof wherein said bacterial infection is caused by a bacterial population, i.e. Enterococci, Pneumococci or Staphylococci, resistant to said beta-lactamase resistant penicillin, wherein said subject is further administered, before or simultaneously to said beta-lactamase resistant penicillin a compound inhibiting isoprenoid lipids synthesis (e.g., inhibitors of staphyloxanthin synthesis), i.e. the statins of formula (I) or (Ia), wherein said compound is for use in reducing, mitigating or reversing resistance to said beta-lactamase resistant penicillin in resistant bacterial populations thereto of said bacterial species.

In addition, in a related aspect, the present invention provides a method of treating a bacterial infection in a subject in need thereof, wherein said bacterial infection is caused by a bacterial species characterized by comprising isoprenoid lipids, such as staphyloxanthin and its derivatives, in its bacterial membrane, i.e. Enterococci, Pneumococci or Staphylococci, wherein said method comprises administering to a subject in need of such treatment, a prophylactically or therapeutically effective amount of a beta-lactamase resistant penicillin and further administering to said subject, before or simultaneously to said beta-lactam antibiotic, a compound inhibiting isoprenoid lipids synthesis , i.e. the statins of formula (I) or (Ia), in a prophylactically or therapeutically effective amount for reducing, mitigating or reversing resistance to said beta-lactamase resistant penicillin in resistant bacterial populations thereto of said bacterial species.

In a further related aspect, it provides the use of a beta-lactmase resistant penicillin in the manufacture of a medicament for the treatment of a bacterial infection in a subject in need thereof in combination with a compound inhibiting isoprenoid lipids synthesis (e.g., inhibitors of staphyloxanthin synthesis), i.e. the statins of formula (I) or (Ia), wherein said bacterial infection is caused by a bacterial species characterized by comprising isoprenoid lipids in its bacterial membrane, i.e. Enterococci, Pneumococci or Staphylococci.

In another particular embodiment, the present invention relates to a beta-lactamase resistant penicillin for use in a method of treating a bacterial infection in a subject in need thereof, wherein said bacterial infection is caused by a bacterial species synthetizing isoprenoids using the mevalonate synthetic pathway, i.e. Enterococci, Pneumococci or Staphylococci, wherein said subject is further administered, before or simultaneously to said beta-lactamase resistant penicillin, a compound inhibiting isoprenoid lipids synthesis selected from a statin of formula (I) or (I)a , wherein said compound is for use in reducing, mitigating or reversing resistance to said beta-lactamase resistant penicillin in resistant bacterial populations thereto of said bacterial species.

In addition, in a related embodiment, the present invention provides a method of treating a bacterial infection in a subject in need thereof, wherein said bacterial infection is caused by a bacterial species characterized by comprising isoprenoid lipids in its bacterial membrane and for synthetizing isoprenoids using the mevalonate synthetic pathway, i.e. Enterococci, Pneumococci or Staphylococci, wherein said method comprises administering to a subject in need of such treatment a prophylactically or therapeutically effective amount of a beta-lactamase resistant penicillin and further administering to said subject, before or simultaneously to said beta-lactamase resistant penicillin, a compound inhibiting isoprenoid lipids synthesis selected from a statin of formula (I) or (Ia), preferably, wherein $R^1$ and $R^2$ are independently selected from the group consisting of H, OH, $CH_3$, $CH_2CH_3$, and halogen, or a pharmaceutically acceptable salt or stereoisomer thereof, in a prophylactically or therapeutically effective amount for reducing, mitigating or reversing resistance to said beta-lactamase resistant penicillin in resistant bacterial populations thereto of said bacterial species.

In a further related embodiment, it provides the use of a beta-lactamase resistant penicillin in the manufacture of a medicament for the treatment of a bacterial infection in a subject in need thereof in combination with a compound inhibiting isoprenoid lipids synthesis selected from statin of formula (I) or (Ia), wherein said bacterial infection is caused by a bacterial species characterized by comprising isoprenoid lipids in its bacterial membrane and for synthetizing isoprenoids using the mevalonate synthetic pathway, i.e. Enterococci, Pneumococci or Staphylococci, wherein said method of treatment is as described herein. Beta-lactam antibiotics are a class of broad-spectrum antibiotics, consisting

of all antibiotic agents that contain a beta-lactam ring in their molecular structures. This includes penicillin derivatives (penams), cephalosporins (cephems), monobactams, and carbapenems. A list is provided in Fig. 16 (The term "penicillin" may also be used herein to refer to beta-lactam antibiotics). Only beta-lactamase resistant penicillins are within the scope of the present invention.

Beta-lactamases are a family of enzymes involved in bacterial resistance to beta-lactam antibiotics, which act by breaking the beta-lactam ring. They can be encoded chromosomally or on extrachromosomal elements. There are two major schemes for beta-lactamase classification, the Ambler and the Bush-Jacoby-Medeiros systems. The Ambler system (Ambler RP et al., Biochem J 1991, 276, 269-70) classifies the enzyme in four different groups A, B, C and D, based on the enzyme structure; whereas the Bush-Jacoby-Medeiros system (Bush K., Jacoby GA, Medeiros AA, Antimicrob Agents Chemother 1995, 39, 1211-33) is based on their substrate profile, i.e., which class of beta-lactams is degraded and to what degree activity is inhibited by the beta-lactamase inhibitor clavulanic acid. For instance, see Table 1 of Drawz S.M. and Bonomo R.A.,(Clinical Microbiology Reviews 2010, 23(1), 160-201) which provides a classification of beta-lactamases under both Ambler class and Bush-Jacoby-Medeiros class and representative enzymes within each group.

Strategies for combating this form of resistance have included the search for new beta-lactam antibiotics that are more resistant to the enzymatic cleavage (a.k.a. beta-lactamase resistant beta-lactam antibiotics) and the development of a class of enzyme inhibitors called beta-lactamase inhibitors that prevent the degradation of beta-lactam antibiotics. These include but are not limited to clavulanic acid, sulbactam, tazobactam, avibactam, relebactam, RG6080 and RPZ7009 (see Table 6 of Bush K, Bradford PA. 2016. β-Lactams and β-Lactamase Inhibitors: An Overview.*Cold Spring Harb Perspect Med.* 6(8)).

Beta- **10** lactamase resistant penicillins are flucloxacillin, cloxacillin, dicloxacillin, methicillin, oxacillin, nafcillin, temocillin, and floxacillin.

In a particular embodiment, optionally in combination with one or more of the features or embodiments described herein, said penicillin resistant to beta-lactamases selected from the group consisting of flucloxacillin, cloxacillin, dicloxacillin, methicillin, oxacillin, nafcillin, temocillin, and floxacillin. Antibiotics belonging to this group are classified by the World Health organization under ATC/DDD Index J01CF.

Bacterial resistance against beta-lactamase resistant penicillins is due to non-enzymatic resistance mechanisms to their activity as described herein below, which may include a reduced access to the PBPs (e.g., by the presence of efflux pumps) or by PBPs of reduced binding affinity, such as PBP2a in MRSA. In addition to *Staphylococcus aureus,* other gram positive and gram negative species have been described for presenting modified PBPs. For instance, Enterococci spp. (e.g., *Enterococcus faecalis, Enterococcus faecium* or *Enterococcus hirae),* pneumococcus strains (e.g., *Streptococcus pneumoniae), Neisseria spp. (e.g., Neisseria gonorrhoeae* and *Neisseria meningitidis), Haemophilus influenzae, Helicobacter pylori, Escherichia coli, Proteus mirabilis, Pseudomonas aeruginosa, Salmonella muenchen, Acinetobacter baumanii, Listeria monocytogenes,* etc. (see for instance Zapun et al. 2008).

The bacterial membrane is known to contain isoprenoid lipids The presence of isoprenoid lipids in the bacterial membrane may be identified for instance by isolating the infectious bacterial species from a biological sample of the subject and obtaining a bacterial membrane extract and determining the presence of isoprenoid compounds by any method known in the art for detecting/quantifying a compound in a biological mixture. This includes for instance all the chromatographic methods which are not limited to gas chromatography, liquid chromatography, supercritical fluid chromatography, ultra-performance liquid chromatography, and combinations thereof with mass spectrometry. For instance, an easy way to detect these isoprenoid compounds is by a thin layer chromatography of the bacterial membrane fraction. Indeed, these isoprenoid lipids are carotenoids and thus are naturally coloured, typically ranging from brown-red-orange-pink depending on the degree of oxidation. The isoprenoid compounds in the sample may also be coloured by reaction with a compound which increases the intensity of the signal (see in the Examples "Thin-layer chromatography (TLC)").

Two isoprenoid synthetic pathways have been described in bacterial species, the classical mevalonate pathway or the alternative 2C-methyl-D-erythritol 4-phosphate (MEP) pathway (see Fig.15). The mevalonate route is also shown in Fig. 13D, wherein it is also indicated the steps in the synthetic route which are down-modulated by a statin of formula (I) or (Ia) and Zaragozic acid.

The presence of the mevalonate isoprenoid synthesis pathway may be measured by determining the presence of genes and/or proteins characteristic of the mevalonate synthetic route. The detail of the mevalonate pathway, specifying the enzymes catalysing each metabolic step as well as the genes encoding the same is provided in Fig.15. Preferably, presence of the mevalonate route is determined by detecting HMG-CoA reductase gene (gene mvaA) and/or its gene product. This may be conducted by any method known by a person skilled in the art for the detection or quantification of proteins or genes.

Molecular biology methods for detecting/quantifying a target nucleic acid sequence are well known in the art. These methods include but are not limited to end point PCR, competitive PCR, reverse transcriptase-PCR (RT-PCR), quantitative PCR (qPCR), reverse transcriptase qPCR (RT-qPCR), PCR-pyrosequencing, PCR-ELISA, DNA microarrays, in situ hybridization assays such as dot-blot or Fluorescence In Situ Hybridization assay (FISH), genome sequencing

and next generation sequencing, and multiplex versions of the above techniques, as well as the next generation of such techniques and combinations thereof. There are several methods for the detection/quantification of peptides and proteins well known to one skilled in the art, such as immunoassays. Various types of immunoassays are known to one skilled in the art for the quantitation of proteins of interest, either in solution or using a solid phase assay. These methods are based on the use of affinity reagents, which may be any antibody or ligand specifically binding to the target protein, which is preferably labeled. For example, western blotting or immunoblotting allows comparison of the abundances of proteins separated by an electrophoretic gel, eg. SDS-PAGE. Another immunoassay commonly used for protein quantification is the enzyme-linked immunosorbent assay (ELISA) in which the detection antibody carries an enzyme that converts a commonly colorless substrate into a colored compound or a non-fluorescent substrate to a fluorescent compound. In other solid phase immunoassays, the antibody may be labeled with a radioactive isotope or a fluorescent reagent. Other methods that can be used for quantification of proteins are techniques based on mass spectrometry (MS) such as liquid chromatography coupled to mass spectrometry (LC / MS), described for example in US2010 / 0173786, or tandem LC-MS / MS (WO2012 / 155019, US2011 / 0039287, M. Rauh, J Chromatogr B Analyt Technol Biomed Life Sci 2012 February 1, 883-884. 59-67) and the use of arrays of peptides, proteins or antibodies and multiplex versions of the above techniques, as well as the next generation of such techniques and combinations thereof. The method of treatment of the present invention may be used for treating a subject having or at risk to have a bacterial infection of Enterococci, Pneumococci or Staphylococci.

In a particular embodiment, said bacterial species is a gram positive or gram negative bacteria. Preferably, said bacterial species is selected from the list of bacterial species consisting of: *Staphylococcus arlettae, Staphylococcus aureus, Staphylococcus capitis, Staphylococcus caprae, Staphylococcus carnosus, Staphylococcus epidermidis, Staphylococcus equorum, Staphylococcus haemolyticus, Staphylococcus hominis, Staphylococcus lugdunensis, Staphylococcus pasteuri, Staphylococcus pettenkoferi, Staphylococcus pseudointermedius, Staphylococcus saprophyticus, Staphylococcus simiae, Staphylococcus simulans, Staphylococcus warneri,* and *Streptococcus* pneumoniae.

Preferably, said bacterial species presents for the synthesis of isoprenoids exclusively the mevalonate route.

In Table 1 below (corresponding to Table 1 of Heuston et al. 2012) is shown the distribution of the MEP and mevalonate pathways for various Gram-positive and Gram-negative pathogenic species.

**Table 1.** Distribution of the MEP and mevalonate pathways amongst representative strains of Gram-positive and Gram-negative pathogens

| Pathogen | MEP | Mevalonate | Reference(s) |
|---|---|---|---|
| **Gram-positive pathogens** | | | |
| *Bacillus anthracis* str. Sterne | + | − | * |
| *Bacillus subtilis* subsp. *subtilis* 168 | +† | − | Laupitz *et al.* (2004); Takagi *et al.* (2004) |
| *Clostridium difficile* 630 | + | − | * |
| *Clostridium botulinum* B1 str. Okra | + | − | * |
| *Clostridium perfringens* E str. JGS1987 | + | − | * |
| *Enterococcus faecalis* | − | + | Boucher & Doolittle (2000) |
| *L. monocytogenes* EGDe | + | + | Begley *et al.* (2004)* |
| *L. innocua* Clip11262 | −‡ | + | Begley *et al.* (2004)* |
| *Listeria seeligeri* | −‡ | + | * |
| *Nocardia terpenica* | + | − | Shigemori *et al.* (1999) |
| *Staph. aureus* | − | + | Hammond & White (1970) |
| *Strep. pneumoniae* | − | + | Wilding *et al.* (2000b) |
| *Strep. pyogenes* | − | + | Wilding *et al.* (2000b) |
| **Gram-negative pathogens** | | | |
| *B. abortus* | +† | −§ | Sangari *et al.* (2010)* |
| *Borrelia burgdorferi* | − | + | Boucher & Doolittle (2000) |
| *Chlamydia trachomatis* 434/Bu | + | − | Rohdich *et al.* (2001) |
| *Chlamydia pneumoniae* | + | − | Eberl *et al.* (2003) |
| *S. enterica* serovar Typhimurium | + | − | Cornish *et al.* (2006)* |
| *E. coli* O157 : H7 | + | −§ | * |
| *E. coli* O127 : H6 | + | − | * |
| *F. tularensis* | + | − | Eberl *et al.* (2003) |
| *Legionella pneumophila* | − | + | Boucher & Doolittle (2000); Gophna *et al.* (2006) |
| *P. aeruginosa* | + | − | Putra *et al.* (1998) |
| *V. cholerae* | + | −‖ | Gophna *et al.* (2006)* |
| *K. pneumoniae* subsp. *pneumoniae* MGH 78578 | + | − | * |
| *Bordetella pertussis* Tahoma I | + | − | * |
| *Haemophilus influenzae* | + | − | Boucher & Doolittle (2000) |
| *H. pylori* | + | − | Pérez-Gil *et al.* (2010) |
| *Shigella flexneri* | + | − | * |
| *Shigella dysenteriae* Sd197 | + | − | * |
| *Neisseria gonorrhoeae* FA | + | − | * |
| *Neisseria meningitidis* | + | − | Boucher & Doolittle (2000) |
| *C. jejuni* subsp. *jejuni* 81116 | + | − | Gabrielsen *et al.* (2004) |
| *Y. enterocolitica* | + | − | * |

*Sequences were analysed by performing BLASTP searches (using the web-based BLAST program) on the National Centre for Biotechnology Information website (http://www.ncbi.nlm.nih.gov/).
†Possess a DRL enzyme instead of DXR.
‡Possess some of the pathway genes; missing the final two MEP genes.
§A type II IPP isomerase has been characterized.
‖HMGR enzyme is present.

In a preferred embodiment, said bacterial species is selected from the group consisting of *Enterococcus faecalis,*
*Staphylococcus aureus,* and *Streptococcus* pneumoniae.

The mode of action of beta-lactam antibiotics and non-enzymatic resistance mechanisms to their activity are intimately linked to the structure and biosynthesis of the bacterial cell wall. The bacteriostatic effect of beta-lactam antibiotics has been described to relate to the inhibition of essential enzymes (transpeptidases, carboxypeptidases) involved in the terminal stages of peptidoglycan biosynthesis. These cytoplasmic membrane-associated target enzymes bind the antibiotics covalently, and hence are known as penicillin-binding proteins (PBPs).

Resistance to beta-lactam antibiotics in Gram-positive bacteria, in the absence of a beta-lactamase, is generally due to various modifications of the PBPs, including enterococci, pneumococcus and staphylococci (see for instance, Zapun et al. 2008; Laible G. and Hakenbeck R. (Journal of Bacteorology 1991, 173(21), 6986-6990 with respect to beta-lactam resistance induced by the low affinity penicillin-binding protein 2x (PBP2x) of *Streptococcus pneumoniae* or Zorzi et al., Journal of Bacteriology 1996, 178(16), 4948-4957 on the low affinity PBP5 (PBP5fm) in *Enterococcus faecium).* In a further particular embodiment, optionally in combination with one or more of the features or embodiments described herein, said bacterial species is a Gram-positive bacterial species.

The term resistant bacterial population has been defined herein above. A bacterial population or bacterial strain

is generally considered to be resistant to a beta-lactam antibiotic when it has MIC values for said beta-lactam antibiotic above a threshold concentration or when its genome contains resistance markers known to be involved in beta-lactam resistance.

In still a further particular embodiment, optionally in combination with one or more of the features or embodiments described herein, said bacterial species is known or typically considered to be resistant to said beta-lactam antibiotic. Typically, a bacterial species is considered to be resistant to an antibiotic where a percentage of more than 80%, preferably more than 85%, 90%, 95%, or 97% of the known strains of this bacterial species are resistant to said antibiotic. Bacterial species typically presenting non-enzymatic resistance to beta-lactam antibiotics associated to the presence of low affinity PBPs are described herein above.

In a preferred embodiment, said bacterial species is a *Staphylococcus aureus* strain, including clinical isolates or any derivatives. As shown in the examples, *S.aureus* strains are characterized by presenting staphyloxantin-rich membrane microdomains. Preferably, said *S.aureus* strain is selected from the group consisting of Methicillin-resistant Staphylococcus aureus (MRSA), Community-associated Methicillin-resistant Staphylococcus aureus (CA-MRSA), vancomycin intermediate resistant staphylococcus aureus (VISA) and vancomycin resistant staphylococcus aureus (VRSA). Preferably, said *S.aureus* strain is a MRSA strain. MRSA identification may be performed by testing susceptibility to antimicrobials, such as by using the agar microdilution method, growing bacteria in chromogenic agar, multilocus sequence typing (MLST), spa typing, SCC mec typing and by genotyping analysis using pulsed-field gel electrophoresis (PFGE). For instance, susceptibility to oxacillin may be determined, wherein a MIC of oxacillin equal to or above 4 microgr/ml is indicative of a methicillin resistant strain (https://www.cdc.gov/mrsa/lab/index.html#fn1).

After identification of a SA strain as MRSA, it is generally performed vancomycin susceptibility testing using a validated MIC method. Centre for Disease Control (CDC) definitions for classifying isolates of S. *aureus* with reduced susceptibility to vancomycin are based on the laboratory breakpoints established by the Clinical and Laboratory Standards Institute (CLSI). MIC values between 4-8 μg/ml indicate VISA isolates and MIC values above 16 μg/ml indicate VRSA isolates (see for instance, Charlene R. Jacksonetl al., J. Clin. Microbiol. April 2013 vol. 51 no. 4 1199-1207; Swenson JM et al., J Clin Microbiol. 2009; 47(7):2013- 2017).

Preferably, said statin is a statin of formula (I) or (Ia) (shown below), or a pharmaceutically acceptable salt or stereoisomer of any thereof:

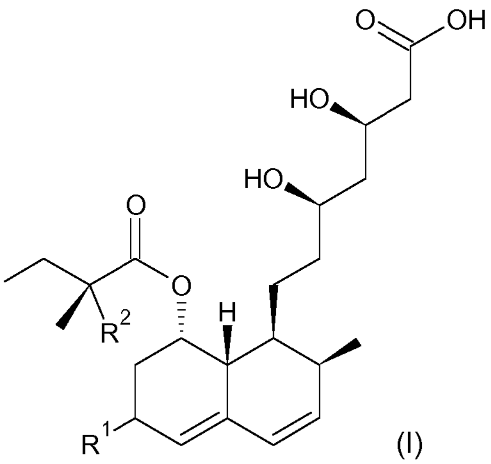
(I)

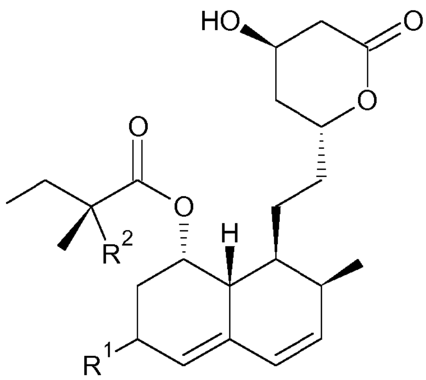
(Ia)

wherein $R^1$ and $R^2$ are independently selected from the group consisting of H, OH, $CH_3$, $CH_2CH_3$, and halogen. It is understood that the statins in the neutral form may either be in the form of the free β,δ-dihydroxy-acid as depicted in formula (I) or in the form of the corresponding lactone of formula (Ia).

The compound of formula (I) or formula (Ia) may be present in any stereochemical form. In a preferred embodiment, the compound of formula (I), formula (Ia) or a pharmaceutically acceptable salt thereof is used in the enantiomeric form depicted in formula (I) and formula (Ia).

Furthermore, the carbon to which $R^1$ is bound may be in the R configuration or the S configuration. In one embodiment, the carbon to which $R^1$ is bound is in the R configuration. In another embodiment, the carbon to which $R^1$ is bound is in the S configuration.

In one embodiment, R1 is selected from the group consisting of H, OH, and CH3. In another embodiment, R2 is selected from the group consisting of H and CH3.

In one embodiment, the compound of formula (I) or formula (Ia) is selected from the group consisting of mevastatin, lovastatin, pravastatin, simvastatin, and pharmaceutically acceptable salts thereof (see Figure 13 C). In another embodiment, the compound of formula (I) or formula (Ia) is selected from the group consisting of mevastatin, lovastatin,

pravastatin, simvastatin, and pharmaceutically acceptable salts thereof.

In still a further embodiment, the compound of formula (I) or formula (Ia) is selected from the group consisting of lovastatin, pravastatin, simvastatin, and pharmaceutically acceptable salts thereof. In another embodiment, the compound of formula (I) or formula (Ia) is lovastatin or a pharmaceutically acceptable salt thereof. In yet another embodiment, the compound of formula I or formula Ia is pravastatin or a pharmaceutically acceptable salt thereof. In still another embodiment, the compound of formula (I) or formula (Ia) is simvastatin or a pharmaceutically acceptable salt thereof.

In further embodiment, R1 and R2 are other than CH3, in other words, the compound of formula (I) or (Ia) is not simvastatin.

Determining resistance of a bacterial strain or population to an antibiotic can be conducted by any method known in the art for antibiotic susceptibility testing (AST). AST is widely used clinically to determine antibiotic resistance profiles of bacterial isolates, typically in a clinical microbiology laboratory, to guide antibiotic treatment decisions, and predict therapeutic outcome.

Illustrative, non-limiting examples of AST technologies include solid media culture tests, such as agar dilution assay (bacteria inoculated on agar plates with antibiotic discs of different concentrations), disk diffusion (bacteria inoculated on agar plates with a single antibiotic disk), E-test (bacteria inoculated on agar plates with a graded antibiotic concentration strips), liquid media culture tests, such as broth dilution assay (bacteria inoculated in liquid media with different antibiotics to monitor growth). Bacterial growth in liquid media can be determined for instance by measuring turbidity using a spectrophotometer (see for instance, Jorgensen JH, Ferraro MJ. Antimicrobial susceptibility testing: a review of general principles and contemporary practices. Clin Infect Dis 2009; 49:1749-55). Possible AST technologies encompass present and future methods, including any multiplexed and automated versions thereto.

The effect of the statins in reducing or reversing resistance may be assayed as described herein and the efficiency of the statin compound in combination with a selected beta-lactam antibiotic against selected microorganisms may be expressed as the MIC value, DR ratio and/or the FIC index.

The Minimal Inhibitory Concentration, (MIC) is defined as the lowest inhibitory concentration showing no visible growth according to the NCCLS Guidelines. The Drug Resistance (DR) ratio is defined as the ratio between the MIC value for anti- infective agent alone divided by the MIC for the anti-infective agent in the presence of the chemosensitising compound (e.g. the statin). This ratio represents the increase in apparent potency of the anti-infective agent caused by the chemosensitising compound, and may be expressed as

$$\text{DR ratio} = (\text{MIC anti-infective agent})/(\text{MIC anti-infective agent} + \text{chemosensitising compound}).$$

The Fractional Inhibitory Concentration (FIC) index may be calculated for each anti- infective agent alone and in combination with chemosensitising according to the following formulae:

$$FIC = FIC_{\text{chemosensitising compound}} + FIC_{\text{anti-infective agent}}$$

where:

$$FIC_{\text{chemosensitising compound}} = (MIC_{\text{chemosensitising compound}} + \text{antl-infective agent})/(MIC_{\text{chemosensitising compound}})$$

$$FIC_{\text{ anti-infective agent}} = (MIC_{\text{anti -infective agent + chemosensitising compound}}/(MIC_{\text{anti-infective agent}})$$

In a particular embodiment, optionally in combination with one or more of the features or embodiments as described herein, the reduction, mitigation or reversal of resistance in said bacterial population to said beta-lactam antibiotic is of at least 60%, preferably of at least 70%, 75%, 80%, 85%, 90%, 95%, 97%, 98%, 99% or more preferably of 100%.

In the method of treatment of the invention, said statin of formula (I) or (Ia) is preferably administered at least 15 minutes before, at least 30 minutes before, preferably at least 1 hour before the administration of said beta-lactam antibiotic, such as 2, 3 , 4 or 5 hours before, up to one or more days before.

In another aspect, the present invention relates to a pharmaceutical composition comprising a beta-lactamase resistant penicillin a statin of formula (I) or (Ia), and a pharmaceutically acceptable excipient or carrier, for use in a method of treatment according to the invention.

The expression "pharmaceutically acceptable excipient or carrier" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio. Likewise, the term "veterinary acceptable" means suitable for use in contact with a non-human animal. Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention. The formulations of the pharmaceutical compositions described herein may be prepared by any method known or hereafter developed in the art of pharmacology. In general, such preparatory methods include the step of bringing the active ingredient into association with a excipient and/or one or more other accessory ingredients, and then, if necessary and/or desirable, shaping and/or packaging the product into a desired single- or multi-dose unit. The administration route of the compounds described herein may be any suitable route that leads to a concentration in the blood or tissue corresponding to a clinically relevant concentration. Thus, e. g., the following administration routes may be applicable although the invention is not limited thereto: the oral route, the parenteral route, the cutaneous route, the percutaneous route, the nasal route, the topical route, the rectal route, the vaginal route and the ocular route. It should be clear to a person skilled in the art that the administration route is dependant on the particular compound in question, particularly, the choice of administration route depends on the physico-chemical properties of the compound together with the age and weight of the patient and on the particular disease or condition and the severity of the same. In general, however, the oral and the parenteral routes are preferred. The compounds described herein may be contained in any appropriate amount in the pharmaceutical composition, and are generally contained in an amount of about 0.1-95% by weight of the total weight of the composition. The composition may be presented in a dosage form, such as a unit dosage form, which is suitable for the oral, parenteral, rectal, cutaneous, percutaneous, nasal, topical, vaginal and/or ocular administration route. Thus, the composition may be in form of, e. g., tablets, capsules, pills, powders, granulates, suspensions, emulsions, solutions, gels including hydrogels, pastes, ointments, creams, plasters, drenches, delivery devices, suppositories, enemas, injectables, implants, sprays, aerosols and in other suitable form.

The pharmaceutical compositions may be formulated according to conventional pharmaceutical practice, see, e. g., "Remington's Pharmaceutical Sciences" and "Encyclopedia of Pharmaceutical Technology", edited by Swarbrick, J. & J. C. Boylan, Marcel Dekker, Inc. , New York, 1988. Typically, the compounds described herein are formulated with (at least) a pharmaceutically acceptable carrier or excipient. Pharmaceutically acceptable carriers or excipients are those known by the person skilled in the art.

In an additional aspect, the present invention refers to a pharmaceutical kit comprising:

iii. a pharmaceutical composition comprising a beta-lactamase resistant penicillin, and
iv. a pharmaceutical composition comprising statin of formula (I) or (Ia), for use in a method of treatment as described herein.

Said pharmaceutical kit optionally comprises instructions for the combined administration of the active ingredients as described herein.

In a further aspect, the method and compositions of the invention can also be applied for disinfecting surfaces, including but not limited to medical devices such as joint replacements and other types of orthopaedic instrumentation, prosthetic heart valves, pacemakers, implantable defibrillators, urinary catheters and stents, peritoneal dialysis catheters, intravascular catheters, cerebrospinal fluid shunts, breast implants, and vascular grafts and stents.

It is contemplated that any features described herein can optionally be combined with any of the embodiments of any medical use, pharmaceutical composition, kit, method of treatment, method of manufacturing a medicament and combination therapies of the invention; and any embodiment discussed in this specification can be implemented with respect to any of these. It will be understood that particular embodiments described herein are shown by way of illustration.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises"

also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, 10, 11, 12, 13, 14 or 15%. Accordingly, the term "about" may mean the indicated value ± 5% of its value, preferably the indicated value ± 2% of its value, most preferably the term "about" means exactly the indicated value (± 0%).

The following examples serve to illustrate the present invention.

EXAMPLES

Zaragozic acid is not a statin presently claimed and is thus considered to be a reference compound in these examples.

## EXAMPLE 1

### Material and Methods

#### Bacterial strains

All bacterial strains used in this study are listed in Key Resource Table. The methicillin-resistant *Staphylococcus aureus* (MRSA) strain USA300 (McDougal et al., 2003) was used for all experiments unless otherwise stated. For cloning purposes, *Escherichia coli* strain DH5α was used and S. *aureus* strain RN4220 served as the recipient for S. *aureus.*

#### Bacterial growth conditions

S. *aureus* strains were cultured in TSB medium supplemented with erythromycin (2 μg/ml) or kanamycin (10 μg/ml) when appropriate; *E. coli* strains were cultured in LB medium with ampicillin (100 μg/ml) when required. To avoid precipitation in aqueous solution, statins were prepared in dimethylsulfoxide (DMSO) stock solution and diluted 1:1 in methanol before addition to S. *aureus* cultures.

#### *In vivo* experiment conditions

For *in vivo* experiments using animal models, all animal studies were approved by the local government of Lower Franconia, Germany (license n° 55.2-DMS-2532-2-57) and performed in strict accordance with the guidelines for animal care and animal experimentation of the German animal protection law and directive 2010/63/EU of the European parliament on the protection of animals used for scientific purposes. Female BALB/c mice (8-10 weeks old, 16-19 g weight) were purchased (Charles River Laboratories), Mice were housed in polypropylene cages in standardized lighting conditions and had *ad libitum* access to food and water.

**Method details**

**Construction of knock-out mutants**

Δ*crt* and Δ*floA* mutants were generated using a two-step recombination process as reported in (Arnaud et al., 2004). Briefly, Δ*crt::km* and Δ*floA::spc* deletion cassettes were generated by using long-flanking-homology PCR and cloned into pMAD plasmid (Arnaud et al., 2004).

The resultant plasmids were inserted into S. *aureus* RN4220 by electroporation and propagated in TSB medium (30°C, 200 RPM) with 1 mM IPTG. After 4 h of incubation, the cultures were shifted for two more hours to 42°C and then plated onto TSB complemented with the antibiotic cassette (kanamycin or spectinomycin) and X-Gal. After 48 h of incubation at 42°C, the blue colonies still carrying the plasmid were discarded. White colonies resistant to kanamycin or spectinomycin were checked by PCR to confirm that the deletion cassette was integrated. φ11 phage lysates were generated from S. *aureus* RN4220 to infect USA300LAC. Clones resistant to kanamycin or spectinomycin were further verified using PCR. Resulting constructs were verified by DNA sequencing. Primers are listed in supplemental dataset S14.

**Generation of labeled strains**

FloA-YFP, FloA-SNAP and FloA-$His^6$ labeled strains were generated using pAmy and pLac plasmids (Yepes et al., 2014). All strains used bore the genetic constructs chromosomally integrated in *amyE* or *lacA* neutral loci of S. *aureus.* Sequence-validated plasmids were transformed in RN4220 and transduced into USA300 using phage ϕ11. Plasmids were integrated into *amyE* or *lacA* as above (Arnaud et al., 2004).

**Flotillin expression and purification**

WT, ΔMAR, ΔPHB and ΔEA4 flotillin variants were overexpressed and purified using pET20b vector (Novagen). Primers are listed in supplemental dataset S4. This plasmid bears a C-terminal His-Tag sequence and a T7 promoter, allowing induction of gene expression using IPTG. For overexpression, plasmids were transformed in *E. coli* BL21-Gold (Novagen). Protein expression was induced with 0.5 mM IPTG and cultures were grown 5 h at 30°C. Cell pellets were resuspended in 50 mM Tris-HCl pH 8, NaCl 200 mM, 5% glycerol buffer containing 1 mM PMSF and protease inhibitors (Roche) and disrupted using a French press. Cell extracts were incubated (1 h, 4°C) with 0.5% DDM (n-docecyl β-D-maltoside) to solubilize membrane proteins and 1% streptomycin sulfate to precipitate DNA, followed by ultracentrifugation (1 h, 100,000 g) to clear the lysate. Lysates were filtered (0.2 μm) and passed through a Ni-NTA agarose resin (Qiagen), which was washed and His-tagged proteins eluted with 250-500 mM imidazole; protein purity was analyzed by SDS-PAGE. Proteins were dialyzed to remove imidazole and kept at -20°C. Purification and dialysis buffers contained 0.02% DDM.

**Cell fractionation and purification of DRM**

Pellets of S. *aureus* TSB cultures were harvested and resuspended in PBS buffer supplemented with 1 mM phenylmethylsulfonylfluoride (PMSF) and 5 μl DNaseI (2000 U/ml). For enzyme lysis of cells, 15 μl lysostaphin (1 mg/ml) was added and cells incubated (15 min, 37°C). Cell suspensions were disrupted using a French press. Unbroken cells and debris were removed by centrifugation (10 min, 10,000 g, 4°C) and supernatant ultracentrifuged (1 h, 100,000 g) to separate the membrane fraction. The pellet was dissolved (overnight, 4°C) in 100-200 μl lysis and separation buffer (Sigma). For FMM isolation, the membrane fraction was processed using the CellLytic MEM protein extraction kit (Sigma) (Lopez and Kolter, 2010). Detergent-resistant (DRM) and -sensitive (DSM) fractions were separated according to the manufacturer's protocol. Samples were analyzed by SDS-PAGE.

**Lipid analysis by UPLC-ESI-qTOF-MS**

After drying samples in vacuum at 60°C, total lipids were isolated from DRM and DSM samples in 100 μl isopropanol. Samples were analyzed using ultra-performance liquid chromatograph coupled to a quadrupole/time-of-flight hybrid mass spectrometer equipped with electrospray ionization source (UPLC-ESI-qTOF-MS). Processing of chromatograms, peak detection and integration were performed using MassLynx software (version 4.1, Waters). Pro-Genesis QI 2.0 (Waters) was used for data preprocessing for untargeted lipidomics and markers were identified with univariate statistical analysis (ANOVA).

### Purification of staphyloxanthin lipids

*Staphylococcus aureus* culture pellets were resuspended in 10 ml methanol and incubated (30 min, 60°C) with continuous agitation. Samples were centrifuged to remove cell debris and the pigment-containing supernatant concentrated in a speedvac. Carotenoids were extracted with 2 ml ethyl acetate/1.7 M NaCl (1:1 v/v). After centrifugation, the ethyl acetate phase containing carotenoids was recovered and the aqueous phase was re-extracted with 0.5 ml ethyl acetate (Pelz et al., 2005). The extract was dried *in vacuo* and the carotenoid powder stored at -20°C.

To purify staphyloxanthin, total carotenoid lipids were resuspended in chloroform and separated by TLC (see below). The staphyloxanthin band (Marshall and Wilmoth, 1981) was scratched off the TLC plates and extracted with 4 ml ethyl acetate/1.7 M NaCl (1:1 v/v) as above. Staphyloxanthin purity was confirmed by analyzing its mass by ESI-TOF-MS and its absorbance by UV-spectrum measurements.

To compare the carotenoid composition of FMM-enriched and -depleted fractions, carotenoid lipids were purified from DRM and DSM fractions by methanol/chloroform/water extraction (1/1/2). DRM and DSM fractions were resuspended in 1 vol $H_2O$, mixed with 1 vol chloroform and of methanol and centrifuged to separate organic and aqueous phases. The carotenoid-containing chloroform phase was dried *in vacuo* and stored at -20°C.

### Thin-layer chromatography (TLC)

Purified lipids from DRM and DSM fractions were resuspended in chloroform and loaded on silica gel 60 plates (Merck). Pigments were resolved using hexane-acetone (60:40, v/v) as mobile phase. Bands corresponding to staphyloxanthin lipids are detected by their yellow color.

### Lectin binding assay

The DRM fraction isolated from S. *aureus* WT and Δ*crt* mutant were spotted on silica gel 60 plates (Merck-Millipore). Dried plates were blocked with 10% skim milk (TBS-Tween 0.05%, 1 h) and incubated with fluorescein-labeled lectins (Vector Laboratories) to detect sugar moieties, at a final concentration of 6.5 μg/ml in TBS-Tween 0.05% (overnight, 4°C, mild agitation). Plates were washed (TBS-Tween 0.05%, 2x 30 min) and fluorescence was detected with a Safe Imager blue-light transilluminator (Invitrogen). Samples were light-protected for all incubation steps. Images were analyzed with FIJI.

### Small unilamellar vesicle preparation (SUV)

Solutions of staphyloxanthin, PE (Sigma) and PG (Sigma) in chloroform (0.4 mg) were dried under nitrogen flow (1 min) and kept under vacuum for 2 h. Multilamellar vesicles (MLV) were obtained by hydration in 100 μl 50 mM Tris-HCI, 150 mM KCI pH 7.5 buffer (final lipid concentration 4 mg/ml). SUV were obtained by sonication of MLV (10-20 min or until the solution was transparent) (Martos et al., 2015). SUV were kept at -20°C.

### Bio-layer interferometry (BLI)

Lipid-protein interactions were measured by bio-layer interferometry using a single channel BLItz system (Forte-Bio). Staphyloxanthin, PG and PE SUV were sonicated (2 min) and diluted in hydration buffer as above to a final concentration of 1 mM. Flotillin variants were diluted ≥20-fold to a final concentration of 0.5 μM (0.001% DDM). Lipids were immobilized on aminopropylsilane biosensor tips, and flotillin variants were added to the biosensor to estimate the affinity constant ($K_D$) (room temperature, with vigorous shaking (2200 rpm)). Each binding reaction was constituted by a 30 s baseline (buffer), followed by a 300 s association phase (lipid or protein binding), and a 300 s dissociation phase (buffer only). BLItz Pro software was used to determine rate constants ($K_a$, $K_d$) for net association and dissociation, the equilibrium dissociation constant ($K_D = K_d/K_a$), and goodness of fit (Chi square $X^2$ and R-square $R^2$). Kinetic constants were calculated in the case of a good fit ($X^2 < 3$ [P>0.05] and $R^2 > 0.95$).

### Liposome-flotation binding assay

SUs of staphyloxanthin, PE and PG lipids were prepared as above, dispersed by sonication (1-2 min) and diluted in hydration buffer (1 mM final concentration). Flotillin variants (0.8 μg) were mixed with SUV and incubated (15 min, room temperature). The protein-lipid mixture was diluted 1:1 with 60% sucrose (w/v in 50 mM Tris-HCl pH 7.5, 200 mM NaCl) to form the bottom layer of the sucrose gradient (200 μl), which was overlaid with 250 μl 25% sucrose (w/v, same buffer) and a top layer of 100 μl buffer (0% sucrose). Samples were centrifuged (270,000 g, 1 h, 4°C). In a control experiment, we used Nile Red (0.1 mg/ml) to stain and localize lipids in the gradient; lipids localized in the top fraction.

Top fractions were analyzed by immunoblot to detect flotillin variants.

**Sucrose gradients**

FloA oligomerization in S. *aureus* WT and Δ*crt* mutant and PBP2a oligomerization were analyzed in sucrose gradient assays. S. *aureus* cultures were harvested and cells crosslinked with 0.5 mM DSP (dithiobis(succinimidyl propionate)) before cell lysis and membrane fraction purification. Purified membrane fractions (200 μg) solubilized with 0.5% DDM were layered on a 5-40% linear sucrose gradient generated by a Biocomp gradient maker, then centrifuged (Beckman; 100,000 g, 16 h, 4°C). Fractions (1 ml) were collected from the bottom of the gradient and proteins associated with each fraction were precipitated with 10% trichloroacetic acid (TCA). Protein fractions were dried and resuspended in PBS prior to immunoblot analyses.

**Size exclusion chromatography**

Purified WT flotillin and variants were adjusted to concentrations of -60 μM and separated by size exclusion chromatography (Superose 6 increase 10/300 GL size-exclusion column; GE Healthcare) in an Äkta pure high-performance liquid chromatography (HPLC) system. For each size exclusion run, 500 μl protein sample was loaded onto a column equilibrated with buffer (300 mM NaCl, 50 mM Tris-HCl pH 8.0, 10% glycerol, 0.02% DDM) and run at a 0.4 ml/min constant flow rate. Protein elution profiles were compared by normalizing UV absorbance of the chromatograms and the graphs overlaid using PRISM. A set of standard proteins was used to calibrate the gel filtration column (Sigma-Aldrich MWGF1000-1KT).

**Fluorescence microscopy**

Cells from liquid cultures were washed in PBS and resuspended in 0.5 ml 4% paraformaldehyde (6 min, room temperature). Samples were washed twice and resuspended in 0.5 ml PBS. Images were acquired on a Leica DMI6000B microscope equipped with a CRT6000 illumination system, with a HCX PL APO oil immersion objective (100×1.47) and a color camera DFC630FX. Leica Application Suite Advanced Fluorescence Software was used for linear image processing. The YFP signal was detected (excitation filter 489 nm, emission filter 508 nm). Excitation times were 567 msec. Transmitted light images were taken with 55 msec excitation time.

**Direct stochastic optical reconstruction microscopy (dSTORM)**

SNAP-tagged cells were incubated in SNAP buffer (250 mM Tris-HCl pH 7.5, 500 mM NaCl, 5 mM DTT (dithiothreitol)) and a final concentration of 0.8 μM SNAP-Cell TMR-Star dye (NEB; 30 min, 37°C in the dark). dSTORM was performed using an ELYRA S.1 super-resolution microscope (Zeiss) equipped with a 100x oil-immersion objective. Image stacks were analyzed with open source rapidSTORM software. Cluster analyses were performed by Python routine (Python 2.7.3, Python Software Foundation). Clusters were defined by one connected pixel area in image-based analysis or by a cloud of scattered localizations with spatial coherence, by calculating the standard deviation of the localization cloud from its center of mass.

**Correlative light and electron microscopy (CLEM)**

For high pressure freezing, overnight S. *aureus* cultures were pelleted and resuspended in 5% bovine serum albumin. Cell suspensions were pipetted into freezing plates (Leica Microsystems), cryoimmobilized, and processed. Briefly, cells were freeze-substituted, fixed with $KMnO_4$ and embedded in LR white resin (London Resin Co.). Ultrathin 100-nm sections were mounted on glass slides and immunostained. For FloA labeling, we used polyclonal anti-FloA and Cy3-conjugated secondary antibody and for PBP2a, polyclonal anti-PBP2a (RayBiotech) and Cy5-conjugated secondary antibody. Detailed antibody information is found in the Key Resource Table. For fluorescence microscopic analyses, we used the ELYRA S.1 super-resolution structured illumination microscope. For SEM imaging, we used a field emission scanning electron microscope JSM-7500F (JEOL) with a LABE detector (for back-scattered electron imaging) at acceleration voltage 5 kV, probe current 0.3 nA, and a working distance of 6-8 mm. Image processing and correlation were as described (Markert et al., 2016).

**Electron tomography**

Samples were processed as for CLEM. High pressure frozen pellets were freeze-substituted, embedded, and processed (Helmprobst et al., 2015). Electron tomography was performed as described (Helmprobst et al., 2015) with

modifications. Tilt series were acquired using an electron microscope JEM-2100 (JEOL) at 200 kV equipped with a F416 digital camera (TVIPS; 4096 × 4096 pixel resolution) automated with Serial EM software. Tilt angle ranges varied from -65°/65° and -70°/70°. Tilt series were acquired systematically in 1° increments. For reconstruction of electron tomograms and segmentation, we used eTomo/IMOD.

**Immunogold FloA detection**

Fixed S. *aureus* cells were cryoimmobilized by rapid immersion in -170°C ethanol using Leica CPC equipment, and progressively cryosubstituted in -40°C methanol/0.5% uranyl acetate before embedding in Lowicryl HM20 resin and thin-sectioning (60 nm) with a Leica EM UC6 ultramicrotome. For immunogold labeling, grids were incubated with chicken anti-FloA antibody (1:10) and gold-conjugated rabbit anti-chicken antibody (BBI; gold particles 10 nm diameter, dilution 1:40). Samples were negatively stained with 2% uranyl acetate and lead citrate, and images acquired with a JEM 1011 transmission electron microscope (JEOL; 100 kV) and a Gatan Erlangshen ES1000W camera.

**Lipid-protein complex analysis by electron microscopy**

Staphyloxanthin, PE and PG SUV were dispersed in a sonication bath and diluted in reaction buffer (50 mM Tris-HCl pH 8, 200 mM NaCl). Purified FloA (0.6 μM, 0.001% DDM) was incubated alone or with 0.4 mM of each lipid sample (30 min, 37°C). The reaction mixture (5 μl) was applied to a copper grid before uranyl acetate staining and visualized using the JEM 1011 transmission electron microscope.

**Label-free protein quantification by mass spectrometry**

For sample preparation, proteins were reduced in 1x LDS sample buffer (Thermo Scientific) with 50 mM DTT (5 min, 95°C), alkylated with 120 mM iodoacetamide, precipitated with acetone and dissolved in 0.5% sodium deoxycholate (SDC; Sigma). Samples were digested with 0.5 μg LysC (Wako) and 0.5 μg trypsin (Promega). SDC was removed by ethyl acetate extraction, desalted with C18 stage tips, and dissolved in 2% acetonitrile.

NanoLC-MS/MS analyses were performed on an Orbitrap Fusion instrument equipped with an EASY-Spray ion source and coupled to an EASY-nLC 1000 (Thermo Scientific). Peptides were loaded on a trapping column (2 cm × 75 μm ID, PepMap C18, 3 μm particles, 100 Å pore size) and separated on an EASY-Spray column (25 cm × 75 μm ID, PepMap C18, 2 μm particles, 100 Å pore size) with a 120-min linear gradient from 3-32% acetonitrile and 0.1% formic acid. MS scans were acquired in the Orbitrap analyzer at a resolution of 120,000 at m/z 200.

For data analysis, MS raw data file processing, database searches and quantification, we used MaxQuant. The search was performed against a S. *aureus* database derived from UniProt; a database containing common contaminants was also used. For protein quantitation, LFQ intensities were used. Proteins with less than two identified razor/unique peptides were dismissed. Further data analysis was done with in-house-developed R scripts. Missing LFQ intensities in control samples (total lysate) were imputed with values near the baseline.

**Bacterial two-hybrid analysis**

Bacterial two-hybrid analysis was used to quantify the interaction between FloA and PBP2a. The coding sequences were cloned into the bacterial two-hybrid expression vectors (EuroMedex) to generate N- and C-terminal fusions to the catalytic domains T25 and T18 of *Bordetella pertussis* adenylate cyclase. Pairwise combinations of plasmids were then cotransformed in E. *coli* BTH101 strain, which harbors a *lacZ* gene under the control of a cAMP-inducible promoter. After interaction, the T25 and T18 catalytic domains of the adenylate cyclase form an active enzyme, leading to cAMP production and hence to reporter expression. Plates were incubated (48 h, 30°C). pKT25-zip and pUT18C-zip, as well as pKT25 and pUT18C, served as positive and negative controls, respectively. For quantitative measurements, β-galactosidase levels were determined and results shown in Miller units.

**Bacterial three-hybrid analysis (B3H assay)**

PBP2a and PBP2, RodA, PrsA, and FtsZ were cloned in pKNT25 and pUT18 vectors (Euromedex). A cotransformed strain was used for protein-protein interaction assays to determine PBP2a interaction efficiency with PBP2, RodA, PrsA, or FtsZ. These strains were subsequently transformed with pSEVA modulable plasmids (Silva-Rocha et al., 2013) that bear distinct replication origins and propagate in *E. coli* at low, medium and high copy numbers (10 μg/ml gentamicin) (Schneider et al., 2015). Experiments that required propagation of pSEVA vectors were performed in LB medium with 100 μg/ml ampicillin and 10 μg/ml gentamicin. For quantitative measurements, β-galactosidase levels were determined and results shown in Miller units.

## Pull-down assay

Pull-down assays were performed in 1.5 ml reaction tubes and samples kept at 4°C throughout the experiment; 250-1000 μl Ni-NTA resin (Qiagen) was used in reactions. The volume needed depends on expression of the bait protein and must be determined empirically. Proteins were bound to resin (1 h, 4°C). To remove supernatant after each step, samples were centrifuged (1000 g, 2 min). To remove unbound and non-specific protein, the resin was washed twice with low-imidazole buffer. For elution, 100 μl elution buffer was added twice; fractions were collected and protein content analyzed by LC-MS vs. the eluted fraction of an unlabeled protein extract.

## Blue-native PAGE (BN-PAGE)

Cultures were grown in TSB medium (24 h, 200 rpm). Cells were harvested and the pellet dissolved in PBS buffer containing 1 mM PMSF and cOmplete protease inhibitors (Roche). Samples were crosslinked with 0.5 mM DSP before cell lysis and fractionation. The membrane fraction (-80 μg) was solubilized in 1x Native PAGE sample buffer (Invitrogen) with 0.5% DDM (overnight, 4°C). Solubilized membranes were mixed with Coomassie dye G-250 and loaded on a native gel with a 3-12% polyacrylamide gradient (Invitrogen). BN-PAGE was carried out according to Wittig et al. (Wittig et al., 2006). BN-PAGE uses Coomassie G-250 to confer a negative charge to proteins and allows resolution of the oligomeric complexes according to their native state. Immunoblotting was used to detect FloA and PBP2a using specific polyclonal antibodies.

## Western blot analysis and immunodetection

80 μg total protein was separated in 12% SDS-PAGE. Proteins were transferred to a PVDF membrane by semi-dry blotting (2 h), which was blocked with 5% skim milk and human serum 2% (1 h) and probed with antibodies listed in Key Resource Table. Proteins were detected using a chemiluminescent substrate kit (Thermo Scientific) and recorded with the ImageQuant LAS4000 illumination system (General Electric).

## *In vitro* MRSA cell growth inhibition

It was determined the bacterial count (CFU/ml) of MRSA cultures (i.e., *Staphylococcus aureus* MRSA strain USA300 (McDougal et al., 2003)) treated with a combination of a statin and a beta-lactam antibiotic.

USA300 was cultured in a TSB plate at 37°C O/N. Biomass was collected and resuspended in 1 ml TSB medium (it typically gets an OD600 of 7-10). This pre-inoculum was used to inoculate cultures at OD600 ≅ 0.05.

To avoid precipitation in aqueous solution, statins were prepared in dimethylsulfoxide (DMSO) stock solution and diluted 1:1 in methanol before addition to S. *aureus* cultures. Cultures were inoculated using TSB medium that was previously conditioned with the statin at different concentrations and distributed in 96 well-plates. 200 μl were added to each well. Then the beta-lactam antibiotic was added to the well plates at different concentrations, namely, concentrations from 1 to 32 μg/ml of oxacillin were used.

The plates were incubated 24h at 37°C in a manner to avoid diseccation, e.g., by filling the wells in the edge of the well with water. The day after, the cultures were collected from the wells and perform serial dilutions to calculate the CFU/ml.

## Mouse infection studies

The S. *aureus* strains were cultured on BHI medium (18 h, 37°C). Cells were collected, washed three times with PBS and diluted to $OD_{600\ nm}$ = 0.05. Viable cell counts were determined by plating inoculum dilutions on TSB agar plates. For survival experiments, cohorts of 10 mice were infected with 150 μl of S. *aureus* cultures ($3 \times 10^7$ cells) via intravenous injection. Each strain was used to infect one mouse cohort. Doses of oxacillin (200 mg/kg) alone or in combination with ZA (50 mg/Kg or 20 mg/Kg) were injected intraperitoneally (i.p.) daily for 1-4 days. ZA concentration was chosen according to concentrations used in studies to evaluate statins as cholesterol-lowering agents in mice (Ghodke et al., 2012). The first dose was administered 30 min after the bacterial inoculation. Infections were allowed to progress until severe infection signs occurred or to a 3-day endpoint. Mice were sacrificed when they met the following criteria: 1) loss of at least 20% body weight, 2) loss of at least 15% body weight and ruffled fur, 3) loss of at least 10% body weight and hunched posture, or 4) 4 days post-infection. For bacterial load experiments, cohorts of 10 mice were instilled with S. *aureus* suspension ($3 \times 10^8$ cells) into left nare of anesthetized mice. Doses of oxacillin (200 mg/kg) alone or combined with ZA (50 mg/Kg or 20 mg/Kg) were injected i.p. daily for 1-2 days. The first dose was administered 30 min after bacterial inoculation. Infection was allowed to progress for two days. Mice were sacrificed and lungs collected aseptically, homogenized in 2 ml sterile PBS in GentleMACS M Tubes (Miltenyi Biotec); serial dilutions were plated on

mannitol-agar plates and incubated (24 h, 37°C).

**Software used in this study**

Protein MS data were analyzed with MaxQuant (http://www.coxdocs.org/doku.php?id=maxquant:start) and deposited in ProteomeXchange via the Pride Partner repository (http://www.ebi.ac.uk/pride/archive). For lipidomics assays, we used MassLinx (http://www.waters.com/waters/en US/MassLynx-Mass-Spectrometry-Software-/nav.htm?cid=513164). Data were analyzed with Progenesis software (http://www.nonlinear.com/progenesis/qi). To quantify the fluorescent signal in fluorescence microscopy images, we used the LAS Leica Application Suite (http://www.leica-microsystems.com/products/microscope-software); for processing, modeling and display programs for tomographic and 3D reconstruction of EM serial sections, we used IMOD software (http://bio3d.colorado.edu/imod).

**Quantification and Statistical Analysis**

**Statistical analyses**

All statistical analyses were performed using Sigma-Plot software (Systac Software). Graphs represent data from at least three independent biological replicates. Each biological replicate represents three independent technical replicates (n = 3). Data are shown as mean ± SEM. For analysis of experiments with two groups, we used the parametric unpaired two-tailed Student's t-test with Welch's correction and the non-parametric unpaired Mann-Whitney test. For analysis of experiments with three or more groups, the parametric one-way ANOVA test was used. *Post hoc* analysis included multiple comparisons Tukey's test, Dunnett's test or Dunn's tests, depending on the data set. Differences were considered significant when p was <0.05. ns = not significant, * p<0.05, ** p<0.01, *** p<0.001.

**Data and Software Availability**

**Data availability**

Original unprocessed images (gels and western blots, microscopy images and movies) have been deposited in Mendeley data (https://data.mendeley.com/) under the Reserved DOI: doi:10.17632/zr92hyx6y5.1.
The mass spectrometry proteomics have been deposited at the ProteomeXchange Consortium via the Pride Partner Repository, with the dataset identifier PDX00654C.

**Key Resource Table**

| REAGENT or RESOURCE | SOURCE | IDENTIFIER |
|---|---|---|
| **Antibodies** | | |
| α-FloA chicken | Davids | This study |
| α-FLAG rabbit | Sigma | Cat#F7425 |
| α-GroEL rabbit | Sigma | Cat#G6532 |
| α-chicken HRP-conjugated secondary | Thermo Scientific | Cat#A1654 |
| α-rabbit HRP-conjugated secondary | BioRad | Cat#1706515 |
| α-PBP2a rabbit | RayBiotech | Cat#130-10073-100 |
| α-rabbit-Cy5 conjugated secondary | Abcam | Cat#Ab97077 |
| α-chicken-Cy3 conjugated secondary | Abcam | Cat#Ab97145 |
| α-chicken-Gold 10 nm diameter conjugated secondary | BBI solutions | Cat#EM.RCHL.10 |
| α-chicken Alexa Fluor 488 conjugated secondary | Thermo Scientific | Cat#A-11029 |
| **Bacterial and Virus Strains** | | |
| List of *E. coli* strains related to protein production, B2H and B3H assays is in supplemental table S14 | This study | N/A |
| *S. aureus* ST24R | (Lopez-Collazo et al., 2015) | N/A |
| *S. aureus* USA300LAC (WT) | (McDougal et al., 2003) | N/A |
| *S. aureus* Δ*floA* | This study | N/A |
| *S. aureus* Δ*crt* | This study | N/A |
| *S. aureus* Δ*mecA* (Δ*pbp2a*) | This study | N/A |
| *S. aureus* WT FloA-YFP | This study | N/A |
| *S. aureus* Δ*crt* FloA-YFP | This study | N/A |
| *S. aureus* WT MAR-YFP | This study | N/A |
| *S. aureus* WT PHB-YFP | This study | N/A |
| *S. aureus* WT EA4-YFP | This study | N/A |

| | | |
|---|---|---|
| *S. aureus* WT FloA-SNAP | This study | N/A |
| *S. aureus* WT FloA-His6 | This study | N/A |
| *S. aureus* WT FloA-FLAG | This study | N/A |
| **Chemicals, Peptides, and Recombinant Proteins** | | |
| TLC silica gel 60 | Merck | Cat#1.05559.0001 |
| Lectin Kit Fluorescein | Vector Laboratories | Cat#FLK-2100 |
| Fluorescein *Solanum tuberosum* (potato) Lectin | Vector Laboratories | Cat#FL-1161 |
| Superose 6 increase 10/300 GL column | General Electric | Cat# 29-0915-96 |
| Phosphatidylethanolamine | Sigma | Cat#P8068 |
| Phosphatidylglycerol | Sigma | Cat#P8318 |
| Uranyl acetate | Electron Microscopy Sciences | Cat#22400 |
| TMR-Star | New England Biolabs | Cat#S9105S |
| Ni-NTA resin | Qiagen | Cat#30210 |
| n-Dodecyl β-D-maltoside | Glycon Biochem | Cat#D97002-C |
| cOmplete Protease Inhibitor Cocktail | Roche | Cat#11697498001 |
| Zaragozic acid | Santa Cruz | Cat#144541-82-2 |
| Lovastatin | Axxora | Cat#LKT-M1687-M |
| Mevastatin | Axxora | Cat#LKT-M1685-M |
| Pravastatin | Axxora | Cat#LKT-P6801-M |
| Simvastatin | Axxora | Cat#LKT-S3449-M |
| Atorvastatin | Axxora | Cat#LKT-A7658-M |
| Fluvastatin | Axxora | Cat#LKT-F4482-M |
| Oxacillin | Sigma | Cat#28221 |
| Methicillin | Sigma | Cat#51454 |

| | | |
|---|---|---|
| Flucoxacillin | Sigma | Cat#SML1023 |
| Nafcillin | Sigma | Cat#N3269 |
| Dicloxacillin | Sigma | Cat#D9016 |
| **Critical Commercial Assays** | | |
| MEM Cell Lytic assay | Sigma | Cat#CE0050 |
| BACTH System Kit | EuroMedex | Cat#EUK001 |
| Chemiluminescent substrate Kit | Thermo-Scientific | Cat#34080 |
| Native polyacrylamide gel system | Invitrogen | Cat# BN1001BOX |
| **Experimental Models: Organisms/Strains** | | |
| Mice: BALB/C | Charles River Laboratory | Strain code 028 |
| **Oligonucleotides** | | |
| See supplemental table S14 for list of primers used in this study | This study | N/A |
| **Recombinant DNA** | | |
| pET20b | Novagen | Cat#69739-3 |
| pSNAPf | New England Biolabs | Cat#N9183S |
| pMAD | (Arnaud et al., 2004) | N/A |
| pLac | (Yepes et al., 2014) | N/A |
| pAmy | (Yepes et al., 2014) | N/A |
| pSEVA621 | (Silva-Rocha et al., 2013) | N/A |
| pSEVA631 | (Silva-Rocha et al., 2013) | N/A |
| pSEVA641 | (Silva-Rocha et al., 2013) | N/A |
| **Software and Algorithms** | | |
| LAS (Leica Application suite) | Leica | http://www.leica-microsystems.com/products/microscope-software/ |
| FIJI | SciJava | https://fiji.sc |

| | | |
|---|---|---|
| IMOD | University of Colorado | http://bio3d.colorado.edu/imod/ |
| MaxQuant | Max Planck Institute for Biochemistry | http://www.coxdocs.org/doku.php?id=maxquant:start |
| MassLynx | Waters | http://www.waters.com/waters/en_US/MassLynx-Mass-Spectrometry-Software-/nav.htm?cid=513164&locale=en_US |
| ProGenesis QI | Waters | http://www.nonlinear.com/progenesis/qi/ |
| **Other** | | |
| Proteomic data, analyses and resources related to proteomic analysis to total membrane DRM and DSM proteome | This paper | ProteomeXchange<br>http://www.ebi.ac.uk/pride<br>Identifier: PXD006546 |
| Original unprocessed images (gels, western blots, microscopy images and movies) | This paper | Mendeley data<br>https://data.mendeley.com/<br>Reserved DOI: doi:10.17632/zr92hyx6y5.1. |
| Ultra-performance liquid chromatograph coupled to a quadrupole/time-of-flight hybrid mass spectrometer equipped with electrospray ionization source (UPLC-ESI-qTOF-MS) | Synap | G2 HDMS |
| BLItz system | ForteBio | - |
| Size exclusion chromatography | General Electric Healthcare | Akta Pure |
| Fluorescence microscope | Leica | DMI6000B |
| dSTORM | ZEISS | Elyra S.1. |

| | | |
|---|---|---|
| Electron Microscope (CLEM) | JEOL | JSM-7500F |
| Electron Microscope (tomography) | JEOL | JEM-2100 |
| Electron Microscope (Immunogold) | JEOL | JEM 1011 |
| NanoLC-MS/MS | Thermo Scientific | Orbitrap Fusion instrument equipped with an EASY-Spray ion source and coupled to an EASY-nLC 1000 |

**Results**

**Constituent lipids of FMM are carotenoids**

Organization of FMM in bacteria relies on the scaffold protein flotillin and aggregation of isoprenoid lipids of yet unknown nature (Bramkamp and Lopez, 2015). To identify the constituent lipids and the mechanism of FMM assembly, we purified *Staphylococcus aureus* MRSA strain USA300LAC (McDougal et al., 2003) cell membranes. Given the different lipid composition and density of FMM, a FMM-rich sample can be obtained by exploiting their insolubility after treatment with nonionic detergents (0.5-1% Triton X-100, 4°C) prior to phase separation (Brown, 2002). This treatment generates a membrane fraction sensitive to detergent disaggregation (detergent-sensitive membrane; DSM) and another that is resistant to disruption with larger FMM-rich fragments (detergent-resistant membrane; DRM). Total lipids were extracted from DRM and DSM fractions and membrane lipids identified in untargeted lipidomics experiments using electrospray ionization (UPLC-ESI-qTOF-MS) (Fig. 8A and B). In all, 39 lipid species were unique in the DRM compared to the control sample (extraction solution with no cells). From the 39 peaks, intensities of 30 peaks were clearly higher in DRM than DSM; 7 were detected consistently in three independent biological replicates (n = 3) and were thus considered FMM lipid markers (fold change >100, Fig. 1A).

These 7 FMM lipid markers were annotated according to retention times (RT) and nominal mass-to-charge ratios (m/z) (Fig. 1A). All marker ions were doubly charged as the m/z difference of the isotope peaks were 0.50 and their low RT suggested polar characteristics (Fig. 8C). Product ion scan at negative ESI showed two common fragments of 556.28 and 574.29 m/z (Fig. 1B), which denotes that these marker lipids belong to the same class. No fragments of glycerol-3-phosphate ion (152.99 m/z), $H_2PO_4$- ion (96.97 m/z) or $PO_3$- ion (78.96 m/z) were detected in the MS/MS spectra, indicating that these are not membrane phospholipids. Elemental composition of these two peaks ($C_{27}H_{44}NO_{12}$ and $C_{27}H_{42}NO_{11}$, the same molecule with and without one $H_2O$) is consistent with that of a staphyloxanthin fragment in which the sugar backbone is decorated with an extra sugar. Product ion scan at positive ESI detected neutral losses of N-acetylglucosamine alone (NAG; 203.09) and with N-acetylmuramic acid (NAG-NAM; 478.30), thus categorizing FMM markers as staphyloxanthin-derived unphosphorylated saccharolipids in which fatty acids are linked directly to a NAM-NAG-containing sugar backbone.

Staphyloxanthin is an unphosphorylated saccharolipid of isoprenoid nature found in S. *aureus* membranes, which gives S. *aureus* its typical yellow color (Pelz et al., 2005). We used thin-layer chromatography (TLC) to identify staphyloxanthin pigmented bands in the DRM sample; no pigmented bands were detected in the DSM sample of WT or samples from a S. *aureus* Δ*crt* mutant that lacks the operon for staphyloxanthin biosynthesis (Fig. 1C). UV-visible spectroscopy of a FMM lipid sample showed peaks at 463 and 490 nm, typical of purified staphyloxanthin (Fig. 1D), which indicated that FMM lipids are staphyloxanthin derivatives. In immunodetection assays using labeled lectins, only wheat germ lectin (WGA) and *Solanum tuberosum* lectin (STL) detected positive signals specific for NAG and NAG-NAM, respectively (Fig. 1E and 9A). Using UPLC-ESI-qTOF-MS, we did not detect any of the 7 FMM lipid markers in the Δ*crt* mutant DRM or DSM fractions (Fig. 1F and 9B), which indicated that staphyloxanthin-derived NAG-NAM-decorated lipids are highly represented in the FMM. A tentative formula consistent with the fragmentation pattern is shown (Fig. 1G).

**Accumulation of flotillin and lipid markers drives FMM assembly**

Our current hypothesis states that FMM assembly requires lateral segregation of FMM lipids in specific microdomains together with recruitment of flotillin (FloA) to these regions. We used a translational fusion of flotillin to a yellow

fluorescence protein (FloA-YFP) to detect FloA organized in discrete dynamic foci distributed across the cell membrane in exponentially growing and stationary cells (Fig. 2A, B and 9C). While WT stationary cells showed an average of 5 foci/cell, the Δ*crt* mutant showed fewer foci (2 foci/cell). WT and Δ*crt* cultures in exponential growth showed reduced numbers of foci, as staphyloxanthin accumulates in cell membranes in the stationary phase (Kullik et al., 1998) (Fig. 9D). Foci were distributed on the membrane in no specific pattern, although dividing cells generally showed foci near the invaginations of the division septum. A small number of septal foci was also detected in the Δ*crt* mutant (Fig. 2B).

To evaluate FloA recruitment to staphyloxanthin-rich microdomains, we quantified staphyloxanthin-FloA interaction using lipid-protein flotation and binding assays with distinct FloA variants (Fig. 2C). In the flotation assay, FloA and lipids were mixed beneath a sucrose gradient. After ultracentrifugation, lipids migrated to the low sucrose density fraction at the top of the tube accompanied by FloA only when interaction occurred, as detected with FloA-specific antibodies (Fig. 2D). Purified WT FloA interacted strongly with purified staphyloxanthin, but not with phospholipids (phosphatidylglycerol, PG; phosphatidylethanolamine, PE). A ΔMAR variant that lacks the N-terminal membrane-anchoring region also interacted strongly with purified staphyloxanthin, as did a ΔEA4 variant altered in one of its four C-terminal glutamine-alanine (EA) repeats, which probably participate in protein-protein interaction (Schneider et al., 2015). A ΔPHB variant lacking the prohibitin domain (PHB), whose function is unknown and is found in all flotillin-related proteins (Bach and Bramkamp, 2015), showed no tendency to interact with staphyloxanthin.

In the binding assay, purified staphyloxanthin was immobilized on a biosensor tip, followed by bio-layer interferometry (BLI). As FloA variants bind to the lipids with different affinity, incident light directed through the biosensor shifts and creates a quantifiable interference pattern (Fig. 2E and 10A, B). WT, ΔMAR and ΔEA4 showed preferential affinity for staphyloxanthin compared to distinct membrane phospholipids ($K_D$ = 3.49·$10^{-8}$, 1.20·$10^{-8}$ and 9.43·$10^{-9}$ M, respectively), thus showing that FloA preferentially binds FMM-constituent lipids. In contrast, the ΔPHB variant showed no preference for staphyloxanthin over membrane phospholipids ($K_D$ = 9.43·$10^{-7}$ M). The flotillin PHB domain is thus responsible for the preferential interaction of flotillin with FMM-constituent lipids. Consequently, a YFP-labeled ΔPHB variant did not form membrane foci, but was distributed homogenously over the S. *aureus* membrane (Fig. 10C).

FloA recognition of FMM lipids and confinement in microdomains could promote FloA-FloA interaction and thus, FloA oligomerization in FMM. FloA oligomeric states were resolved by size-exclusion chromatography (Fig. 3A); we detected distinct peaks in the WT, ΔMAR and ΔPHB profiles that were attributable to different oligomeric states, ranging from a 35 kDa monomer to a >600 kDa oligomer. In contrast, the ΔEA4 profile showed no peaks corresponding to large oligomers, which indicated the importance of the C-terminal region in FloA multimerization. A YFP-labeled version of the ΔEA4 variant did not organize in membrane foci but showed homogenous distribution over the S. *aureus* membrane (Fig. 10C). Based on these results, we hypothesized that preferential staphyloxanthin-FloA binding, via the PHB domain, leads to FloA localization in staphyloxanthin-rich microdomains. The PHB domain is not involved in FloA oligomerization. FloA oligomerizes via a C-terminal interaction to organize FMM (Fig. 3B). To test this, we incubated purified FloA with purified staphyloxanthin- or phospholipid-containing suspensions and examined FloA oligomerization using electron microscopy (Fig. 3C and S4A). We detected large FloA assemblies only in the staphyloxanthin-containing sample. Control samples with staphyloxanthin or phospholipids alone did not form large assemblies in our assay conditions. Using *in vivo* approaches, we monitored FloA oligomerization efficiency in S. *aureus* purified membrane fractions of WT and Δ*crt* strains using blue-native (BN)-PAGE, which allows separation of membrane protein complexes in their natural oligomeric states (Wittig et al., 2006), or sucrose gradient centrifugation (5-40%), and we detected FloA by immunoblotting (anti-FloA antibody) (Fig. 3C). BN-PAGE showed a reduction in FloA-containing high molecular weight protein complexes in Δ*crt* samples. In the sucrose gradient, the FloA signal concentrated in the high-density fractions, where high MW protein complexes accumulated. In Δ*crt,* the FloA signal was detected in low-density fractions at the top of the tube, in which low MW protein complexes accumulated. These results are consistent with the hypothesis that FMM assembly is a two-step process in which flotillin is recruited to staphyloxanthin-rich membrane microdomains, where it oligomerizes to organize FMM.

**Visualization of FMM in cell membranes**

Using super-resolution array tomography (srAT) (Markert et al., 2016) to visualize FMM assemblies in cell membranes, FloA was immunodetected and coarsely located in 100-nm sectioned cells using structured illumination microscopy (SIM) (Fig. 4A and 11B). Next, samples were carbon-coated and imaged using scanning electron microscopy (SEM). Fluorescence and SEM images were correlated and FloA distribution reconstructed. Flotillin was distributed in ~5 foci on the cell membrane, with no defined distribution pattern except its usual localization at septal invaginations in dividing cells. FloA-localizing regions were further examined by transmission electron microscopy (TEM). Uranyl acetate is typically used for section staining, as this stain binds preferentially to phospholipid phosphate head groups and provides intense contrast to membranes (Hayat, 1993; Ting-Beall, 1980). Our flotillin-containing microdomains nonetheless showed poor contrast, and appeared as light electron-dense regions compared to the remainder of the membrane (Fig. 4B). This is consistent with enrichment of unphosphorylated staphyloxanthin lipids in the FMM, which causes only light

uranyl acetate staining of these microdomains, and with the lack of these light electron-dense regions in the Δ*crt* mutant (Fig. 11C). We confirmed this observation using TEM, in which we simultaneously detected colocalization of FloA using immunogold labeling, and light electron-dense membrane regions using uranyl acetate staining, in 40 to 200 nm membrane microdomains (Fig. 4C, D and 11D). This finding indicates the possibility of visualizing FMM using this approach.

Using super-resolution imaging by direct stochastic optical reconstruction microscopy (*d*STORM), we examined subcellular flotillin location (Fig. 5A). In *S. aureus* cells labeled with a SNAP-tagged FloA, switching of the fluorophore SNAP conjugate TMR-Star (tetramethylrhodamine) was used to reconstruct high-resolution images. As before, we found FloA located in membrane foci and near the septal invaginations of dividing cells. Approximately 25% of the FloA signal was detected in the cytoplasm surrounding the membrane foci, which suggested dynamic flotillin activity in these microdomains. Electron tomography was used to examine FloA-containing microdomains in greater detail (Fig. 5B-D). Cells were uranyl acetate-stained prior to performing the tilt series used to calculate 3D image tomograms. FloA-localizing regions were identified in the tomograms as 100-300 nm light electron-dense membrane areas that stained poorly with uranyl acetate, and showed accumulation of several nanodomains rather than a single uniform microdomain. The cytoplasmic area surrounding these membrane regions concentrated electron-dense small particles, previously characterized as large protein complexes (McQuillen, 1962; Palade, 1955). We did not detect concentration of these particles in Δ*floA* tomograms, which suggested that FMM engage in active protein organization, metabolism and/or trafficking.

**PBP2a is part of the FMM protein cargo**

Having determined that FMM could be involved in protein organization, we identified and quantified FMM-associated proteins by MS-based label-free quantification (LFQ) of total membrane proteome, DRM, and DSM fractions in distinct growth conditions (exponential [EXP], stationary [EST], late stationary [LST] and nutrient-limiting [NLC]) (Fig. 6A). Protein in DRM and DSM fractions was calculated relative to total membrane proteome content (log2 ratio), plotted for each condition tested, and shown in a heatmap using unsupervised hierachical clustering (Fig. 6B, and 12). In DRM fractions, a core of ~100 proteins was consistently enriched in all conditions (Fig. 6B; protein clusters A + B), whereas a number of other proteins were detected in these fractions in specific growth conditions (Fig. 6B; protein clusters C [EXP], D [NLC], E [EST] and F [LST]). As control, detergent-extracted (DDM 0.5%) DRM proteins were identified in a MS-based FloA pulldown assay, which suggests that DRM proteins interact with FloA and are thus FMM-associated. Functional classification of FMM-associated proteins varies among the growth conditions (Fig. 6C), although a common feature to all is their multimeric nature. We detected protein complexes involved in membrane lipid metabolism (e.g., mgt, Igt, MurJ, LtaA, UppP, TagH and DltD), membrane transport (BmrA, Opp, CzcD, NarT, SirA), protein quality control (Sec, YajC, FtsH) and virulence (Rny, ebpS, T7SS, TcaA), as well as proteins related to cell wall organization, such as PBP2a and others that can influence PBP2a activity such as PrsA, RodA or PBP2 (Cho et al., 2016; Jousselin et al., 2015; Jousselin et al., 2012; Leski and Tomasz, 2005; Pinho et al., 2001b).

Association of protein complexes with FMM is consistent with their resemblance to eukaryotic lipid rafts and the role of rafts as platforms that promote efficient oligomerization between interacting protein partners. To explore this, we studied PBP2a as a case in point, given its importance in MRSA antimicrobial resistance. Furthermore, recent publications show *S. aureus* PBP localization in membrane foci in addition to their typical septal localization (Gautam et al., 2015; Monteiro et al., 2015). Physical interaction between PBP2a and FloA was detected first by heterologous PBP2a and FloA expression in a bacterial two-hybrid (B2H) assay (Fig. 7A). Second, using pull-down experiments, we detected PBP2a in FloA co-eluted protein samples in immunoblot with anti-PBP2a antibodies (Fig. 7B). Finally, srAT thin sections (100 nm) showed PBP2a transient colocalization with FloA in membrane foci (Fig. 7C and 13A).

Scaffold proteins stabilize protein complexes by tethering interacting partners (Good et al., 2011); a likely effect of flotillin on PBP2a activity would be FloA promotion of more efficient PBP2a oligomerization with interacting partners. Using a bacterial three-hybrid (B3H) assay, we measured PBP2a interaction with the potential partners PrsA, RodA or PBP2 in strains that produced low, medium or high FloA levels (Fig. 13B). Interaction of PBP2a and protein partners clearly improved at low and medium FloA concentrations, while a slight decrease was detected at high FloA levels. This finding is consistent with the fact that optimal scaffold concentration tethers interaction partners, whereas high concentrations titrate interacting partners and inhibit interaction thus indicating that FloA behaves as a scaffold protein (Good et al., 2011). We analyzed oligomerization of PBP2a (-80 kDa) in *S. aureus* cells using BN-PAGE, followed by PBP2a immunodetection (Fig. 7D). We identified signals (-240 and 300 kDa) in WT that was absent in Δ*pbp2a* samples (or Δ*mecA*), attributable to PBP2a oligomerization. These bands were less intense in Δ*floA* and Δ*crt* mutants, concurrent with additional bands at ~80 and ~160 kDa that correspond to partial PBP2a oligomerization. FMM architectural alterations thus appear to compromise PBP2a oligomerization.

Based on this finding, we designed an approach to exogenously perturb PBP2a oligomerization by inhibiting biosynthesis of FMM-constituent lipids using cholesterol-lowering drugs (statins) (Fig. 13C). Statins inhibit the mevalonate pathway and thus, cholesterol synthesis, in patients with hypercholesterolemia. *S. aureus* uses the mevalonate pathway to produce staphyloxanthin thus statins inhibit staphyloxanthin biosynthesis (Liu et al., 2008) (Fig. 13D). When treated

with the statin zaragozic acid (ZA, 50 μM), *S. aureus* cells showed no growth defects (Fig. 14A, B), but the number of FloA membrane foci was reduced (Fig. 14C) (Lopez and Kolter, 2010). When ZA-treated MRSA cells were tested for PBP2a oligomerization using BN-PAGE (Fig. 7D), we detected bands at ~80 kDa, which denoted deficient PBP2a oligomerization. The membrane fraction of untreated and ZA-treated *S. aureus* cells was resolved by velocity sucrose gradient centrifugation (Fig. 7E). FloA and PBP2a immunodetection in WT samples showed both signals concentrated in high-density sucrose fractions, where high MW protein complexes were detected. In contrast, ZA-treated samples showed signals in low-density sucrose fractions, in which low MW protein complexes were detected, denoting reduced oligomerization of flotillin and PBP2a in ZA-treated MRSA.

**FMM disruption inhibits penicillin resistance in MRSA**

Evidence that FMM perturbation affects PBP2a oligomerization led us to test whether β-lactam antibiotics inhibit growth of a MRSA Δ*floA* mutant. Cultures of WT and the Δ*floA* mutant were incubated with various β-lactam antibiotics (methicillin, oxacillin flucoxacillin, nafcillin and dicloxacillin), which are normally used to treat methicillin-sensitive *S. aureus* (MSSA) infections but do not eradicate MRSA infections (Peacock and Paterson, 2015) (Fig. 7F). We detected severe growth inhibition in Δ*floA* compared to WT samples at antibiotic concentrations that typically inhibit MSSA growth. As controls, WT and Δ*floA* showed comparable resistance to ampicillin, as resistance to this β-lactam antibiotic is via secretion of a β-lactamase rather than via PBP2a (Ayliffe, 1963). We then compared β-lactam sensitivity of untreated and ZA-treated MRSA cultures (Fig. 7F). The antibiotic sensitivity profiles of ZA-treated cultures resembled those of the Δ*floA* mutant, as did those of other statin molecules (Fig. 14D-F), which indicates that disturbance of the FMM interferes with penicillin resistance in MRSA.

To determine whether MRSA penicillin sensitivity can be achieved in an *in vivo* infection, we compared the infective potential of WT and Δ*floA* strains in a murine infection model. Infected mice ($n$ = 10; 3 x $10^7$ colony-forming units, CFU) were treated with oxacillin (200 mg/kg/day) (Hertlein et al., 2014) and the survival rate was monitored three days post-infection (Fig. 7G). Mice infected with the WT strain showed higher mortality (10% survival rate) than those infected with a Δ*floA* mutant (70% survival rate; $p<0.01$). To compare antibiotic sensitivity of untreated and ZA-treated WT cells, infected mice ($n$ = 10; 3 x $10^7$ CFU) were treated with oxacillin (200 mg/kg/day) or with a combination of oxacillin (200 mg/kg/day) and ZA (50 mg/kg/day); survival was monitored as before (Fig. 7G and 14G). Infected mice treated with oxacillin + ZA had a significantly higher survival rate ($p<0.01$) than those treated with oxacillin alone. In another experiment, a MRSA strain isolated from a pneumonia patient (Lopez-Collazo et al., 2015) was used to infect mice intranasally (n = 10; 3 x $10^8$ CFU) prior to oxacillin treatment (200 mg/kg/day) or treatment with oxacillin (200 mg/kg/day) and ZA (50 mg/kg/day) (Fig. 7H). Infections were allowed to progress for two days before lungs were collected and bacterial load counted. Infected mice treated with a combination of oxacillin + ZA showed significantly reduced bacterial load ($p<0.001$) compared to oxacillin-treated mice.

**Discussion**

Evidence for the importance of membrane lipid domains in bacterial cell organization is increasing (Garcia-Lara et al., 2015; Nickels et al., 2017). Here we used the human pathogen MRSA to characterize bacterial FMM structurally and functionally. FMM-constituent lipids are isoprenoid saccharolipids derived from the carotenoid staphyloxanthin. The scaffold protein flotillin preferentially binds these lipids and oligomerizes in staphyloxanthin-rich membrane microdomains. In a similar manner, eukaryotic lipid rafts are also constituted by glucolipids (i.e., sphingolipids) (Simons and Toomre, 2000). Differences in glucolipid headgroup structure dictates their lateral segregation in microdomains within the phospholipid membrane, whereas hydrogen bonding between headgroup sugars stabilizes glucolipid interactions for microdomain rigidity (Rock et al., 1990; Thompson and Tillack, 1985). Such headgroup structure differences of constituent lipids enabled us to visualize FMM using electron microscopy techniques and uranyl acetate staining; we recognized these regions as light electron-dense membrane areas to which flotillin localizes preferentially and attracts a number of multimeric protein complexes.

We used FloA-mediated PBP2a oligomerization to demonstrate the biological significance of FMM. FloA scaffold activity promotes efficient PBP2a oligomerization, and lack of flotillin inhibits PBP2a hetero-oligomerization, probably preceding FtsZ recruitment. This scaffold activity might contribute to oligomerization of other FMM-associated proteins, which could explain the virulence defect in flotillin mutants of *C. jejuni* (Tareen et al., 2013) or B. *anthracis* (Somani et al., 2016), as well as the thylakoid organization defect in cyanobacteria (Bryan et al., 2011).

When studying the influence of FMM organization on PBP2a, we observed that perturbation of FMM architecture affects PBP2a oligomerization and reduces MRSA proliferative capacity in the presence of beta-lactam antibiotics. Repurposing statins, currently used to treat hypercholesterolemia, to inhibit the biosynthesis of FMM-constituent lipids led to interference in FMM assembly in MRSA. As a result, statin-treated MRSA infections became susceptible to conventional antibiotic therapy in a mouse model.

Our results show that statin-mediated FMM dispersal requires production of FMM-constituent lipids via the mevalonate pathway, which is not universal in bacteria (Heuston et al., 2012). Moreover, our study shows that statins cause FMM dispersal and thus do not have intrinsic bactericidal activity; rather interfere with bacterial processes and synergize with other antimicrobials to kill pathogens.

The organization of FMM platforms, which in some structural and functional aspects resemble lipid rafts of eukaryotic cells, reveals a remarkable level of sophistication that is unexpected in bacteria. Disassembly of these platforms in pathogens such as MRSA could simultaneously affect numerous infection-related processes including inhibition of antibiotic resistance, and could thus be used in the development of antimicrobial therapies for multidrug- resistant bacteria.

## References

Arnaud, M., Chastanet, A., and Debarbouille, M. (2004). New vector for efficient allelic replacement in naturally nontransformable, low-GC-content, gram-positive bacteria. Appl Environ Microbiol 70, 6887-6891.

Ayliffe, G.A. (1963). Ampicillin inactivation and sensitivity of coliform bacilli. J Gen Microbiol 30, 339-348.

Bach, J.N., and Bramkamp, M. (2013). Flotillins functionally organize the bacterial membrane. Mol Microbiol 88, 1205-1217.

Bach, J.N., and Bramkamp, M. (2015). Dissecting the molecular properties of prokaryotic flotillins. PLoS One 10, e0116750.

Bergman, P., Linde, C., Putsep, K., Pohanka, A., Normark, S., Henriques-Normark, B., Andersson, J., and Bjorkhem-Bergman, L. (2011). Studies on the antibacterial effects of statins--in vitro and in vivo. PLoS One 6, e24394.

Bickel, P.E., Scherer, P.E., Schnitzer, J.E., Oh, P., Lisanti, M.P., and Lodish, H.F. (1997). Flotillin and epidermal surface antigen define a new family of caveolae-associated integral membrane proteins. J Biol Chem 272, 13793-13802.

Bramkamp, M., and Lopez, D. (2015). Exploring the Existence of Lipid Rafts in Bacteria. Microbiol Mol Biol Rev 79, 81-100.

Brown, D.A. (2002). Isolation and use of rafts. Curr Protoc Immunol Chapter 11, Unit 11 10.

Bryan, S.J., Burroughs, N.J., Evered, C., Sacharz, J., Nenninger, A., Mullineaux, C.W., and Spence, E.M. (2011). Loss of the SPHF homologue Slr1768 leads to a catastrophic failure in the maintenance of thylakoid membranes in Synechocystis sp. PCC 6803. PLoS One 6, e19625.

Cho, H., Wivagg, C.N., Kapoor, M., Barry, Z., Rohs, P.D., Suh, H., Marto, J.A., Garner, E.C., and Bernhardt, T.G. (2016). Bacterial cell wall biogenesis is mediated by SEDS and PBP polymerase families functioning semi-autonomously. Nat Microbiol, 16172.

Donovan, C., and Bramkamp, M. (2009). Characterization and subcellular localization of a bacterial flotillin homologue. Microbiology 155, 1786-1799.

Falagas, M.E., Makris, G.C., Matthaiou, D.K., and Rafailidis, P.I. (2008). Statins for infection and sepsis: a systematic review of the clinical evidence. J Antimicrob Chemother 61, 774-785.

Farmer, A. R., et al. ( 2013). Effect of HMG-CoA reductase inhibitors on antimicrobial susceptibilities for Gram-Negative rods. J. Basic Microbiol. 2013, 53, 336-339.

Feng, X., Hu, Y., Zheng, Y., Zhu, W., Li, K., Huang, C.H., Ko, T.P., Ren, F., Chan, H.C., Nega, M., et al. (2014). Structural and functional analysis of Bacillus subtilis YisP reveals a role of its product in biofilm production. Chem Biol 21, 1557-1563.

Fishovitz, J., Hermoso, J.A., Chang, M., and Mobashery, S. (2014). Penicillin-binding protein 2a of methicillin-resistant Staphylococcus aureus. IUBMB Life 66, 572-577.

Garcia-Lara, J., Weihs, F., Ma, X., Walker, L., Chaudhuri, R.R., Kasturiarachchi, J., Crossley, H., Golestanian, R., and Foster, S.J. (2015). Supramolecular structure in the membrane of Staphylococcus aureus. Proc Natl Acad Sci U S A 112, 15725-15730. Gautam, S., Kim, T., and Spiegel, D.A. (2015). Chemical probes reveal an extraseptal mode of cross-linking in Staphylococcus aureus. J Am Chem Soc 137, 7441-7447.

Ghodke, R.M., Tour, N., and Devi, K. (2012). Effects of statins and cholesterol on memory functions in mice. Metab Brain Dis 27, 443-451.

Good, M.C., Zalatan, J.G., and Lim, W.A. (2011). Scaffold proteins: hubs for controlling the flow of cellular information. Science 332, 680-686.

Hayat, M.A. (1993). Stains and cytochemical methods (New York and London: Plenun Press).

Heimesaat, M.M., Lugert, R., Fischer, A., Alutis, M., Kuhl, A.A., Zautner, A.E., Tareen, A.M., Hennessy, E., et al, (2016). Is There Potential for Repurposing Statins as Novel Antimicrobials? Antimicrobial Agents and Chemotherapy 60, 9, 5111-5121.

Heuston, S., et al. (2012). Isoprenoid biosynthesis in bacterial pathogens. Microbiology (2012), 158, 1389-1401.

Gobel, U.B., and Bereswill, S. (2014). Impact of Campylobacter jejuni cj0268c knockout mutation on intestinal colonization, translocation, and induction of immunopathology in gnotobiotic IL-10 deficient mice. PLoS One 9, e90148.
Helmprobst, F., Frank, M., and Stigloher, C. (2015). Presynaptic architecture of the larval zebrafish neuromuscular junction. J Comp Neurol 523, 1984-1997.
Hertlein, T., Sturm, V., Lorenz, U., Sumathy, K., Jakob, P., and Ohlsen, K. (2014). Bioluminescence and 19F magnetic resonance imaging visualize the efficacy of lysostaphin alone and in combination with oxacillin against Staphylococcus aureus in murine thigh and catheter-associated infection models. Antimicrob Agents Chemother 58, 1630-1638.
Heuston, S., Begley, M., Gahan, C.G., and Hill, C. (2012). Isoprenoid biosynthesis in bacterial pathogens. Microbiology 158, 1389-1401.
Jousselin, A., Manzano, C., Biette, A., Reed, P., Pinho, M.G., Rosato, A.E., Kelley, W.L., and Renzoni, A. (2015). The Staphylococcus aureus Chaperone PrsA Is a New Auxiliary Factor of Oxacillin Resistance Affecting Penicillin-Binding Protein 2A. Antimicrob Agents Chemother 60, 1656-1666.
Jousselin, A., Renzoni, A., Andrey, D.O., Monod, A., Lew, D.P., and Kelley, W.L. (2012). The posttranslocational chaperone lipoprotein PrsA is involved in both glycopeptide and oxacillin resistance in Staphylococcus aureus. Antimicrob Agents Chemother 56, 3629-3640.
Koch, G., Wermser, C., Acosta, I.C., Kricks, L., Stengel, S.T., Yepes, A., and Lopez, D. (2017). Attenuating Staphylococcus aureus Virulence by Targeting Flotillin Protein Scaffold Activity. Cell Chem Biol 24, 845-857 e846.
Kraft, M.L. (2013). Plasma membrane organization and function: moving past lipid rafts. Mol Biol Cell 24, 2765-2768.
Kreiswirth, B., Kornblum, J., Arbeit, R.D., Eisner, W., Maslow, J.N., McGeer, A., Low, D.E., and Novick, R.P. (1993). Evidence for a clonal origin of methicillin resistance in Staphylococcus aureus. Science 259, 227-230.
Kullik, I., Giachino, P., and Fuchs, T. (1998). Deletion of the alternative sigma factor sigmaB in Staphylococcus aureus reveals its function as a global regulator of virulence genes. J Bacteriol 180, 4814-4820.
LaRocca, T.J., Pathak, P., Chiantia, S., Toledo, A., Silvius, J.R., Benach, J.L., and London, E. (2013). Proving lipid rafts exist: membrane domains in the prokaryote Borrelia burgdorferi have the same properties as eukaryotic lipid rafts. PLoS Pathog 9, e1003353.
Leski, T.A., and Tomasz, A. (2005). Role of penicillin-binding protein 2 (PBP2) in the antibiotic susceptibility and cell wall cross-linking of Staphylococcus aureus: evidence for the cooperative functioning of PBP2, PBP4, and PBP2A. J Bacteriol 187, 1815-1824.
Liappis, A.P., Kan, V.L., Rochester, C.G., and Simon, G.L. (2001). The effect of statins on mortality in patients with bacteremia. Clin Infect Dis 33, 1352-1357.
Liu, C.I., Liu, G.Y., Song, Y., Yin, F., Hensler, M.E., Jeng, W.Y., Nizet, V., Wang, A.H., and Oldfield, E. (2008). A cholesterol biosynthesis inhibitor blocks Staphylococcus aureus virulence. Science 319, 1391-1394.
Lopez, D., and Kolter, R. (2010). Functional microdomains in bacterial membranes. Genes Dev 24, 1893-1902.
Lopez-Collazo, E., Jurado, T., de Dios Caballero, J., Perez-Vazquez, M., Vindel, A., Hernandez-Jimenez, E., Tamames, J., Cubillos-Zapata, C., Manrique, M., Tobes, R., et al. (2015). In vivo attenuation and genetic evolution of a ST247-SCCmecI MRSA clone after 13 years of pathogenic bronchopulmonary colonization in a patient with cystic fibrosis: implications of the innate immune response. Mucosal Immunol 8, 362-371.
Lopez-Cortes, L.E., Galvez-Acebal, J., Del Toro, M.D., Velasco, C., de Cueto, M., Caballero, F.J., Muniain, M.A., Pascual, A., and Rodriguez-Bano, J. (2013). Effect of statin therapy in the outcome of bloodstream infections due to Staphylococcus aureus: a prospective cohort study. PLoS One 8, e82958.
Lorent, J.H., and Levental, I. (2015). Structural determinants of protein partitioning into ordered membrane domains and lipid rafts. Chem Phys Lipids 192, 23-32.
Markert, S.M., Britz, S., Proppert, S., Lang, M., Witvliet, D., Mulcahy, B., Sauer, M., Zhen, M., Bessereau, J.L., and Stigloher, C. (2016). Filling the gap: adding super-resolution to array tomography for correlated ultrastructural and molecular identification of electrical synapses at the C. elegans connectome. Neurophotonics 3, 041802.
Marshall, J.H., and Wilmoth, G.J. (1981). Proposed pathway of triterpenoid carotenoid biosynthesis in Staphylococcus aureus: evidence from a study of mutants. J Bacteriol 147, 914-919.
Martos, A., Raso, A., Jimenez, M., Petrasek, Z., Rivas, G., and Schwille, P. (2015). FtsZ Polymers Tethered to the Membrane by ZipA Are Susceptible to Spatial Regulation by Min Waves. Biophys J 108, 2371-2383.
McDougal, L.K., Steward, C.D., Killgore, G.E., Chaitram, J.M., McAllister, S.K., and Tenover, F.C. (2003). Pulsed-field gel electrophoresis typing of oxacillin-resistant Staphylococcus aureus isolates from the United States: establishing a national database. J Clin Microbiol 41, 5113-5120.
McQuillen, K. (1962). Bacterial Ribosomes and Protein Synthesis. J Gen Microbiol 29, 53-57.
Monteiro, J.M., Fernandes, P.B., Vaz, F., Pereira, A.R., Tavares, A.C., Ferreira, M.T., Pereira, P.M., Veiga, H., Kuru, E., VanNieuwenhze, M.S., et al. (2015). Cell shape dynamics during the staphylococcal cell cycle. Nat Commun 6, 8055.
Nickels, J.D., Chatterjee, S., Stanley, C.B., Qian, S., Cheng, X., Myles, D.A.A., Standaert, R.F., Elkins, J.G., and

Katsaras, J. (2017). The in vivo structure of biological membranes and evidence for lipid domains. PLoS Biol 15, e2002214.
Otero, L.H., Rojas-Altuve, A., Llarrull, L.I., Carrasco-Lopez, C., Kumarasiri, M., Lastochkin, E., Fishovitz, J., Dawley, M., Hesek, D., Lee, M., et al. (2013). How allosteric control of Staphylococcus aureus penicillin binding protein 2a enables methicillin resistance and physiological function. Proc Natl Acad Sci U S A 110, 16808-16813.
Palade, G.E. (1955). A small particulate component of the cytoplasm. J Biophys Biochem Cytol 1, 59-68.
Parihar, S.P., Guler, R., Khutlang, R., Lang, D.M., Hurdayal, R., Mhlanga, M.M., Suzuki, H., Marais, A.D., and Brombacher, F. (2014). Statin therapy reduces the Mycobacterium tuberculosis burden in human macrophages and in mice by enhancing autophagy and phagosome maturation. J Infect Dis 209, 754-763.
Peacock, S.J., and Paterson, G.K. (2015). Mechanisms of Methicillin Resistance in Staphylococcus aureus. Annu Rev Biochem 84, 577-601.
Pelz, A., Wieland, K.P., Putzbach, K., Hentschel, P., Albert, K., and Gotz, F. (2005). Structure and biosynthesis of staphyloxanthin from Staphylococcus aureus. J Biol Chem 280, 32493-32498.
Pinho, M.G., de Lencastre, H., and Tomasz, A. (2001a). An acquired and a native penicillin-binding protein cooperate in building the cell wall of drug-resistant staphylococci. Proc Natl Acad Sci U S A 98, 10886-10891.
Pinho, M.G., Filipe, S.R., de Lencastre, H., and Tomasz, A. (2001b). Complementation of the essential peptidoglycan transpeptidase function of penicillin-binding protein 2 (PBP2) by the drug resistance protein PBP2A in Staphylococcus aureus. J Bacteriol 183, 6525-6531.
Rock, P., Allietta, M., Young, W.W., Jr., Thompson, T.E., and Tillack, T.W. (1990). Organization of glycosphingolipids in phosphatidylcholine bilayers: use of antibody molecules and Fab fragments as morphologic markers. Biochemistry 29, 8484-8490.
Schneider, J., Klein, T., Mielich-Suss, B., Koch, G., Franke, C., Kuipers, O.P., Kovacs, A.T., Sauer, M., and Lopez, D. (2015). Spatio-temporal remodeling of functional membrane microdomains organizes the signaling networks of a bacterium. PLoS Genet 11, e1005140. Schneider, J., Yepes, A., Garcia-Betancur, J.C., Westedt, I., Mielich, B., and Lopez, D. (2012). Streptomycin-induced expression in Bacillus subtilis of YtnP, a lactonase-homologous protein that inhibits development and streptomycin production in Streptomyces griseus. Appl Environ Microbiol 78, 599-603.
Silva-Rocha, R., Martinez-Garcia, E., Calles, B., Chavarria, M., Arce-Rodriguez, A., de Las Heras, A., Paez-Espino, A.D., Durante-Rodriguez, G., Kim, J., Nikel, P.I., et al. (2013). The Standard European Vector Architecture (SEVA): a coherent platform for the analysis and deployment of complex prokaryotic phenotypes. Nucleic Acids Res 41, D666-675.
Simons, K., and Ikonen, E. (1997). Functional rafts in cell membranes. Nature 387, 569-572. Simons, K., and Toomre, D. (2000). Lipid rafts and signal transduction. Nat Rev Mol Cell Biol 1, 31-39.
Singer, S.J., and Nicolson, G.L. (1972). The fluid mosaic model of the structure of cell membranes. Science 175, 720-731.
Somani, V.K., Aggarwal, S., Singh, D., Prasad, T., and Bhatnagar, R. (2016). Identification of Novel Raft Marker Protein, FlotP in Bacillus anthracis. Front Microbiol 7, 169.
Tareen, A.M., Luder, C.G., Zautner, A.E., Grobeta, U., Heimesaat, M.M., Bereswill, S., and Lugert, R. (2013). The Campylobacter jejuni Cj0268c protein is required for adhesion and invasion in vitro. PLoS One 8, e81069.
Thangamani , S., et al. (2015). Exploring simvastatin, an antihyperlipidemic drug, as a potential topical antibacterial agent. Scientific reports 5, 16407.
Thompson, T.E., and Tillack, T.W. (1985). Organization of glycosphingolipids in bilayers and plasma membranes of mammalian cells. Annu Rev Biophys Biophys Chem 14, 361-386. Ting-Beall, H.P. (1980). Interactions of uranyl ions with lipid bilayer membranes. J Microsc 118, 221-227.
Wan, Y.D., Sun, T.W., Kan, Q.C., Guan, F.X., and Zhang, S.G. (2014). Effect of statin therapy on mortality from infection and sepsis: a meta-analysis of randomized and observational studies. Crit Care 18, R71.
Wieland, B., Feil, C., Gloria-Maercker, E., Thumm, G., Lechner, M., Bravo, J.M., Poralla, K., and Gotz, F. (1994). Genetic and biochemical analyses of the biosynthesis of the yellow carotenoid 4,4'-diaponeurosporene of Staphylococcus aureus. J Bacteriol 176, 7719-7726.
Wittig, I., Braun, H.P., and Schagger, H. (2006). Blue native PAGE. Nat Protoc 1, 418-428. Yepes, A., Koch, G., Waldvogel, A., Garcia-Betancur, J.C., and Lopez, D. (2014). Reconstruction of mreB expression in Staphylococcus aureus via a collection of new integrative plasmids. Appl Environ Microbiol 80, 3868-3878.
Zapun, A., Contreras-Martel, C., and Vernet, T. (2008). Penicillin-binding proteins and betalactam resistance. FEMS Microbiol Rev 32, 361-385.

## Claims

1. A beta-lactamase resistant penicillin for use in a method of treating a bacterial infection in a subject in need thereof,

wherein said bacterial infection is caused by a bacterial population from a Enterococci, Pneumococci or Staphylococci bacterial species wherein said bacterial population has resistance to said penicillin induced by low affinity penicillin-binding proteins (PBPs) ;

wherein said subject is further administered, before or simultaneously to said penicillin, a statin of formula (I) or (Ia), or a pharmaceutically acceptable salt or stereoisomer thereof:

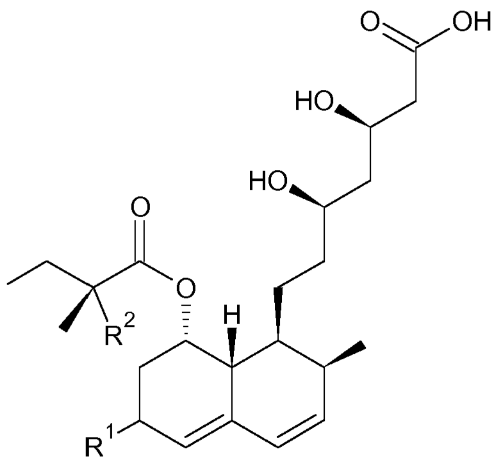

(I)

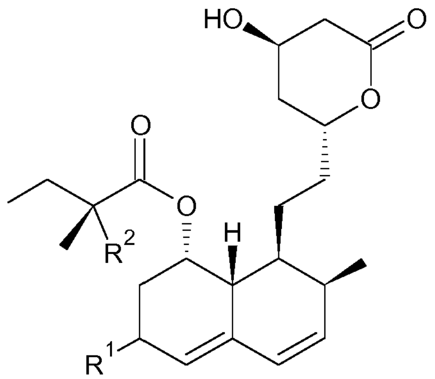

(Ia),

wherein $R^1$ and $R^2$ are independently selected from the group consisting of H, OH, $CH_3$, $CH_2CH_3$, and halogen, and
wherein said statin reduces or reverses resistance to said penicillin in said resistant bacterial population.

**2.** The beta-lactamase resistant penicillin for use in a method of treatment according to claim 1, with the proviso that said statin of formula (I) or (Ia) is not simvastatin.

**3.** The beta-lactamase resistant penicillin for use in a method of treatment according to any of claims 1 or 2, wherein said statin reduces or reverses resistance induced by low affinity penicillin-binding proteins (PBPs) to said penicillin in said resistant bacterial population.

**4.** The beta-lactamase resistant penicillin for use in a method of treatment according to any of claims 1 to 3, wherein said bacterial species is a *Staphylococcus aureus* strain.

**5.** The beta-lactamase resistant penicillin for use in a method of treatment according to any of claims 1 to 4 wherein said *S.aureus* strain is selected from the group consisting of Methicillin-resistant Staphylococcus aureus (MRSA), Community-associated Methicillin-resistant Staphylococcus aureus (CA-MRSA), vancomycin intermediate resistant staphylococcus aureus (VISA) and vancomycin resistant staphylococcus aureus (VRSA).

**6.** The beta- lactamase resistant penicillin for use in a method of treatment according to any of claims 1 to 5, wherein said β-lactamase resistant penicillin is selected from the group consisting of flucloxacillin, cloxacillin, dicloxacillin, methicillin, oxacillin, and nafcillin.

**7.** The beta-lactamase resistant penicillin for use in a method of treatment according to any of claims 1 to 6, wherein $R^1$ in said statin of formula (I) or or (Ia) is selected from the group consisting of H, OH, and $CH_3$.

**8.** The beta-lactamase resistant penicillin for use in a method of treatment according to any of claims 1 to 7, wherein $R^2$ in said statin of formula (I) or (Ia) is selected from the group consisting of H and $CH_3$.

**9.** The beta-lactamase resistant penicillin for use in a method of treatment according to any of claims 1 to 8, wherein said statin of formula (I) or (Ia) is selected from the group consisting of lovastatin, mevastatin, pravastatin and

simvastatin.

**10.** The beta-lactamase resistant penicillin for use in a method of treatment according to any of claims 1 to 9, wherein said statin of formula (I) or (Ia) is administered at least 15 minutes before, at least 30 minutes before, preferably at least 1 hour before the administration of said penicillin.

**11.** Pharmaceutical composition comprising a beta-lactamase resistant penicillin, a statin of formula (I) or (Ia), and a pharmaceutically acceptable excipient or carrier, for use in a method of treatment according to any of claims 1 to 10.

**12.** A pharmaceutical kit comprising:

i. a pharmaceutical composition comprising a beta-lactamase resistant penicillin, and
ii. a pharmaceutical composition comprising a statin of formula (I) or (Ia),

for use in a method of treatment according to any of claims 1 to 11.

**Patentansprüche**

**1.** Beta-Laktamase-resistentes Penicillin für dessen Verwendung in einem Verfahren zur Behandlung einer bakteriellen Infektion in einem Individuum, das es benötigt, wobei die genannte bakterielle Infektion von einer Bakterienpopulation aus einer Enterokokken-, Pneumokokken- oder Staphylokokken-Bakterienspezies hervorgerufen wird, wobei die genannte Bakterienpopulation Resistenz gegenüber dem genannten Penicillin aufweist, welche von niedrig affinen Penicillin-bindenden Proteinen (PBP) induziert wird;

wobei zusätzlich dem genannten Individuum, vor dem oder gleichzeitig zum genannten Penicillin, ein Statin der Formel (I) oder (Ia), oder ein pharmazeutisch akzeptables Salz oder Stereoisomer desselben verabreicht wird:

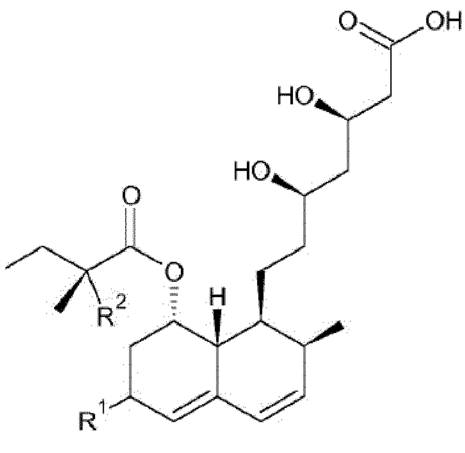

(I)

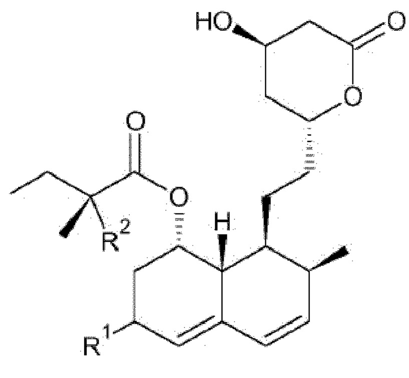

(Ia),

in welchen $R^1$ und $R^2$ unabhängig aus der Gruppe bestehend aus H, OH, $CH_3$, $CH_2CH_3$ und Halogen ausgewählt werden, und
wobei das genannte Statin Resistenz gegenüber dem genannten Penicillin in der genannten resistenten Bakterienpopulation verringert oder umkehrt.

**2.** Beta-Laktamase-resistentes Penicillin für dessen Verwendung in einem Behandlungsverfahren nach Anspruch 1, unter der Bedingung, dass das genannte Statin der Formel (I) oder (Ia) nicht Simvastatin ist.

**3.** Beta-Laktamase-resistentes Penicillin für dessen Verwendung in einem Behandlungsverfahren nach einem der Ansprüche 1 oder 2, wobei das genannte Statin die von niedrig affinen Penicillin-bindenden Proteinen (PBP) induzierte Resistenz gegenüber dem genannten Penicillin in der genannten resistenten Bakterienpopulation verringert oder umkehrt.

**4.** Beta-Laktamase-resistentes Penicillin für dessen Verwendung in einem Behandlungsverfahren nach einem der Ansprüche 1 bis 3, wobei die genannte Bakterienspezies ein *Staphylococcus* aureus-Stamm ist.

**5.** Beta-Laktamase-resistentes Penicillin für dessen Verwendung in einem Behandlungsverfahren nach einem der Ansprüche 1 bis 4, wobei der genannte *S. aureus*-Stamm aus der Gruppe bestehend aus Methicillin-resistentem *Staphylococcus aureus* (MRSA), ambulant erworbenem Methicillin-resistentem *Staphylococcus aureus* (CA-MRSA), Vancomycin-intermediär-resistentem *Staphylococcus aureus* (VISA) und Vancomycinresistentem *Staphylococcus aureus* (VRSA) ausgewählt wird.

**6.** Beta-Laktamase-resistentes Penicillin für dessen Verwendung in einem Behandlungsverfahren nach einem der Ansprüche 1 bis 5, wobei das genannte β-Laktamase-resistente Penicillin aus der Gruppe bestehend aus Flucloxacillin, Cloxacillin, Dicloxacillin, Methicillin, Oxacillin und Nafcillin ausgewählt wird.

**7.** Beta-Laktamase-resistentes Penicillin für dessen Verwendung in einem Behandlungsverfahren nach einem der Ansprüche 1 bis 6, wobei $R^1$ im genannten Statin der Formel (I) oder (Ia) aus der Gruppe bestehend aus H, OH, und $CH_3$ ausgewählt wird.

**8.** Beta-Laktamase-resistentes Penicillin für dessen Verwendung in einem Behandlungsverfahren nach einem der Ansprüche 1 bis 7, wobei $R^2$ im genannten Statin der Formel (I) oder (Ia) aus der Gruppe bestehend aus H und $CH_3$ ausgewählt wird

**9.** Beta-Laktamase-resistentes Penicillin für dessen Verwendung in einem Behandlungsverfahren nach einem der Ansprüche 1 bis 8, wobei das genannte Statin der Formel (I) oder (Ia) aus der Gruppe bestehend aus Lovastatin, Mevastatin, Pravastatin und Simvastatin ausgewählt wird.

**10.** Beta-Laktamase-resistentes Penicillin für dessen Verwendung in einem Behandlungsverfahren nach einem der Ansprüche 1 bis 9, wobei das genannte Statin der Formel (I) oder (Ia) mindestens 15 Minuten vor, mindestens 30 Minuten vor, vorzugsweise mindestens 1 Stunde vor der Verabreichung des genannten Penicillins verabreicht wird.

**11.** Pharmazeutische Zusammensetzung umfassend ein Beta-Laktamase-resistentes Penicillin, ein Statin der Formel (I) oder (Ia), und ein pharmazeutisch akzeptabler Hilfsstoff oder Träger, für dessen Verwendung in einem Behandlungsverfahren nach einem der Ansprüche 1 bis 10.

**12.** Pharmazeutisches Kit umfassend:

i. eine pharmazeutische Zusammensetzung umfassend ein Beta-Laktamase-resistentes Penicillin, und
ii. eine pharmazeutische Zusammensetzung umfassend ein Statin der Formel (I) oder (Ia),

für dessen Verwendung in einem Behandlungsverfahren nach einem der Ansprüche 1 bis 11.

## Revendications

**1.** Pénicilline résistante aux bêta-lactamases pour son utilisation dans une méthode de traitement d'une infection bactérienne chez un sujet qui en a besoin, dans laquelle ladite infection bactérienne est provoquée par une population bactérienne d'une espèce bactérienne de *Enterococcus, Pneumococcus* ou *Staphylococcus,* dans laquelle ladite population bactérienne a une résistance à ladite pénicilline induite par des protéines de liaison à la pénicilline (PBP) de faible affinité ;

dans laquelle l'on administre en outre audit sujet, avant ou simultanément avec ladite pénicilline, une statine de formule (I) ou (Ia), ou un sel pharmaceutiquement acceptable ou un stéréoisomère de celle-ci :

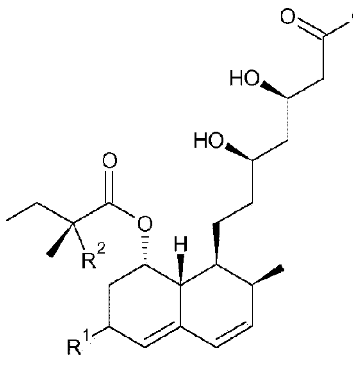 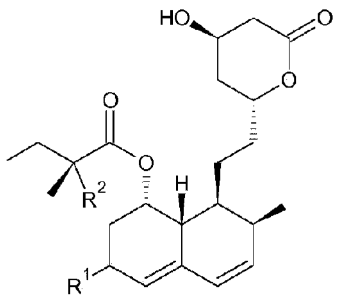

(I) (Ia),

dans lesquelles $R^1$ et $R^2$ sont sélectionnés indépendamment du groupe qui consiste en H, OH, $CH_3$, $CH_2CH_3$ et halogène, et
dans laquelle ladite statine réduit ou renverse la résistance à ladite pénicilline dans ladite population bactérienne résistante.

2. Pénicilline résistante aux bêta-lactamases pour son utilisation dans une méthode de traitement selon la revendication 1, avec la condition que ladite statine de formule (I) ou (Ia) n'est pas de la simvastatine.

3. Pénicilline résistante aux bêta-lactamases pour son utilisation dans une méthode de traitement selon l'une quelconque des revendications 1 ou 2, dans laquelle ladite statine réduit ou renverse la résistance induite par des protéines de liaison à la pénicilline (PBP) de faible affinité à ladite pénicilline dans ladite population bactérienne résistante.

4. Pénicilline résistante aux bêta-lactamases pour son utilisation dans une méthode de traitement selon l'une quelconque des revendications 1 à 3, dans laquelle ladite espèce bactérienne est une souche de *Staphylococcus aureus.*

5. Pénicilline résistante aux bêta-lactamases pour son utilisation dans une méthode de traitement selon l'une quelconque des revendications 1 à 4, dans laquelle ladite souche de *S. aureus* est sélectionnée du groupe qui consiste en *Staphylococcus aureus* résistant à la méticilline (MRSA), *Staphylococcus aureus* résistant à la méticilline associé à la communauté (CA-MRSA), *Staphylococcus aureus* avec résistance intermédiaire à la vancomycine (VISA) et *Staphylococcus aureus* résistant à la vancomycine (VRSA).

6. Pénicilline résistante aux bêta-lactamases pour son utilisation dans une méthode de traitement selon l'une quelconque des revendications 1 à 5, dans laquelle ladite pénicilline résistante aux β-lactamases est sélectionnée du groupe qui consiste en flucloxacilline, cloxacilline, dicloxacilline, méticilline, oxacilline et nafcilline.

7. Pénicilline résistante aux bêta-lactamases pour son utilisation dans une méthode de traitement selon l'une quelconque des revendications 1 à 6, dans laquelle $R^1$ dans ladite statine de formule (I) ou (Ia) est sélectionnée du groupe qui consiste en H, OH et $CH_3$.

8. Pénicilline résistante aux bêta-lactamases pour son utilisation dans une méthode de traitement selon l'une quelconque des revendications 1 à 7, dans laquelle $R^2$ dans ladite statine de formule (I) ou (Ia) est sélectionnée du groupe qui consiste en H et $CH_3$.

9. Pénicilline résistante aux bêta-lactamases pour son utilisation dans une méthode de traitement selon l'une quelconque des revendications 1 à 8, dans laquelle ladite statine de formule (I) ou (Ia) est sélectionnée du groupe qui consiste en lovastatine, mevastatine, pravastatine et simvastatine.

10. Pénicilline résistante aux bêta-lactamases pour son utilisation dans une méthode de traitement selon l'une quelconque des revendications 1 à 9, dans laquelle ladite statine de formule (I) ou (Ia) est administrée au moins 15 minutes avant, au moins 30 minutes avant, préférablement au moins 1 heure avant l'administration de ladite pénicilline.

**11.** Composition pharmaceutique qui comprend une pénicilline résistante aux bêta-lactamases, une statine de formule (I) ou (Ia) et un excipient ou porteur pharmaceutiquement acceptable, pour son utilisation dans une méthode de traitement selon l'une quelconque des revendications 1 à 10.

**12.** Kit pharmaceutique, qui comprend :

i. une composition pharmaceutique qui comprend une pénicilline résistante aux bêta-lactamases ; et
ii. une composition pharmaceutique qui comprend une statine de formule (I) ou (Ia),

pour son utilisation dans une méthode de traitement selon l'une quelconque des revendications 1 à 11.

Fig. 1

A

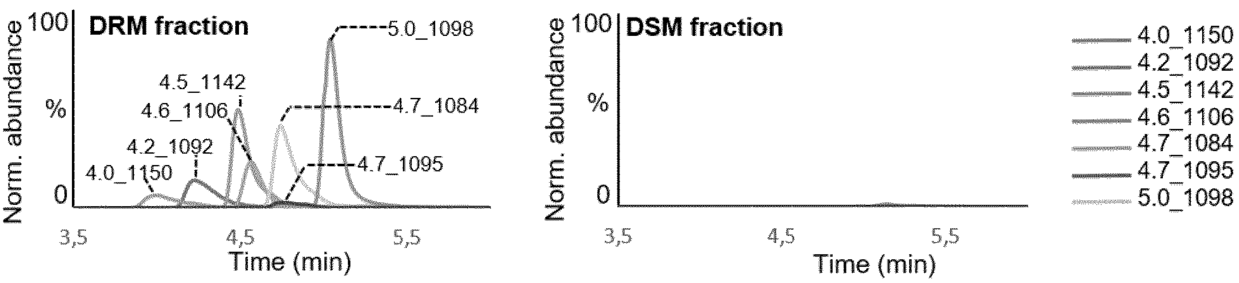
DRM fraction
Norm. abundance
100
%
0
5.0_1098
4.5_1142
4.6_1106
4.7_1084
4.2_1092
4.7_1095
4.0_1150
3,5
4,5
5,5
Time (min)
DSM fraction
Norm. abundance
100
%
0
3,5
4,5
5,5
Time (min)
4.0_1150
4.2_1092
4.5_1142
4.6_1106
4.7_1084
4.7_1095
5.0_1098

B

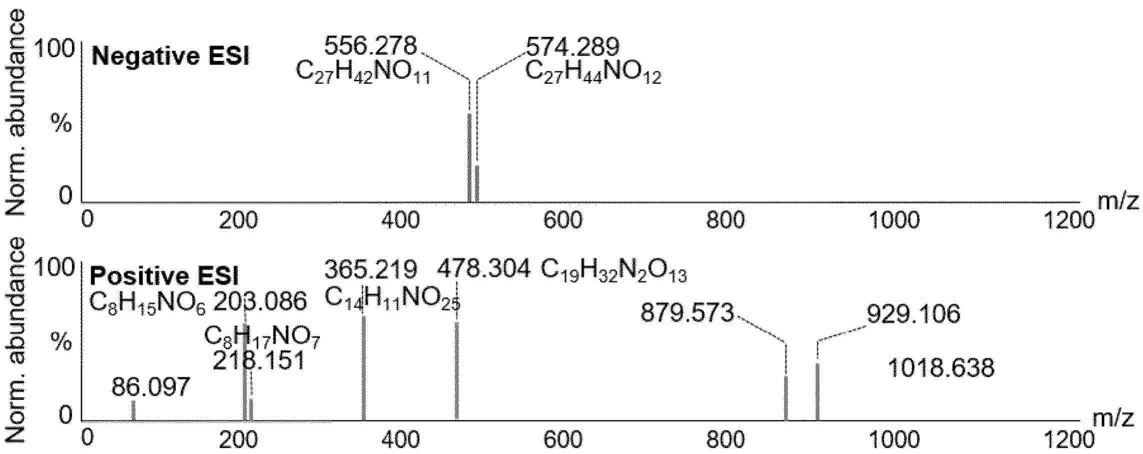
Negative ESI
Norm. abundance
100
%
0
556.278
C27H42NO11
574.289
C27H44NO12
0
200
400
600
800
1000
1200
m/z
Positive ESI
Norm. abundance
100
%
0
C8H15NO6 203.086
365.219
C14H11NO25
478.304 C19H32N2O13
C8H17NO7
218.151
86.097
879.573
929.106
1018.638
0
200
400
600
800
1000
1200
m/z

**Fig. 1 (cont.)**

C

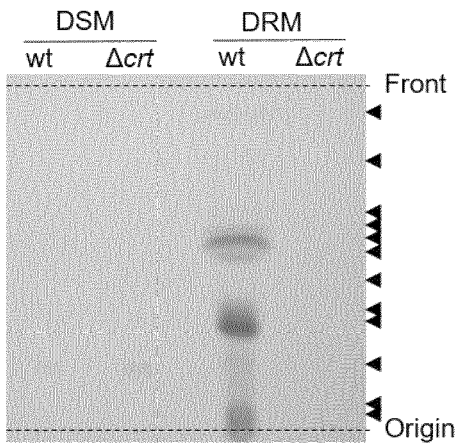
DSM
DRM
wt
Δcrt
wt
Δcrt
Front
Origin

D

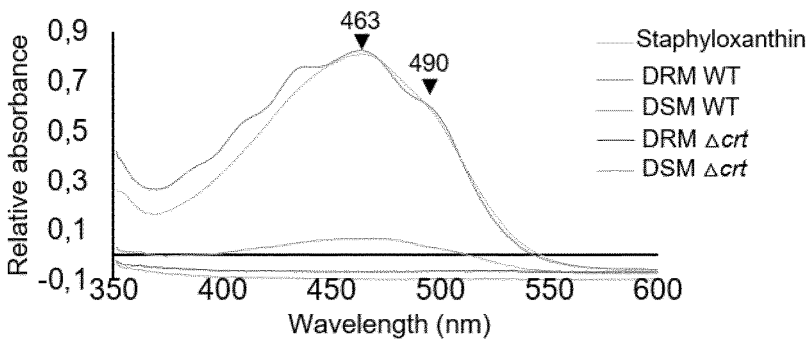
463
490
Staphyloxanthin
DRM WT
DSM WT
DRM Δcrt
DSM Δcrt
Relative absorbance
0,9
0,7
0,5
0,3
0,1
-0,1
350
400
450
500
550
600
Wavelength (nm)

Fig. 1 (cont.)

E

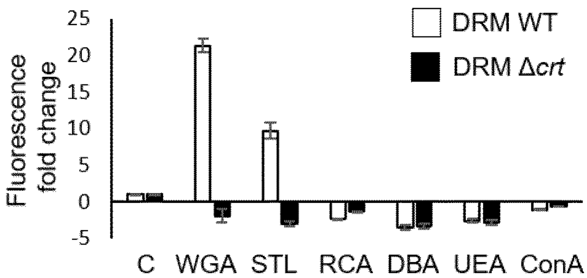


F

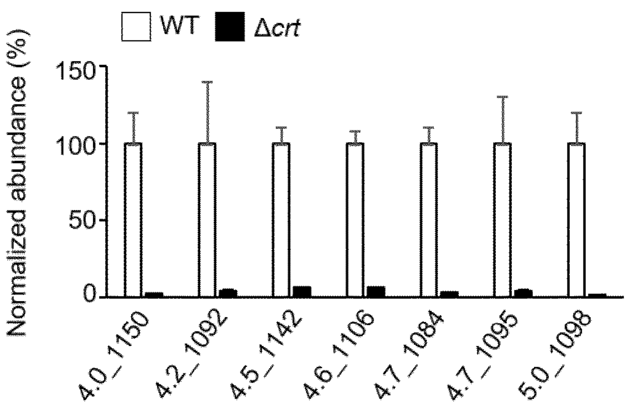


G

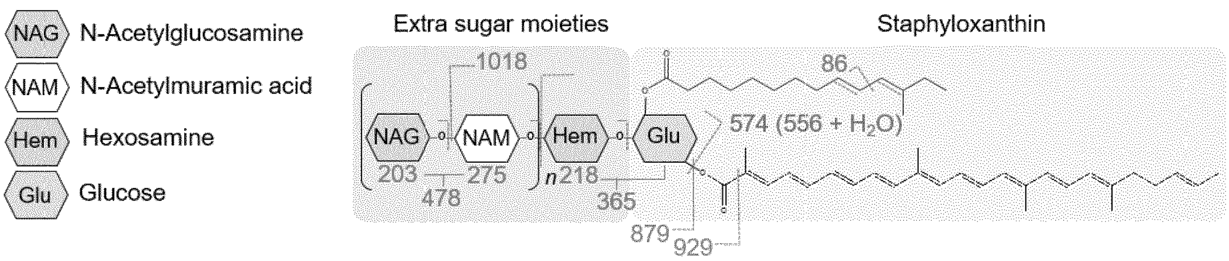

Fig. 2

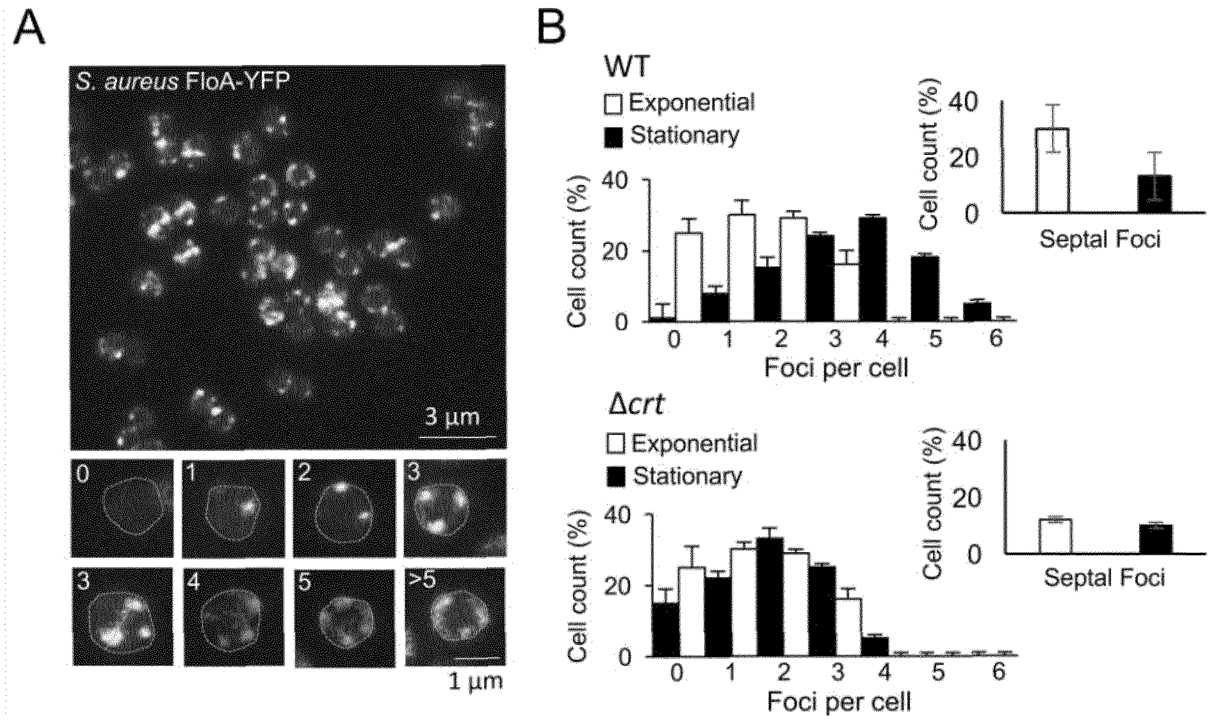
A
S. aureus FloA-YFP
3 µm
0
1
2
3
3
4
5
>5
1 µm
B
WT
Exponential
Stationary
Cell count (%)
40
20
0
0 1 2 3 4 5 6
Foci per cell
Septal Foci
Δcrt
Exponential
Stationary
Cell count (%)
Foci per cell
Septal Foci

**Fig. 2 (cont.)**

**C**

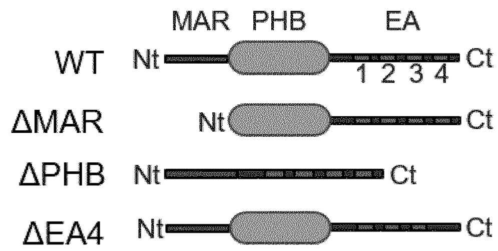
MAR PHB EA
WT Nt Ct
1 2 3 4
ΔMAR Nt Ct
ΔPHB Nt Ct
ΔEA4 Nt Ct

**D**

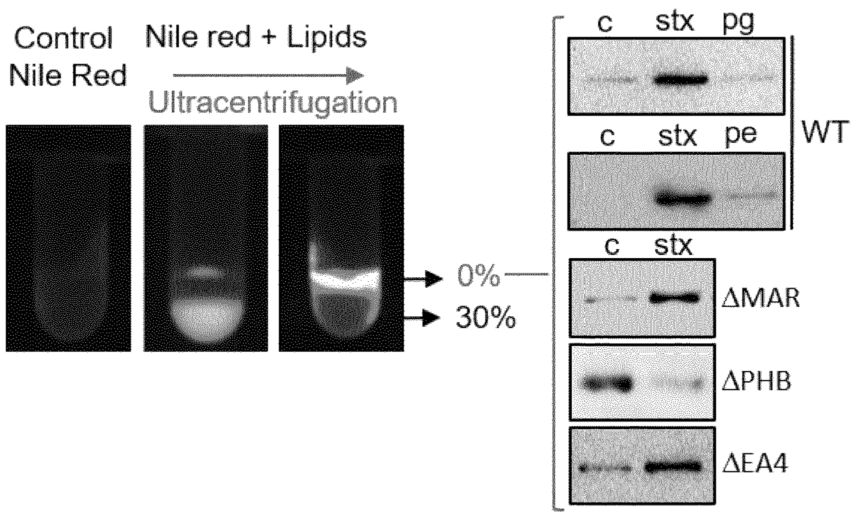
Control Nile Red
Nile red + Lipids
Ultracentrifugation
0%
30%
c stx pg
c stx pe
WT
c stx
ΔMAR
ΔPHB
ΔEA4

Fig. 2 (cont.)

E

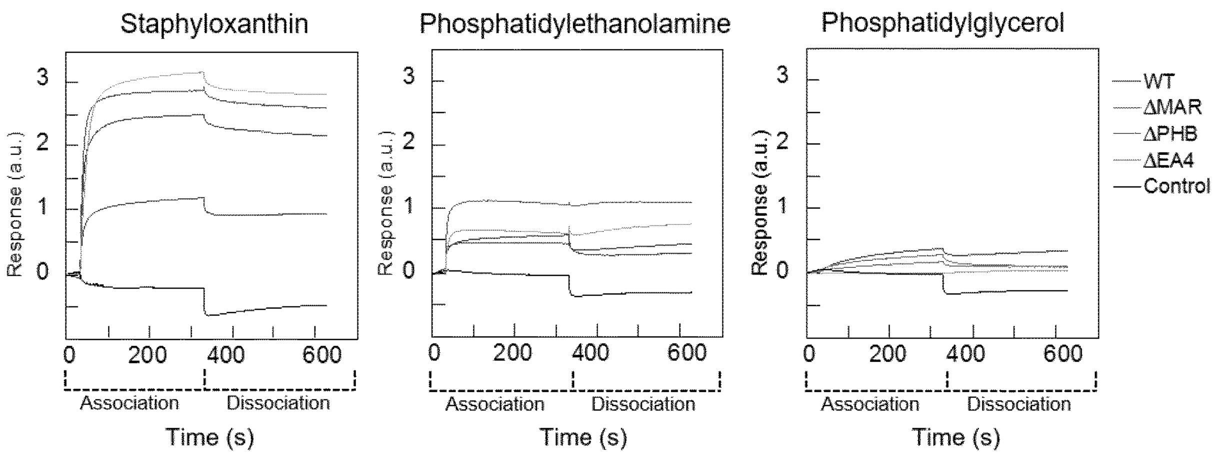
Staphyloxanthin
Phosphatidylethanolamine
Phosphatidylglycerol
Response (a.u.)
3
2
1
0
0
200
400
600
Association
Dissociation
Time (s)
WT
ΔMAR
ΔPHB
ΔEA4
Control

Fig. 3

A

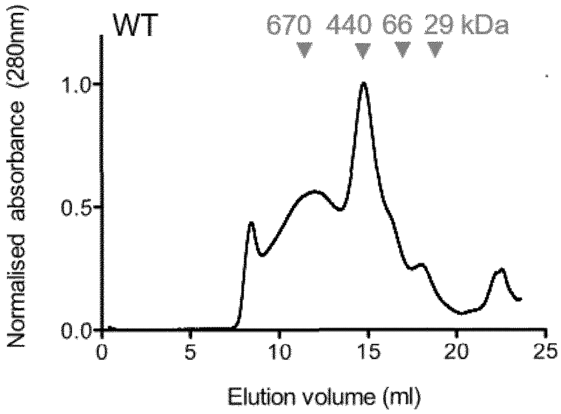
WT
670 440 66 29 kDa
Normalised absorbance (280nm)
1.0
0.5
0.0
0 5 10 15 20 25
Elution volume (ml)

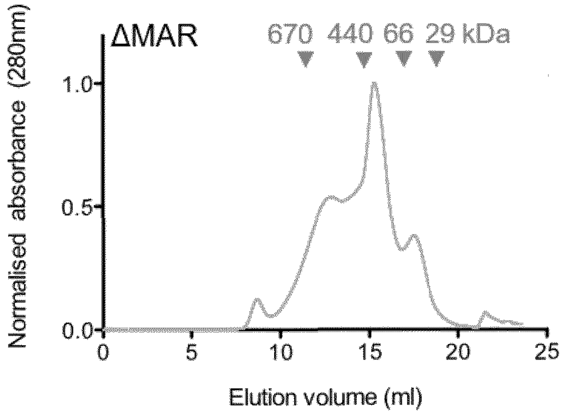
ΔMAR
670 440 66 29 kDa
Normalised absorbance (280nm)
1.0
0.5
0.0
0 5 10 15 20 25
Elution volume (ml)

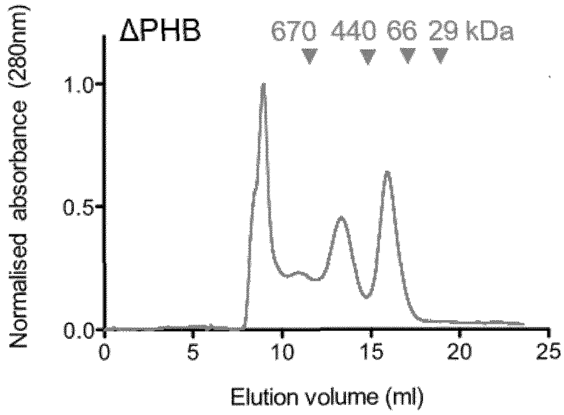
ΔPHB
670 440 66 29 kDa
Normalised absorbance (280nm)
1.0
0.5
0.0
0 5 10 15 20 25
Elution volume (ml)

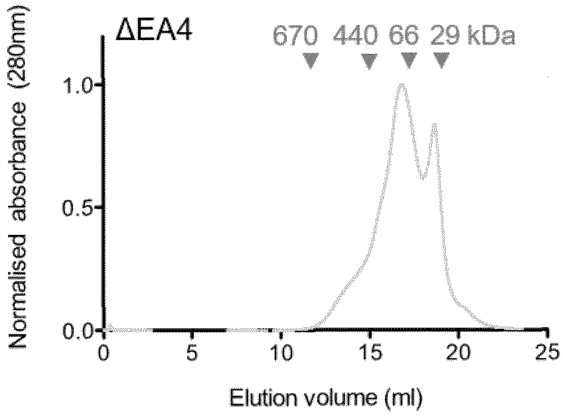
ΔEA4
670 440 66 29 kDa
Normalised absorbance (280nm)
1.0
0.5
0.0
0 5 10 15 20 25
Elution volume (ml)

Fig. 3 (cont.)

B

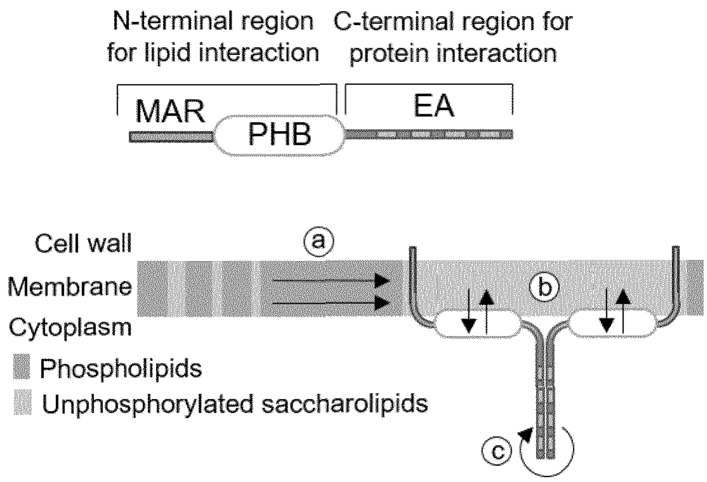
N-terminal region
for lipid interaction
C-terminal region for
protein interaction
MAR
PHB
EA
Cell wall
Membrane
Cytoplasm
a
b
c
Phospholipids
Unphosphorylated saccharolipids

C

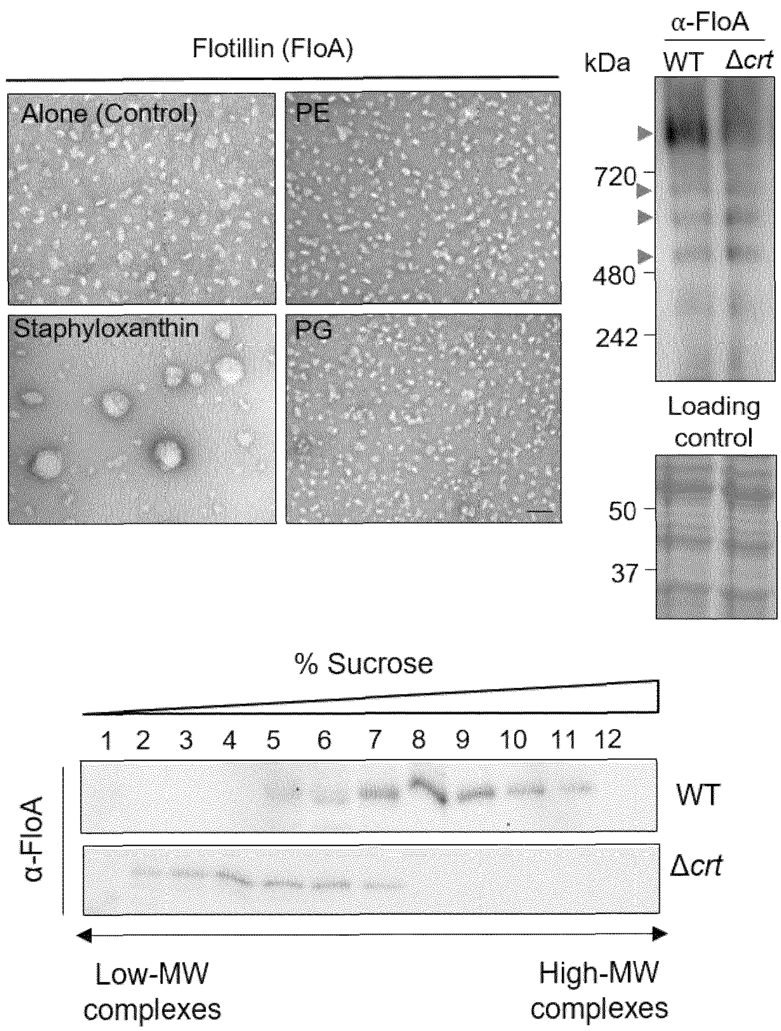
Flotillin (FloA)
Alone (Control)
PE
Staphyloxanthin
PG
α-FloA
kDa
WT
Δcrt
720
480
242
Loading
control
50
37
% Sucrose
1
2
3
4
5
6
7
8
9
10
11
12
α-FloA
WT
Δcrt
Low-MW
complexes
High-MW
complexes

Fig. 4

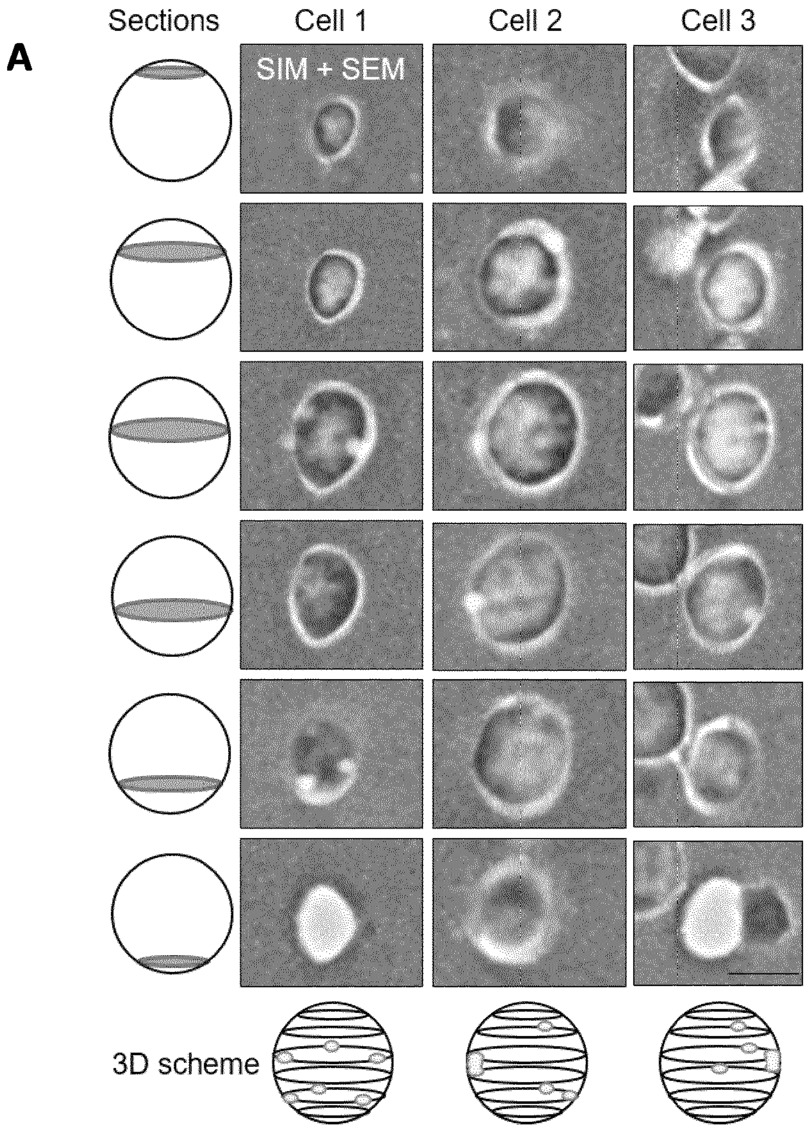

Fig. 4 (cont.)

B

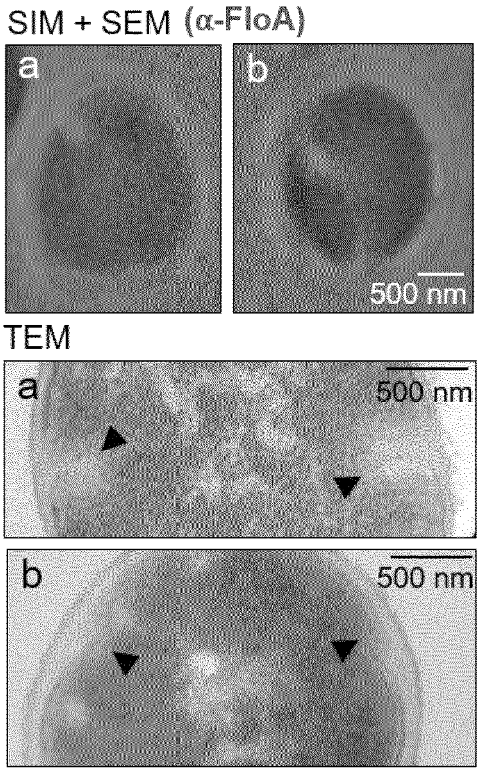


C

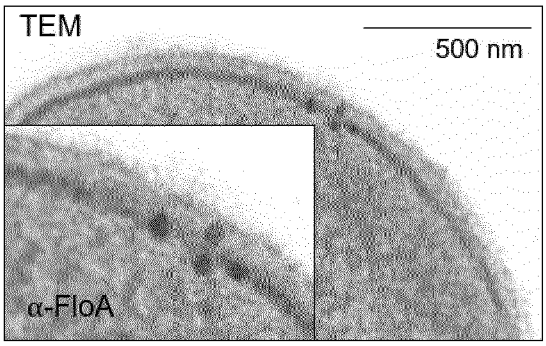


D

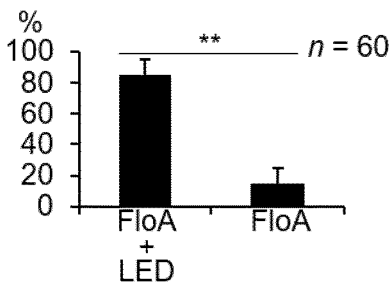

Fig. 5

**A**

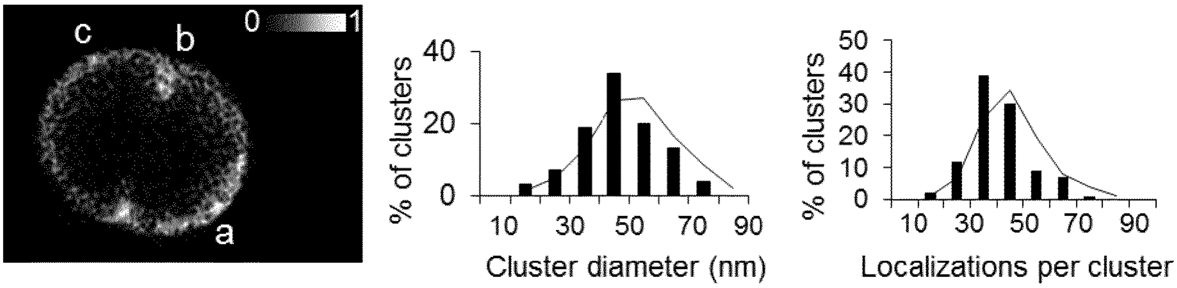
c
b
0
1
a
% of clusters
40
20
0
10
30
50
70
90
Cluster diameter (nm)
% of clusters
50
40
30
20
10
0
10
30
50
70
90
Localizations per cluster

**B**

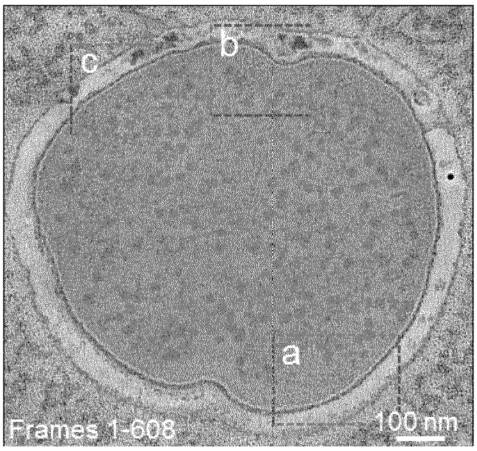
c
b
a
Frames 1-608
100 nm

Fig. 5 (cont.)

C

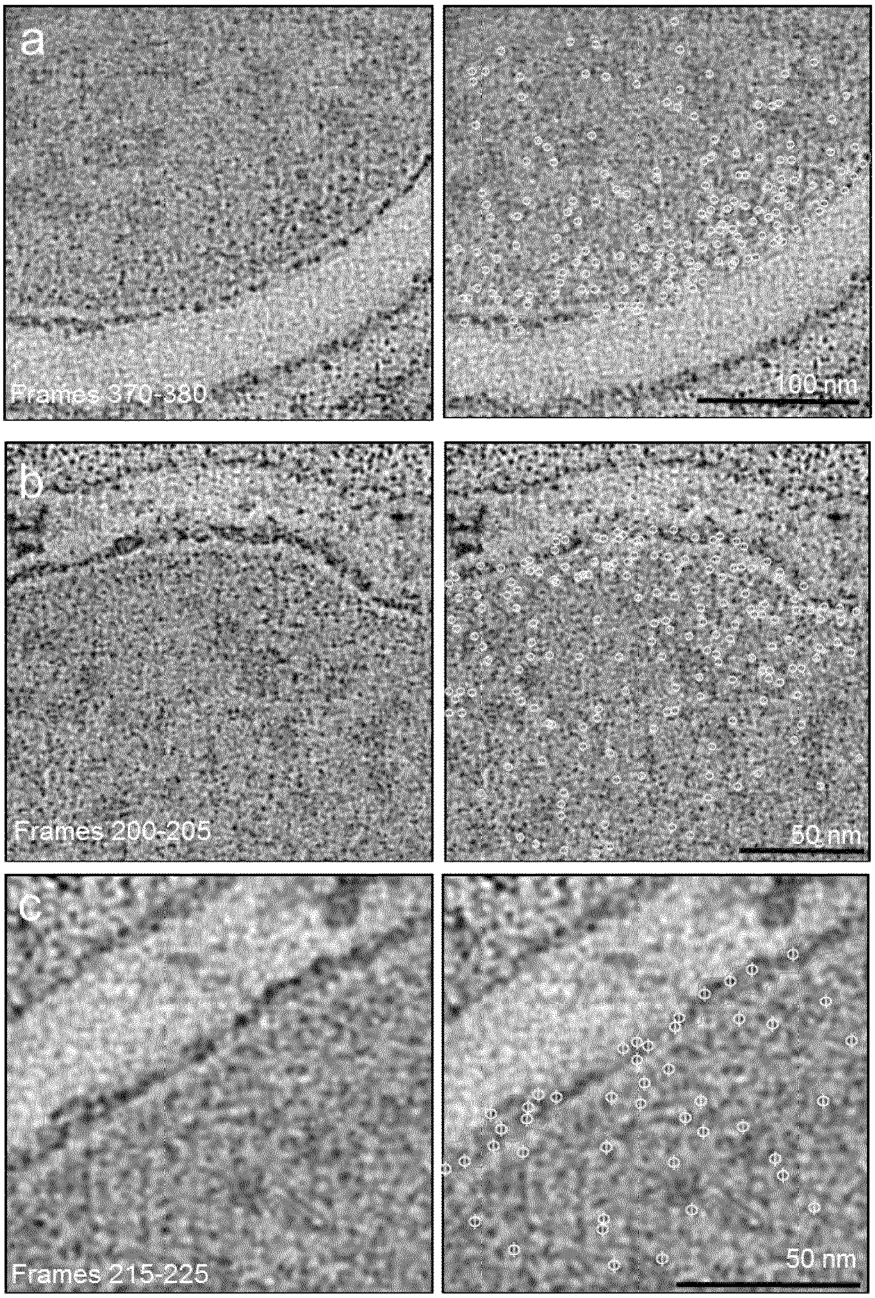
a
Frames 370-380
100 nm
b
Frames 200-205
50 nm
c
Frames 215-225
50 nm

Fig. 5 (cont.)

D

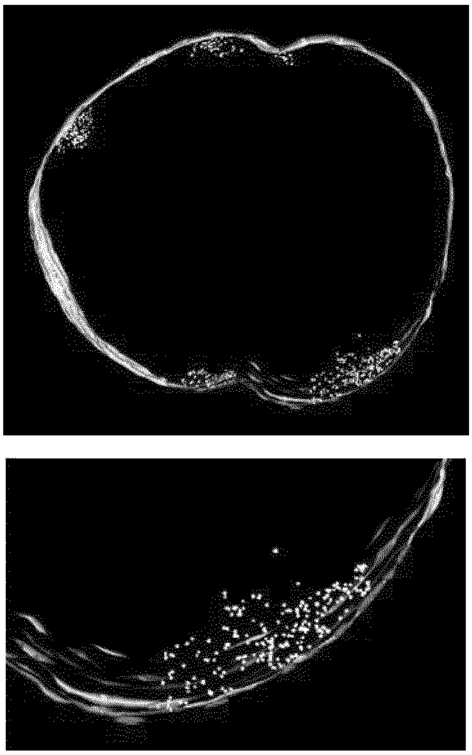

Fig. 6

A

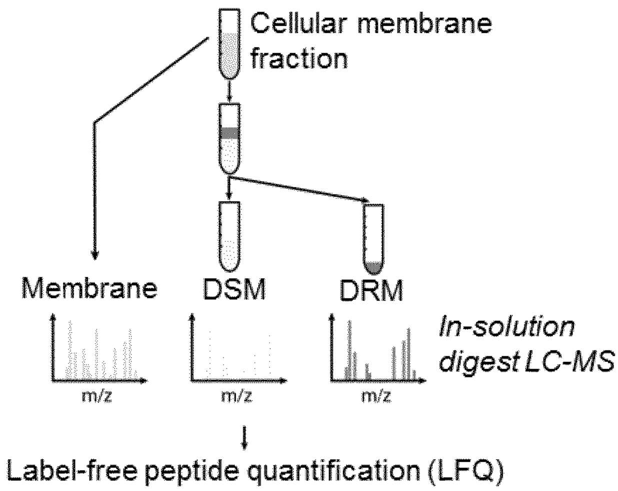
Cellular membrane fraction
Membrane
DSM
DRM
In-solution digest LC-MS
m/z
m/z
m/z
Label-free peptide quantification (LFQ)

Fig. 6 (cont.)

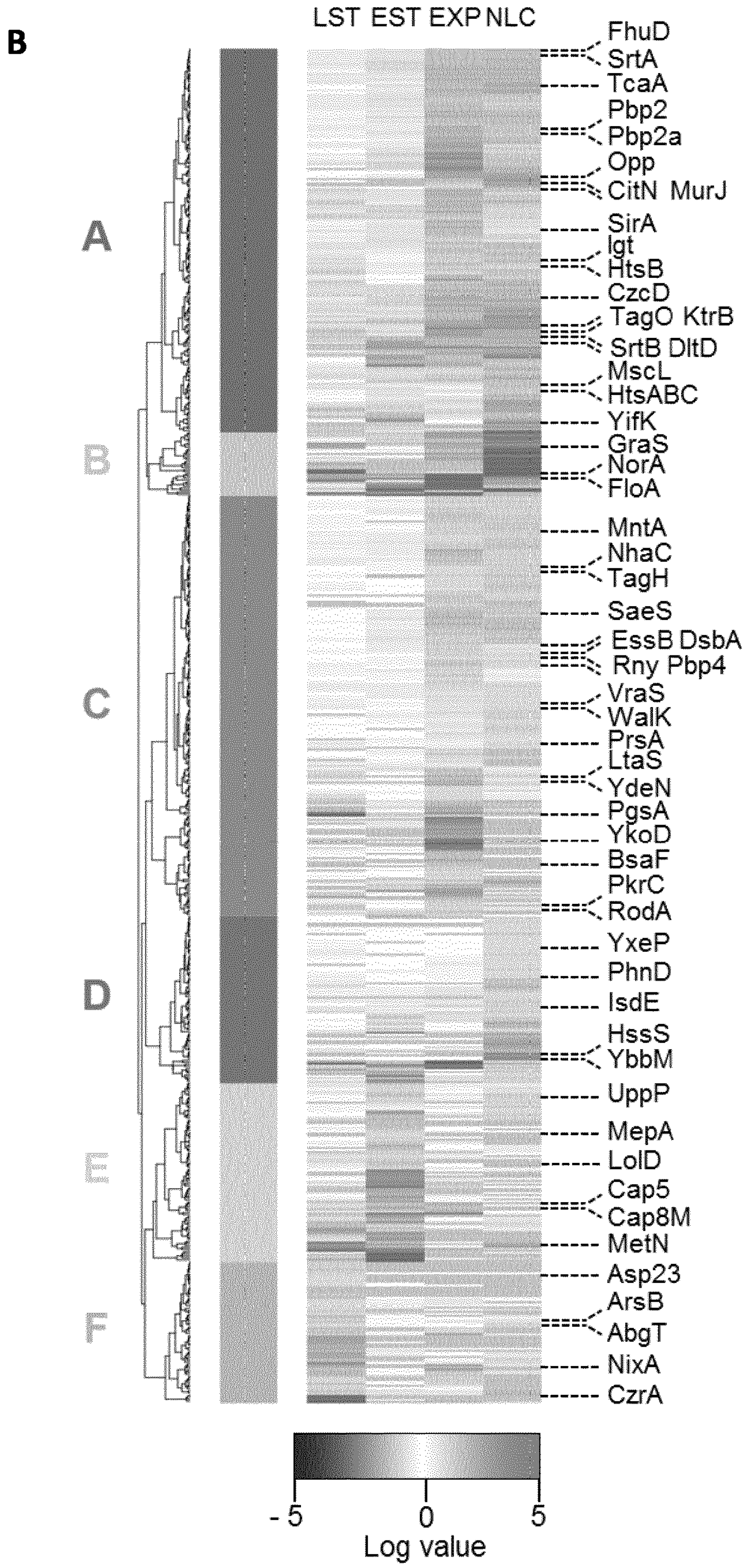
B
LST
EST
EXP
NLC
A
B
C
D
E
F
FhuD
SrtA
TcaA
Pbp2
Pbp2a
Opp
CitN MurJ
SirA
lgt
HtsB
CzcD
TagO KtrB
SrtB DltD
MscL
HtsABC
YifK
GraS
NorA
FloA
MntA
NhaC
TagH
SaeS
EssB DsbA
Rny Pbp4
VraS
WalK
PrsA
LtaS
YdeN
PgsA
YkoD
BsaF
PkrC
RodA
YxeP
PhnD
IsdE
HssS
YbbM
UppP
MepA
LolD
Cap5
Cap8M
MetN
Asp23
ArsB
AbgT
NixA
CzrA
- 5
0
5
Log value

Fig. 6 (cont.)

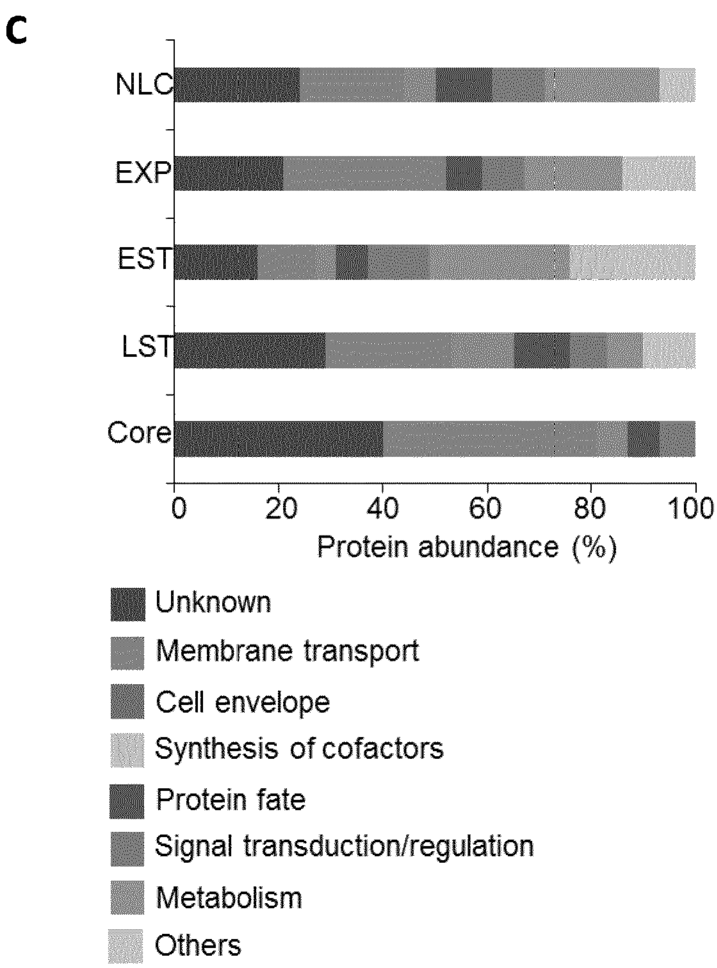
C
NLC
EXP
EST
LST
Core
0
20
40
60
80
100
Protein abundance (%)
Unknown
Membrane transport
Cell envelope
Synthesis of cofactors
Protein fate
Signal transduction/regulation
Metabolism
Others

Fig. 7

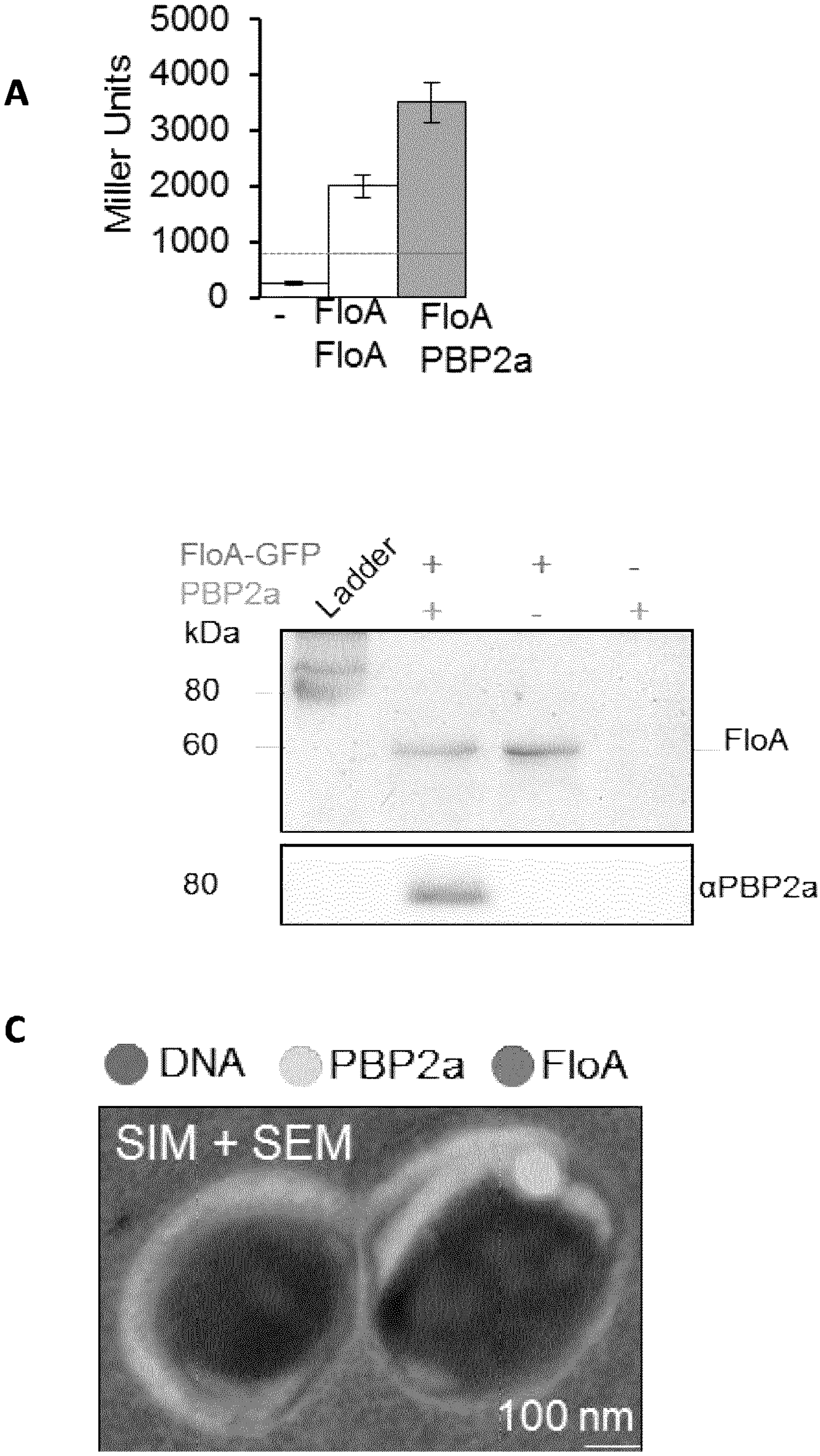

A
Miller Units
5000
4000
3000
2000
1000
0
-
FloA
FloA
FloA
PBP2a
B
FloA-GFP
PBP2a
Ladder
+
+
-
+
-
+
kDa
80
60
FloA
80
αPBP2a
C
DNA
PBP2a
FloA
SIM + SEM
100 nm

Fig. 7 (cont.)

D

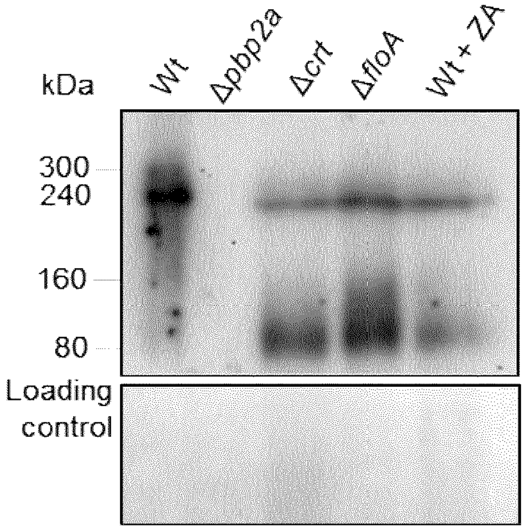

kDa
Wt
Δpbp2a
Δcrt
ΔfloA
Wt + ZA
300
240
160
80
Loading
control


E

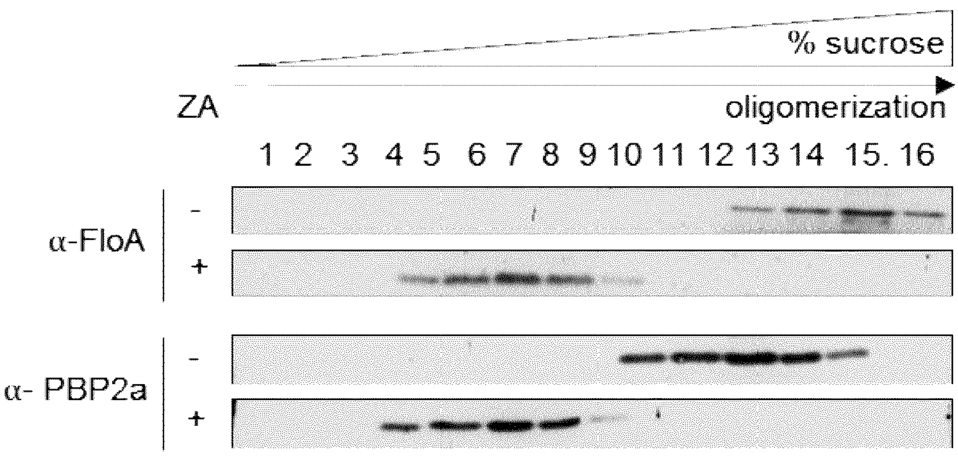

% sucrose
ZA
oligomerization
1 2 3 4 5 6 7 8 9 10 11 12 13 14 15. 16
α-FloA
-
+
α- PBP2a
-
+

Fig. 7 (cont.)

F

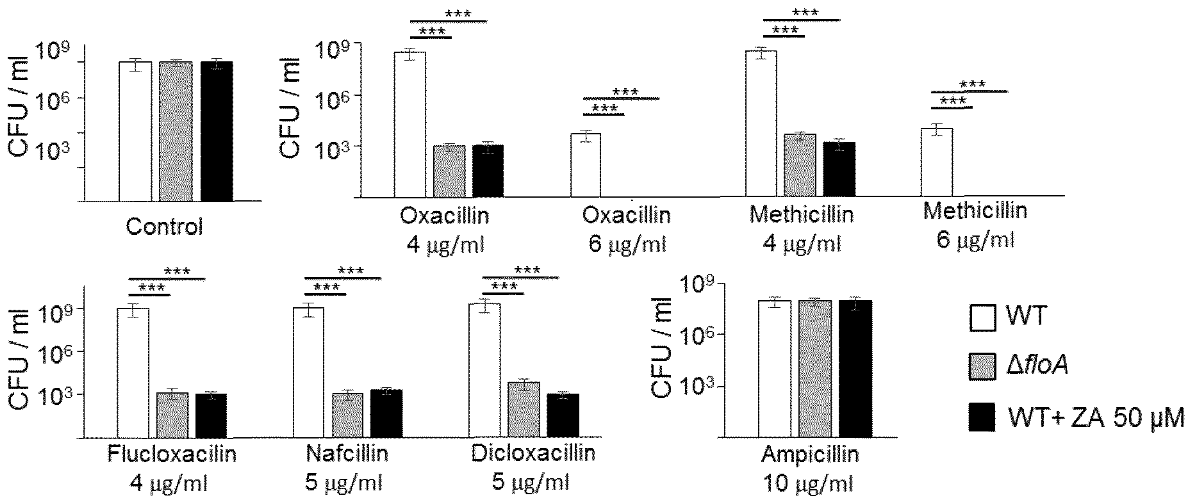
CFU / ml
10^9
10^6
10^3
Control
Oxacillin 4 µg/ml
Oxacillin 6 µg/ml
Methicillin 4 µg/ml
Methicillin 6 µg/ml
Flucloxacilin 4 µg/ml
Nafcillin 5 µg/ml
Dicloxacillin 5 µg/ml
Ampicillin 10 µg/ml
***
WT
ΔfloA
WT+ ZA 50 µM

G

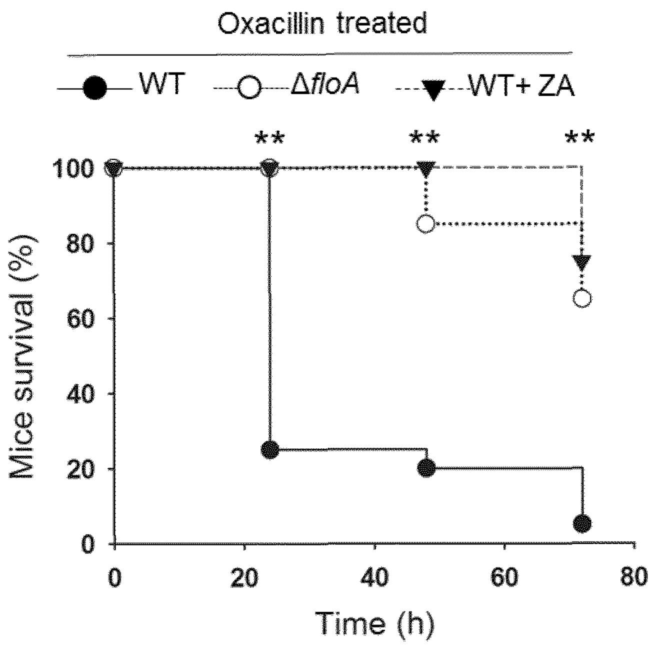
Oxacillin treated
WT
ΔfloA
WT+ ZA
**
**
**
Mice survival (%)
100
80
60
40
20
0
0
20
40
60
80
Time (h)

**Fig. 7 (cont.)**

H

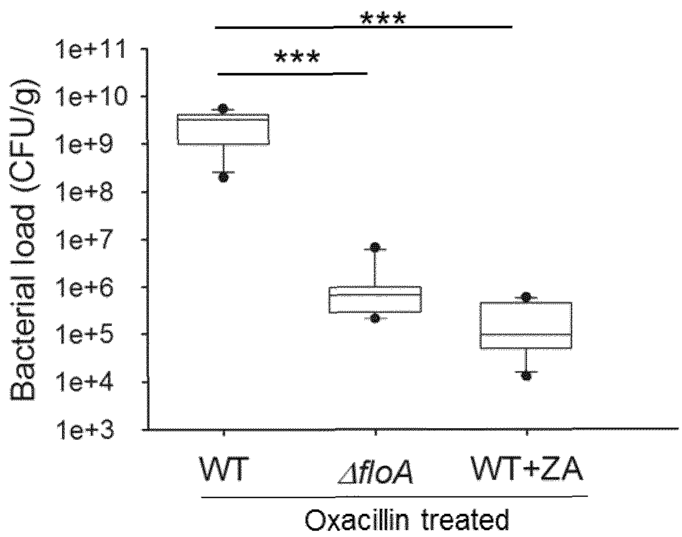


I

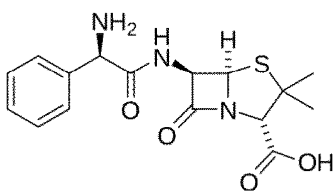

Ampicillin

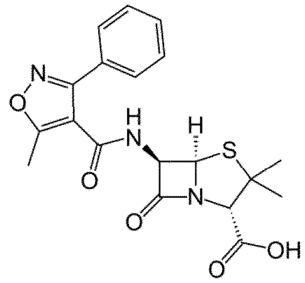

Oxacillin

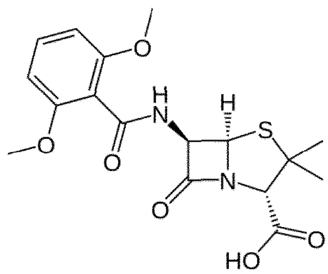

Methicillin

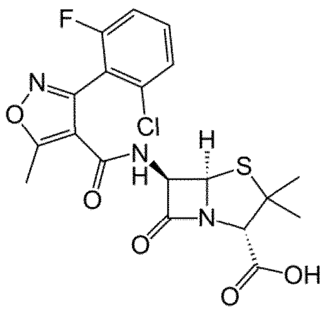

Flucoxacillin

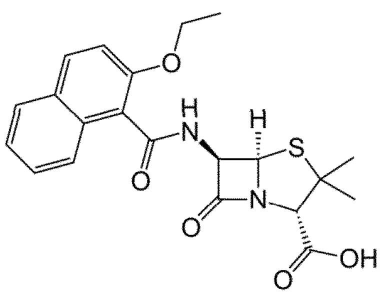

Nafcillin

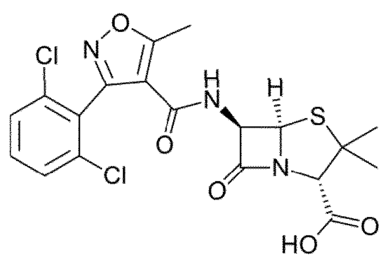

Dicloxacillin

Fig. 8

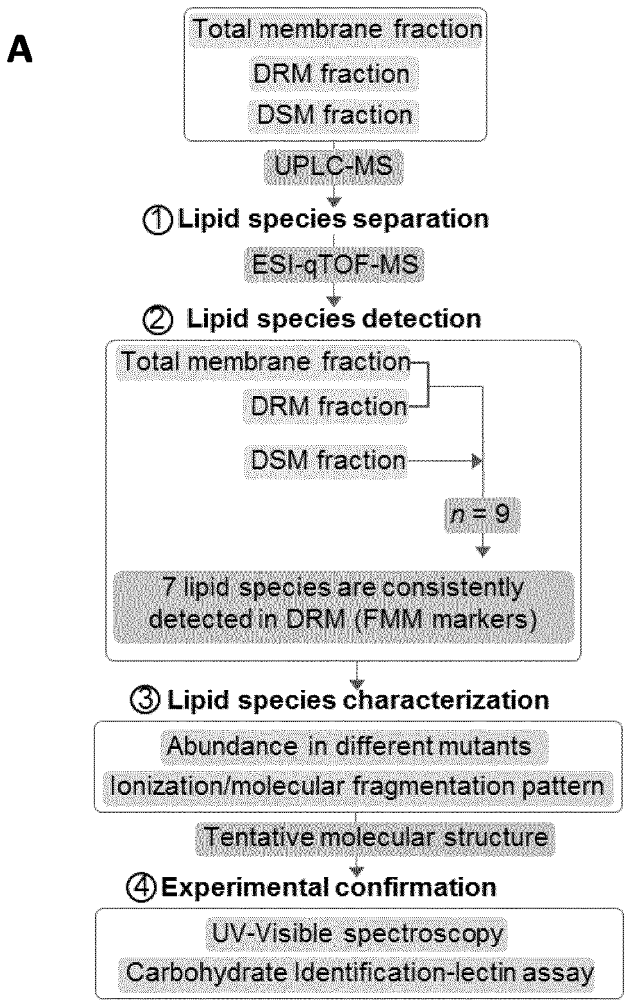
A
Total membrane fraction
DRM fraction
DSM fraction
UPLC-MS
① Lipid species separation
ESI-qTOF-MS
② Lipid species detection
Total membrane fraction
DRM fraction
DSM fraction
n = 9
7 lipid species are consistently detected in DRM (FMM markers)
③ Lipid species characterization
Abundance in different mutants
Ionization/molecular fragmentation pattern
Tentative molecular structure
④ Experimental confirmation
UV-Visible spectroscopy
Carbohydrate Identification-lectin assay

**Fig. 8 (cont.)**

**B**

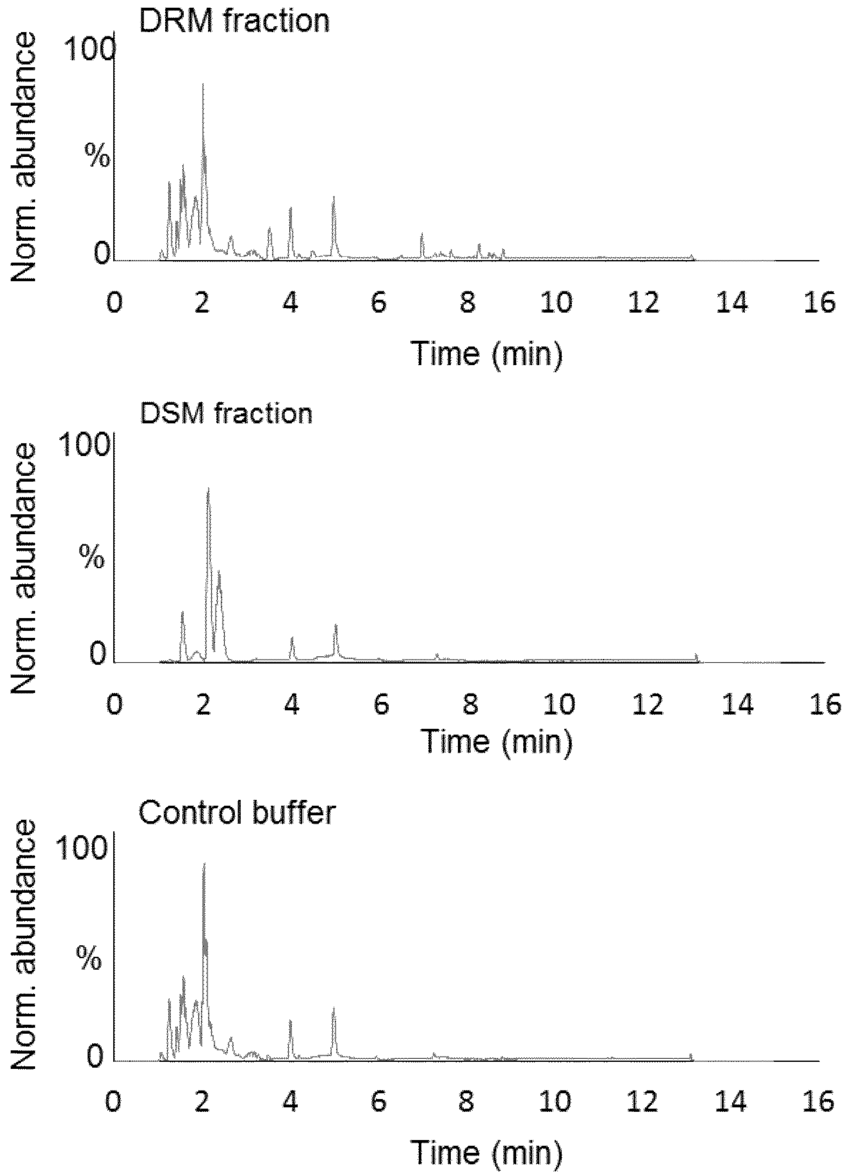
DRM fraction
Norm. abundance
100
%
0
0 2 4 6 8 10 12 14 16
Time (min)
DSM fraction
Norm. abundance
100
%
0
0 2 4 6 8 10 12 14 16
Time (min)
Control buffer
Norm. abundance
100
%
0
0 2 4 6 8 10 12 14 16
Time (min)

Fig. 8 (cont.)

C

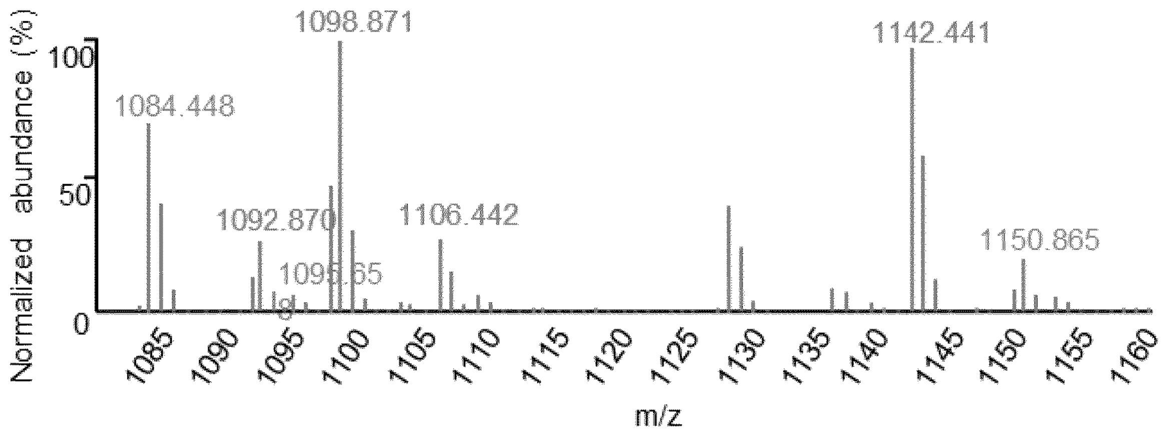
Normalized abundance (%)
100
50
0
1098.871
1142.441
1084.448
1092.870
1106.442
1150.865
1085
1090
1095
1100
1105
1110
1115
1120
1125
1130
1135
1140
1145
1150
1155
1160
m/z

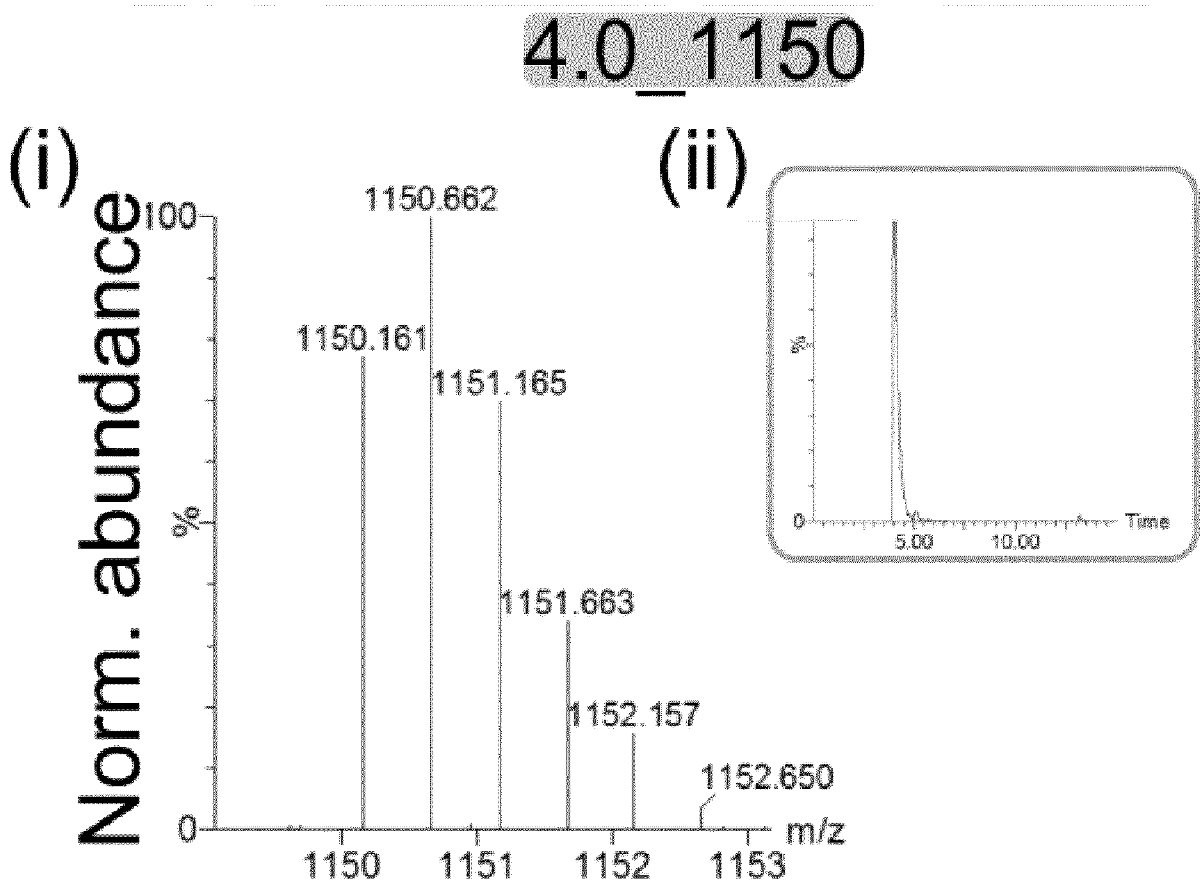
4.0_1150
(i)
(ii)
Norm. abundance
%
100
0
1150.662
1150.161
1151.165
1151.663
1152.157
1152.650
1150
1151
1152
1153
m/z
%
0
Time
5.00
10.00

Fig. 8 (cont.)

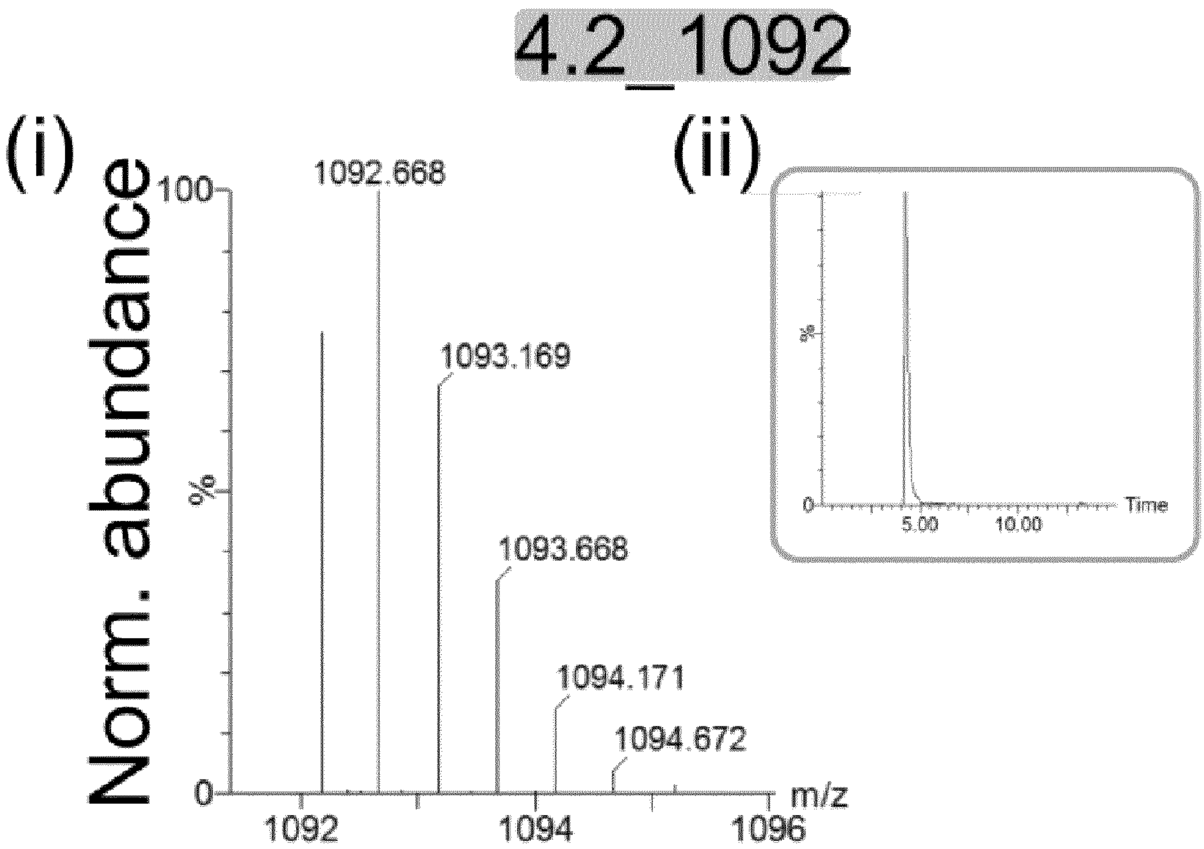
4.2_1092
(i)
(ii)
Norm. abundance
100
%
0
1092.668
1093.169
1093.668
1094.171
1094.672
1092
1094
1096
m/z
Time
5.00
10.00

Fig. 8 (cont.)

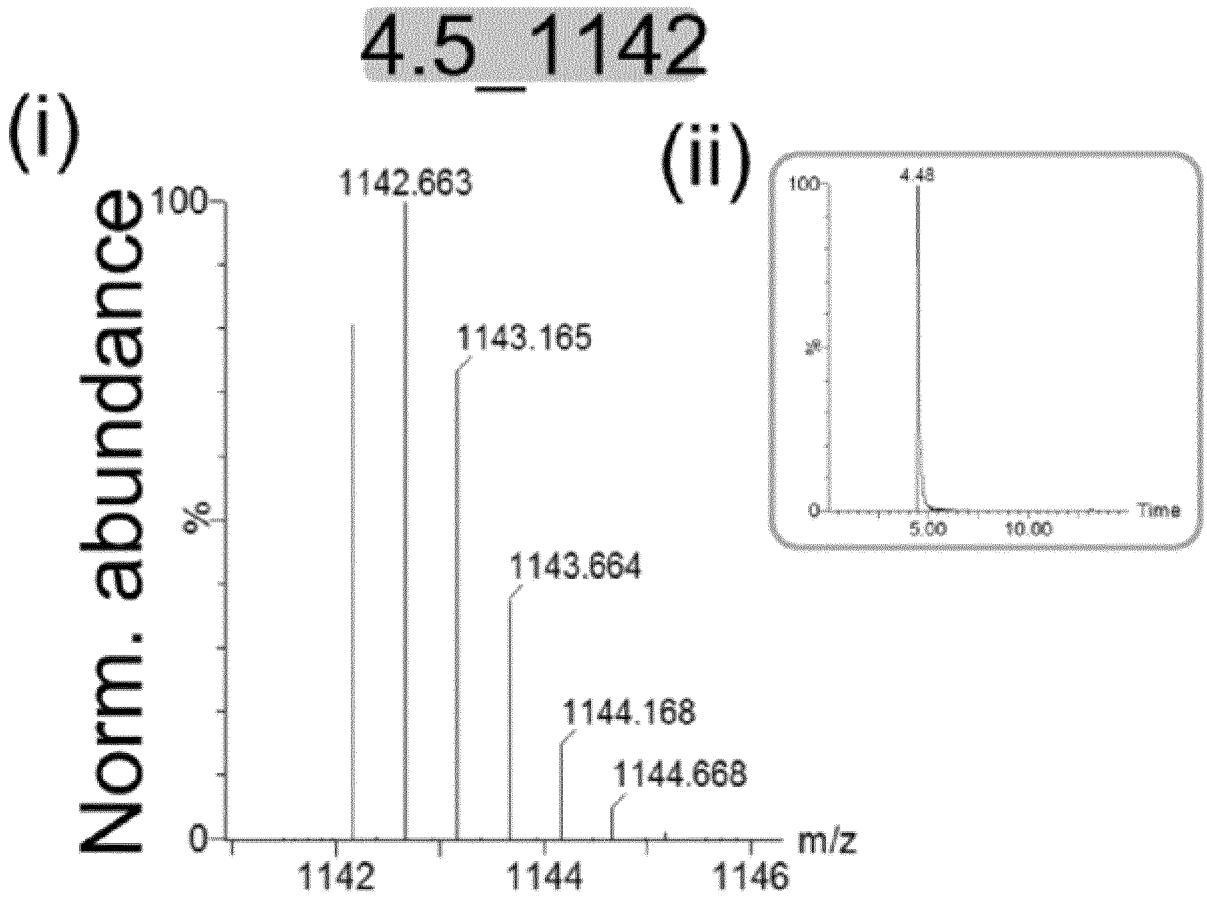
4.5_1142
(i)
(ii)
Norm. abundance
%
100
0
1142.663
1143.165
1143.664
1144.168
1144.668
1142
1144
1146
m/z
100
4.48
%
0
5.00
10.00
Time

Fig. 8 (cont.)

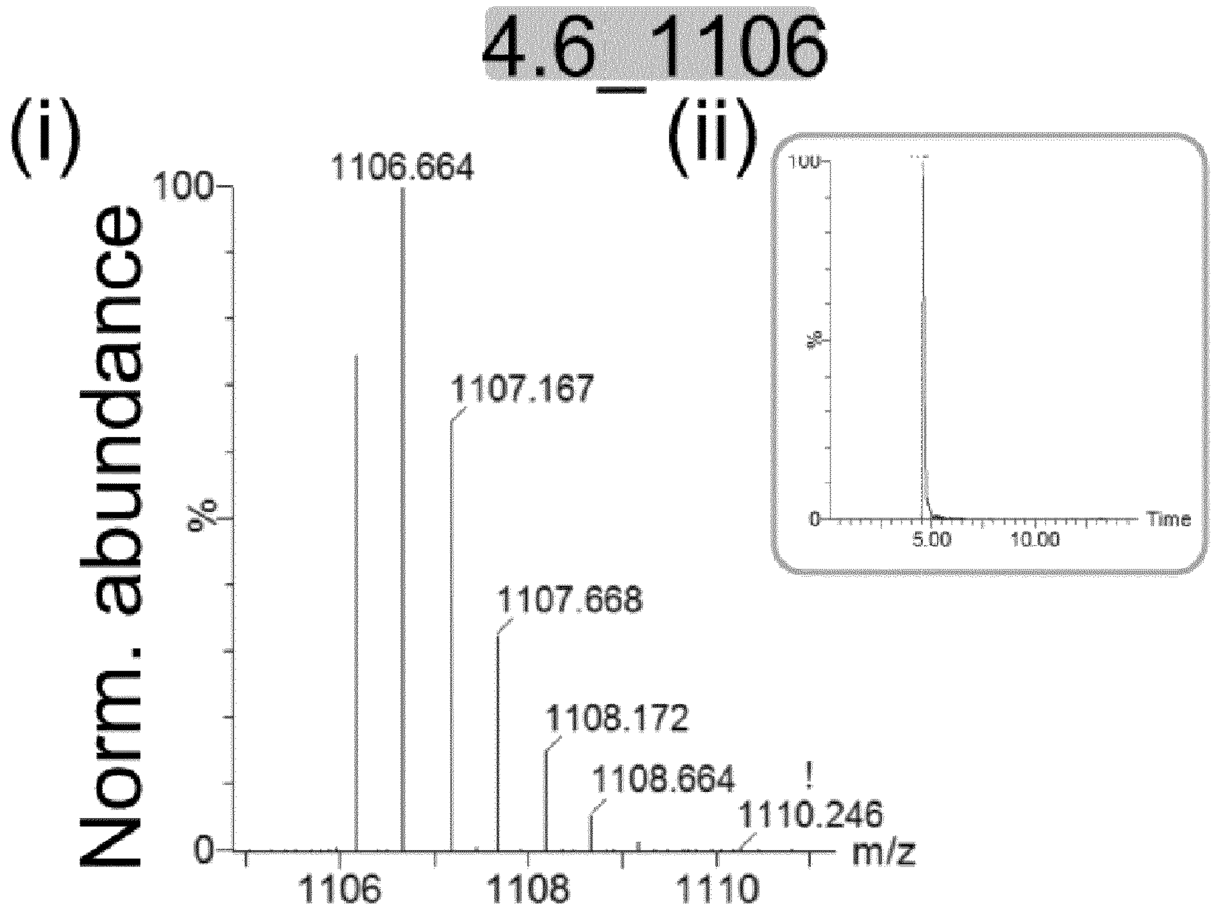
4.6_1106
(i)
(ii)
Norm. abundance
%
100
0
1106.664
1107.167
1107.668
1108.172
1108.664
1110.246
!
1106
1108
1110
m/z
100
%
0
Time
5.00
10.00

Fig. 8 (cont.)

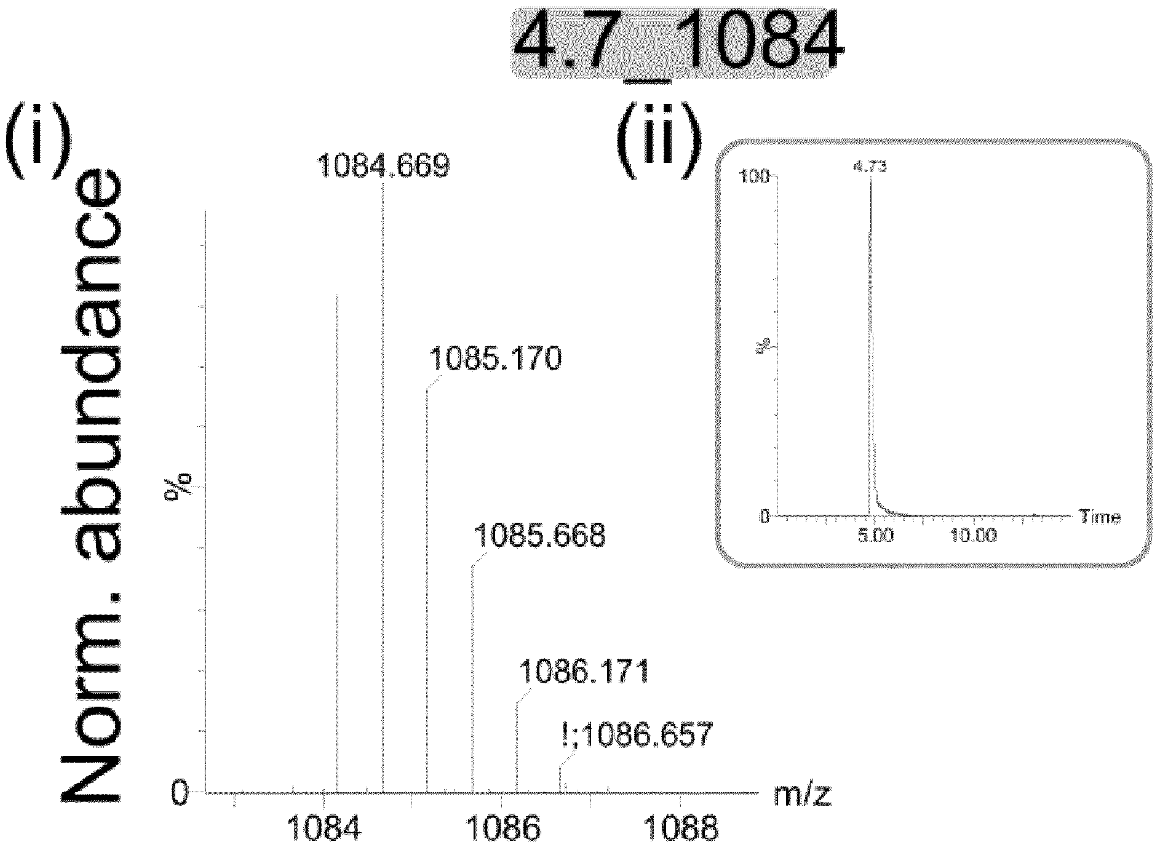
4.7_1084
(i)
(ii)
Norm. abundance
%
1084.669
1085.170
1085.668
1086.171
!;1086.657
0
1084
1086
1088
m/z
100
4.73
%
0
5.00
10.00
Time

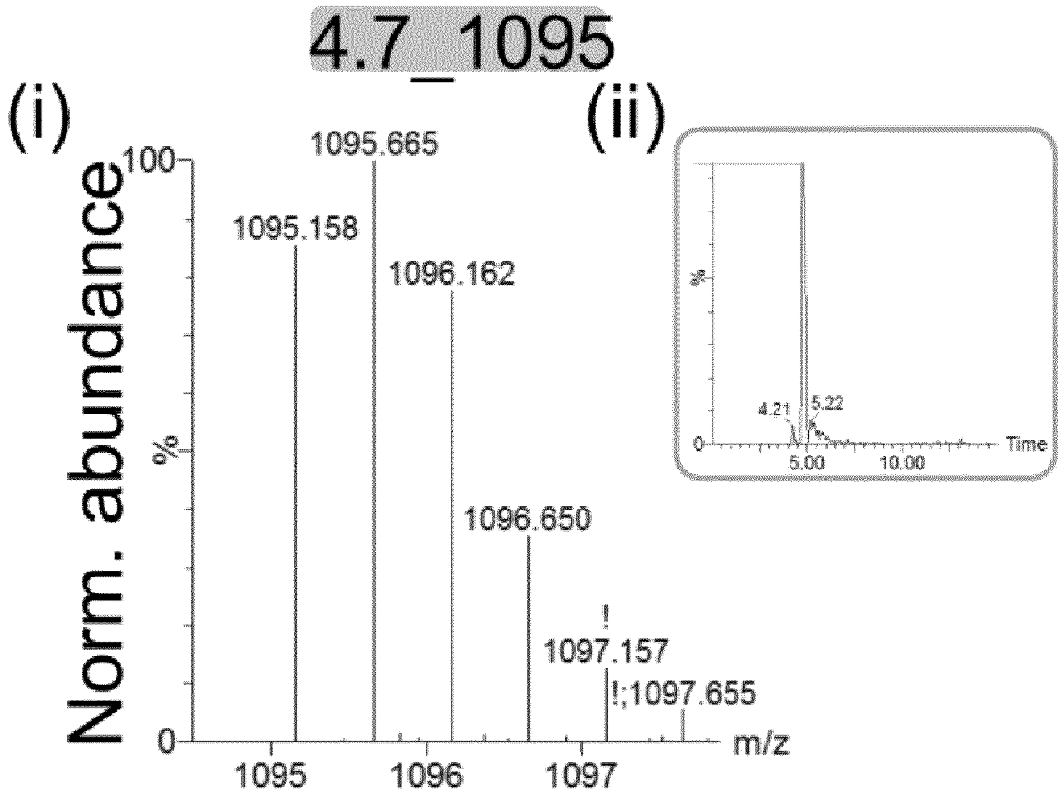
4.7_1095
(i)
(ii)
Norm. abundance
100
%
0
1095.665
1095.158
1096.162
1096.650
!
1097.157
!;1097.655
1095
1096
1097
m/z
%
4.21
5.22
0
5.00
10.00
Time

Fig. 8 (cont.)

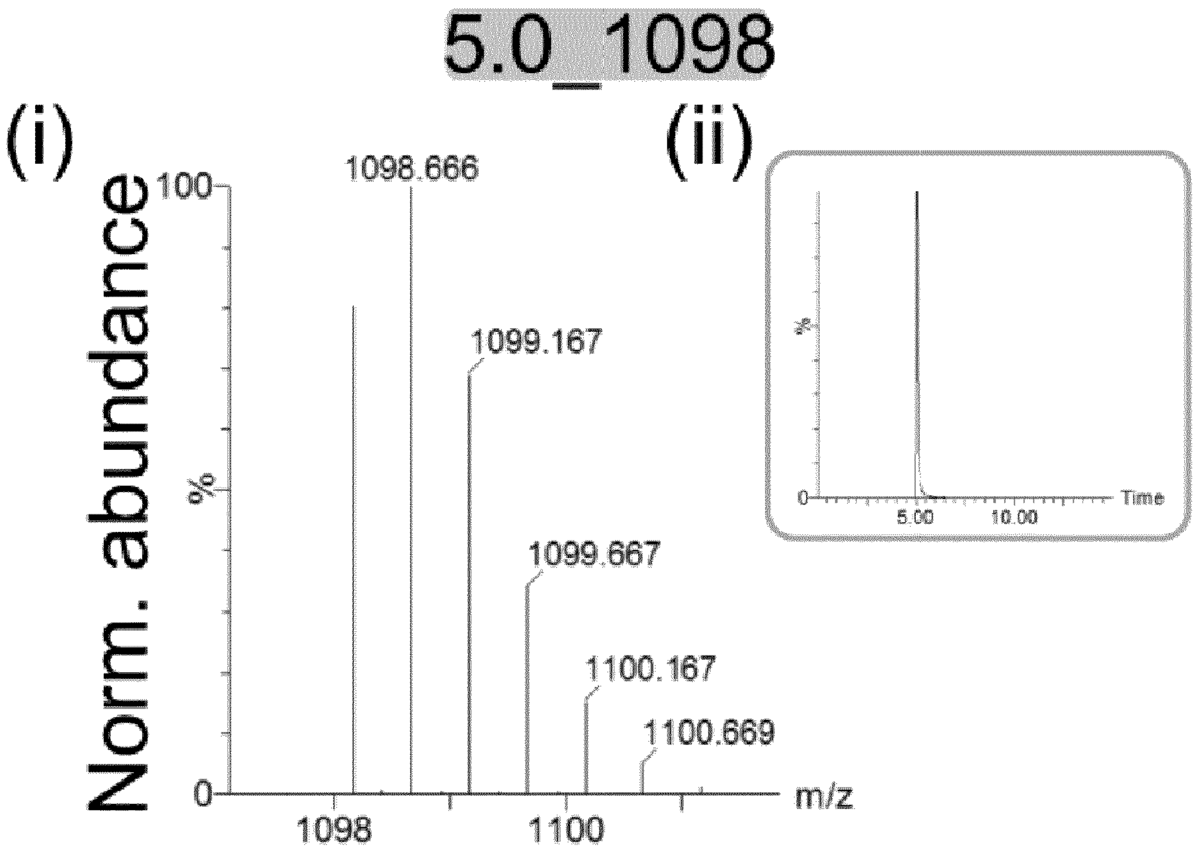
5.0_1098
(i)
(ii)
Norm. abundance
%
100
0
1098.666
1099.167
1099.667
1100.167
1100.669
1098
1100
m/z
%
0
Time
5.00
10.00

Fig. 9

**A**

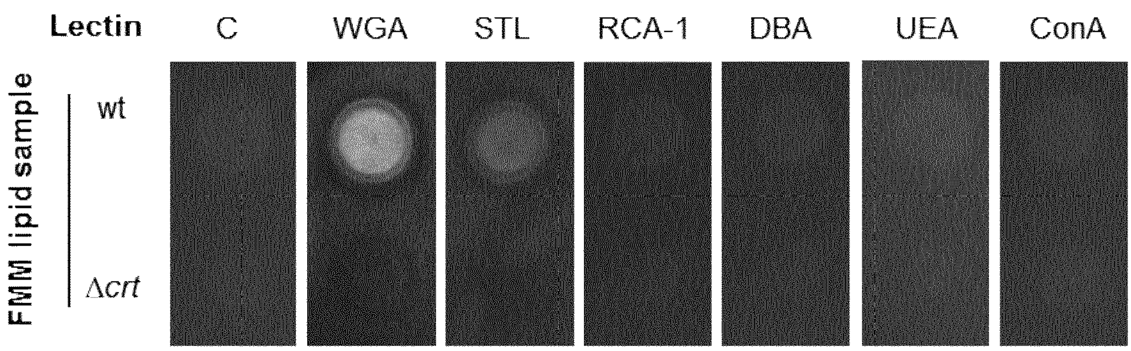


| Lectin | Abbreviation | Sugar recognition specificity |
|---|---|---|
| Wheat germ agglutinin | WGA | N-Acetylglucosamine |
| *Solanun tuberosum* lectin | STL | N-Acetylglucosamine-N-Acetylmuramic oligosaccharides |
| *Ricinus communis* agglutinin | RCA-1 | Galactose / N-Acetylgalactosamine |
| Dolichos biflorus agglutinin | DBA | N-acetylgalactosamine |
| *Ullex Europaeus* agglutinin-1 | UEA | Fucose / Arabinose |
| Concanavalin A | ConA | Mannose |

Fig. 9 (cont.)

B

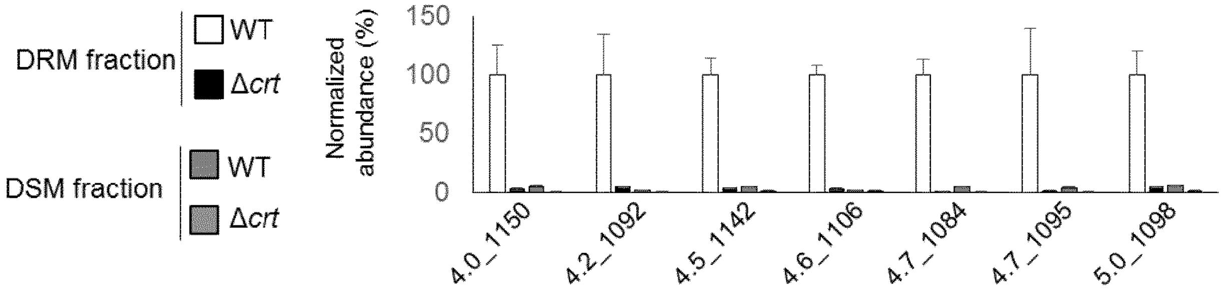


C

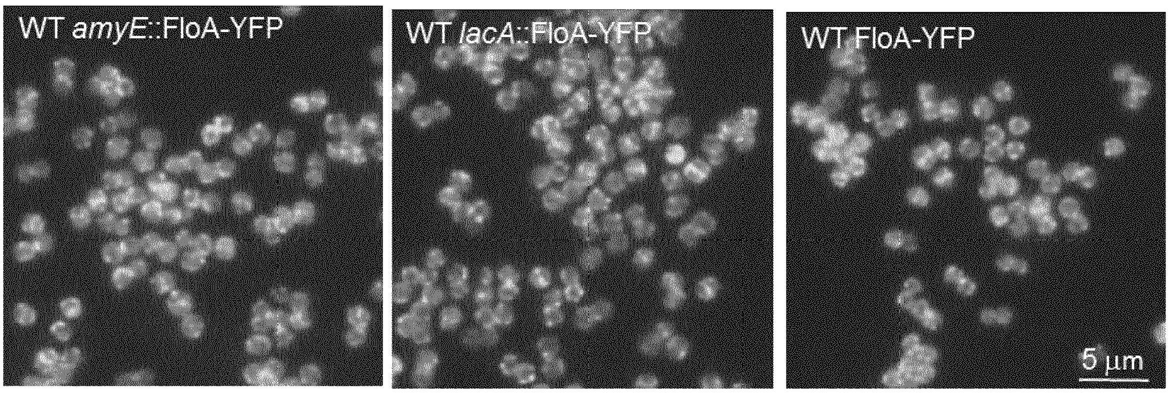


D

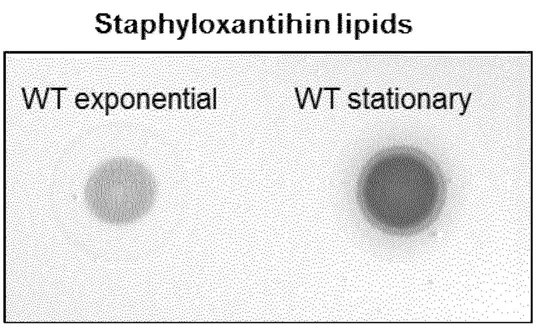

Fig. 10

A

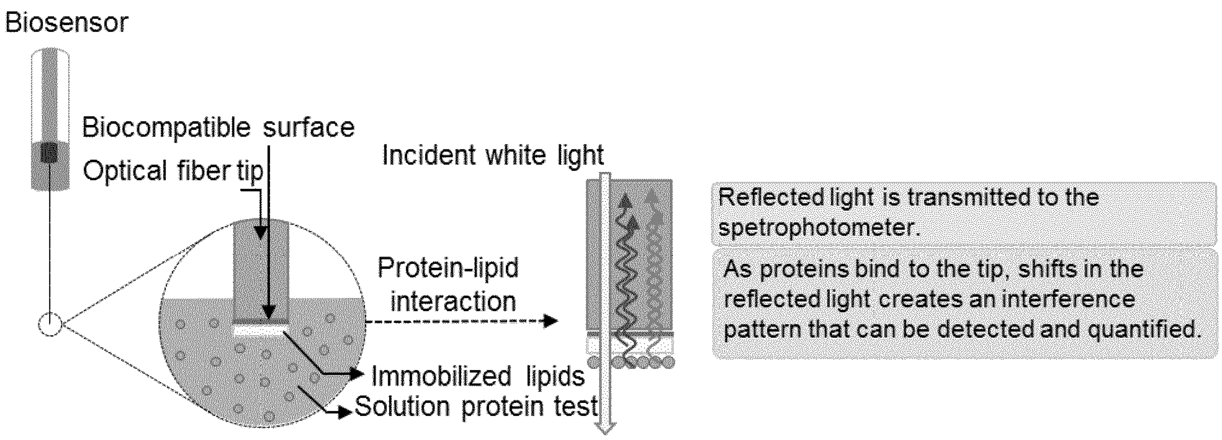
Biosensor
Biocompatible surface
Optical fiber tip
Incident white light
Protein-lipid interaction
Immobilized lipids
Solution protein test
Reflected light is transmitted to the spetrophotometer.
As proteins bind to the tip, shifts in the reflected light creates an interference pattern that can be detected and quantified.

Fig. 10 (Cont.)

B

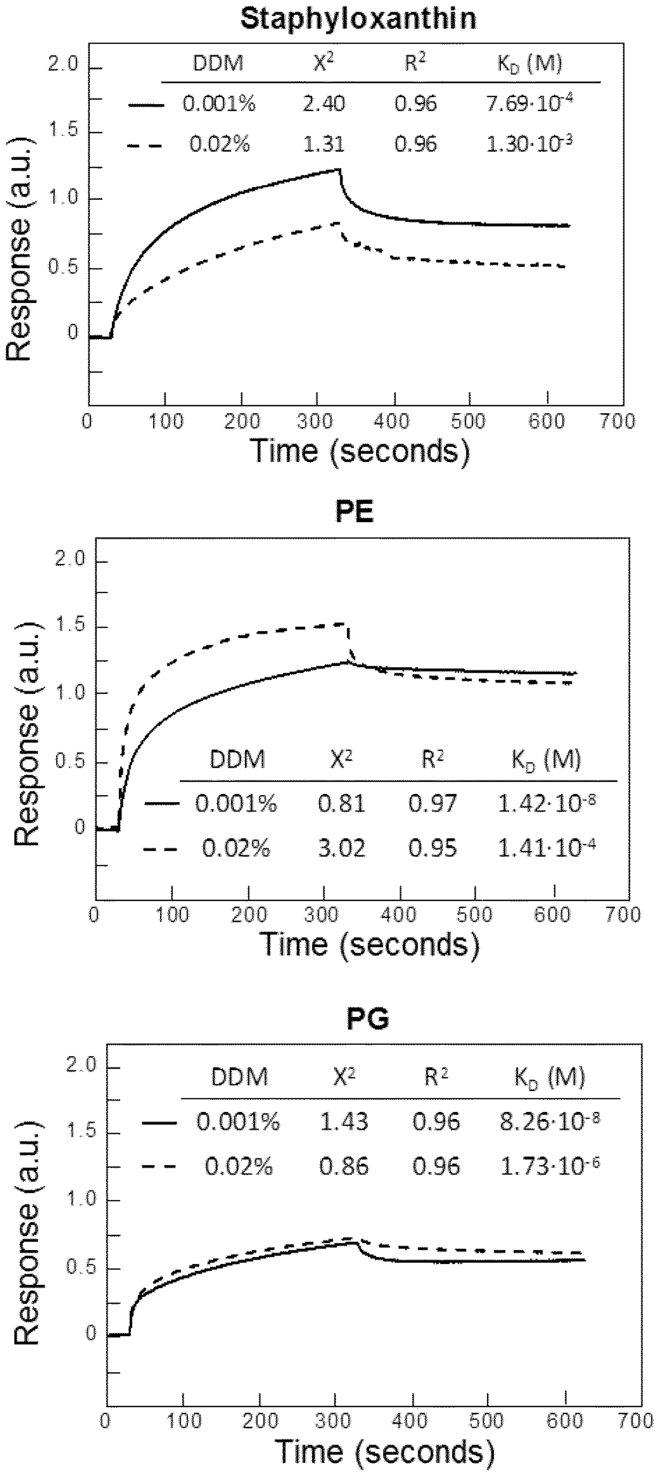
Staphyloxanthin
DDM $X^2$ $R^2$ $K_D$ (M)
0.001% 2.40 0.96 $7.69\cdot10^{-4}$
0.02% 1.31 0.96 $1.30\cdot10^{-3}$
Response (a.u.)
2.0 1.5 1.0 0.5 0
0 100 200 300 400 500 600 700
Time (seconds)
PE
DDM $X^2$ $R^2$ $K_D$ (M)
0.001% 0.81 0.97 $1.42\cdot10^{-8}$
0.02% 3.02 0.95 $1.41\cdot10^{-4}$
Response (a.u.)
2.0 1.5 1.0 0.5 0
0 100 200 300 400 500 600 700
Time (seconds)
PG
DDM $X^2$ $R^2$ $K_D$ (M)
0.001% 1.43 0.96 $8.26\cdot10^{-8}$
0.02% 0.86 0.96 $1.73\cdot10^{-6}$
Response (a.u.)
2.0 1.5 1.0 0.5 0
0 100 200 300 400 500 600 700
Time (seconds)

Fig. 10 (Cont.)

| STX | Flotillin | | | |
|---|---|---|---|---|
| | WT | ΔMAR | ΔPHB | ΔEA4 |
| $K_D$ (M) | $3.49 \cdot 10^{-8}$ | $1.20 \cdot 10^{-8}$ | $1.27 \cdot 10^{-7}$ | $9.43 \cdot 10^{-9}$ |
| $K_D$ Error (M) | $0.467 \cdot 10^{-8}$ | $0.042 \cdot 10^{-8}$ | $0.007 \cdot 10^{-7}$ | $0.106 \cdot 10^{-9}$ |
| $k_a(M^{-1}\ s^{-1})$ | $7.15 \cdot 10^{4}$ | $1.03 \cdot 10^{5}$ | $5.86 \cdot 10^{4}$ | $7.31 \cdot 10^{4}$ |
| $K_d\ (s^{-1})$ | $2.52 \cdot 10^{-3}$ | $2.50 \cdot 10^{-4}$ | $7.40 \cdot 10^{-3}$ | $6.95 \cdot 10^{-4}$ |

| | | Flotillin | | | |
|---|---|---|---|---|---|
| | | WT | ΔMAR | ΔPHB | ΔEA4 |
| STX | $X^2$ | 0.65 | 0.84 | 1.01 | 3.09 |
| | $R^2$ | 0.98 | 0.99 | 0.97 | 0.98 |
| PG | $X^2$ | 0.94 | 0.38 | 0.16 | 0.9 |
| | $R^2$ | 0.88 | 0.93 | 0.87 | 0.00 |
| PE | $X^2$ | 0.72 | 0.68 | 0.37 | 3.3 |
| | $R^2$ | 0.71 | 0.90 | 0.93 | 0.45 |

**C**

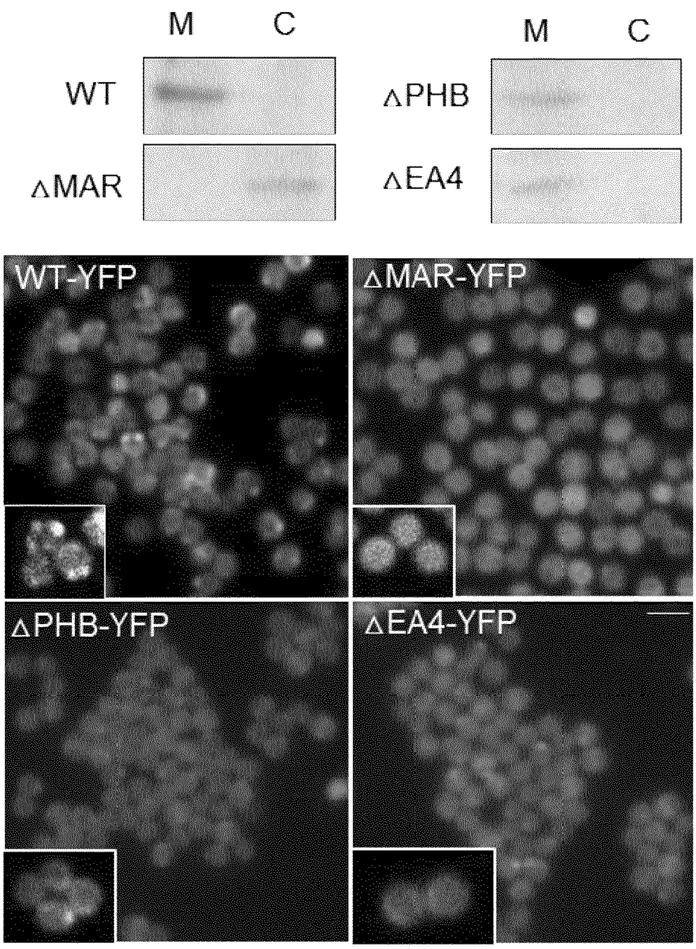

Fig. 11

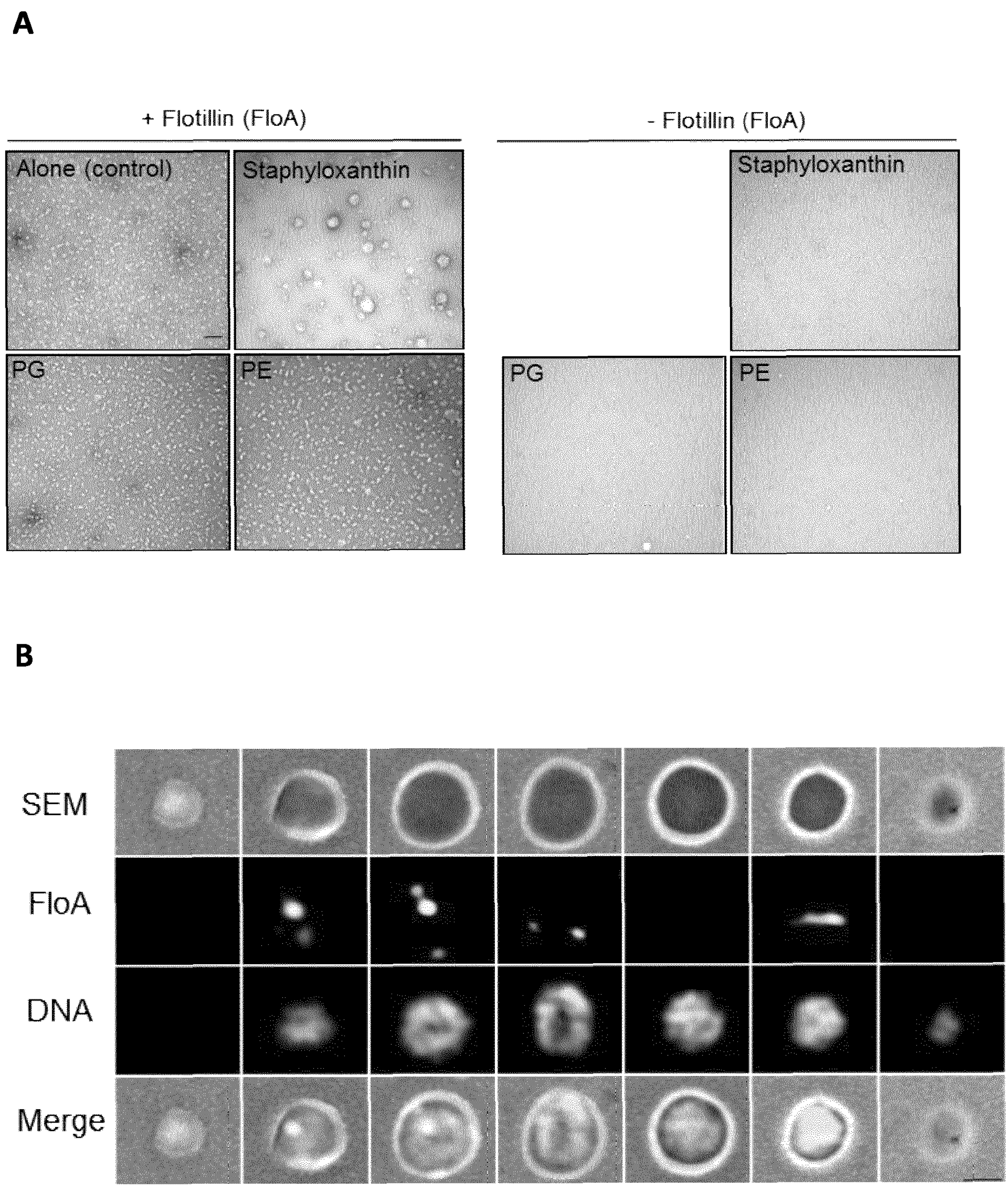
A
+ Flotillin (FloA)
- Flotillin (FloA)
Alone (control)
Staphyloxanthin
Staphyloxanthin
PG
PE
PG
PE
B
SEM
FloA
DNA
Merge

Fig. 11 (Cont.)

C

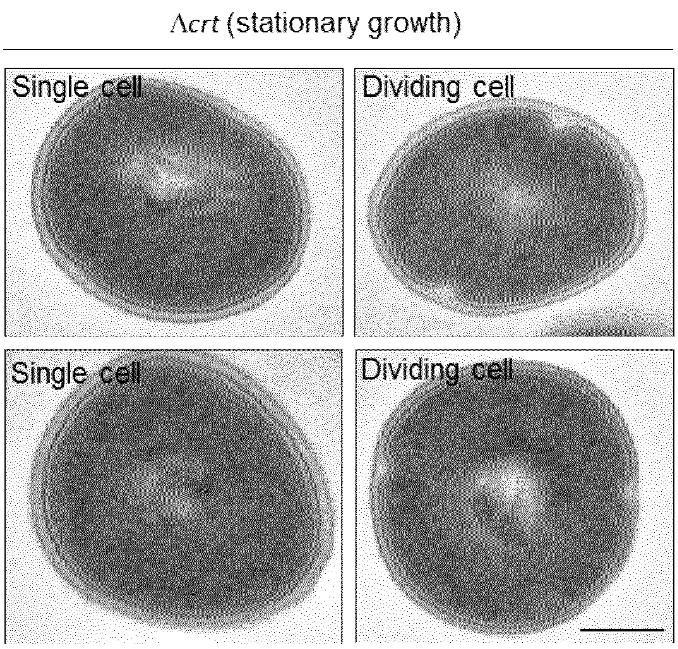


D

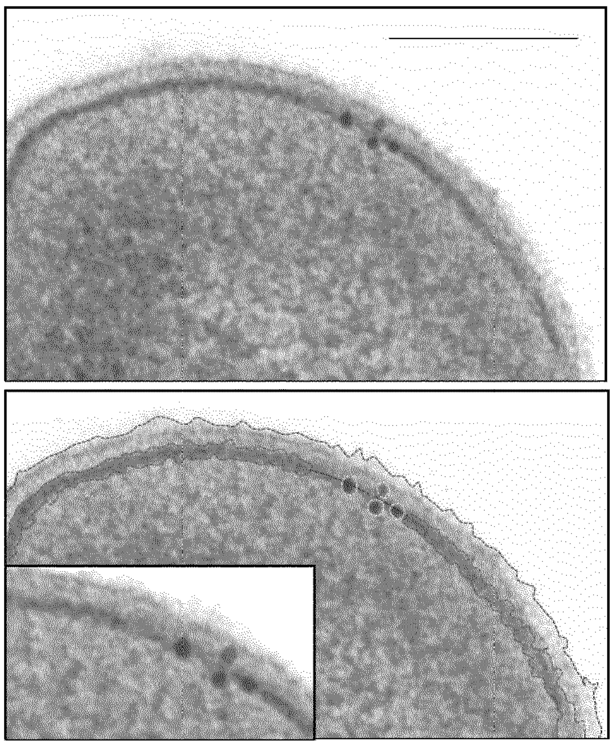

Fig. 12

A

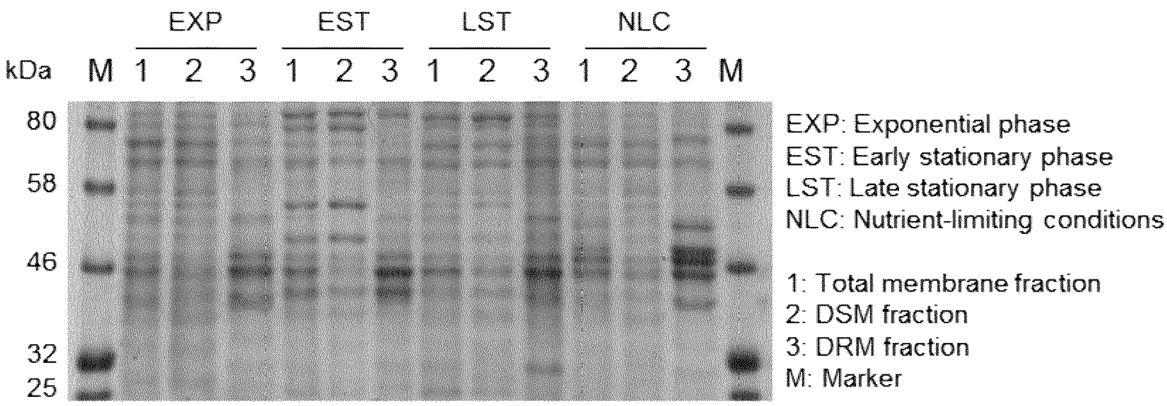
EXP
EST
LST
NLC
kDa
M 1 2 3 1 2 3 1 2 3 1 2 3 M
80
58
46
32
25
EXP: Exponential phase
EST: Early stationary phase
LST: Late stationary phase
NLC: Nutrient-limiting conditions
1: Total membrane fraction
2: DSM fraction
3: DRM fraction
M: Marker

Fig. 12(cont.)

**B**

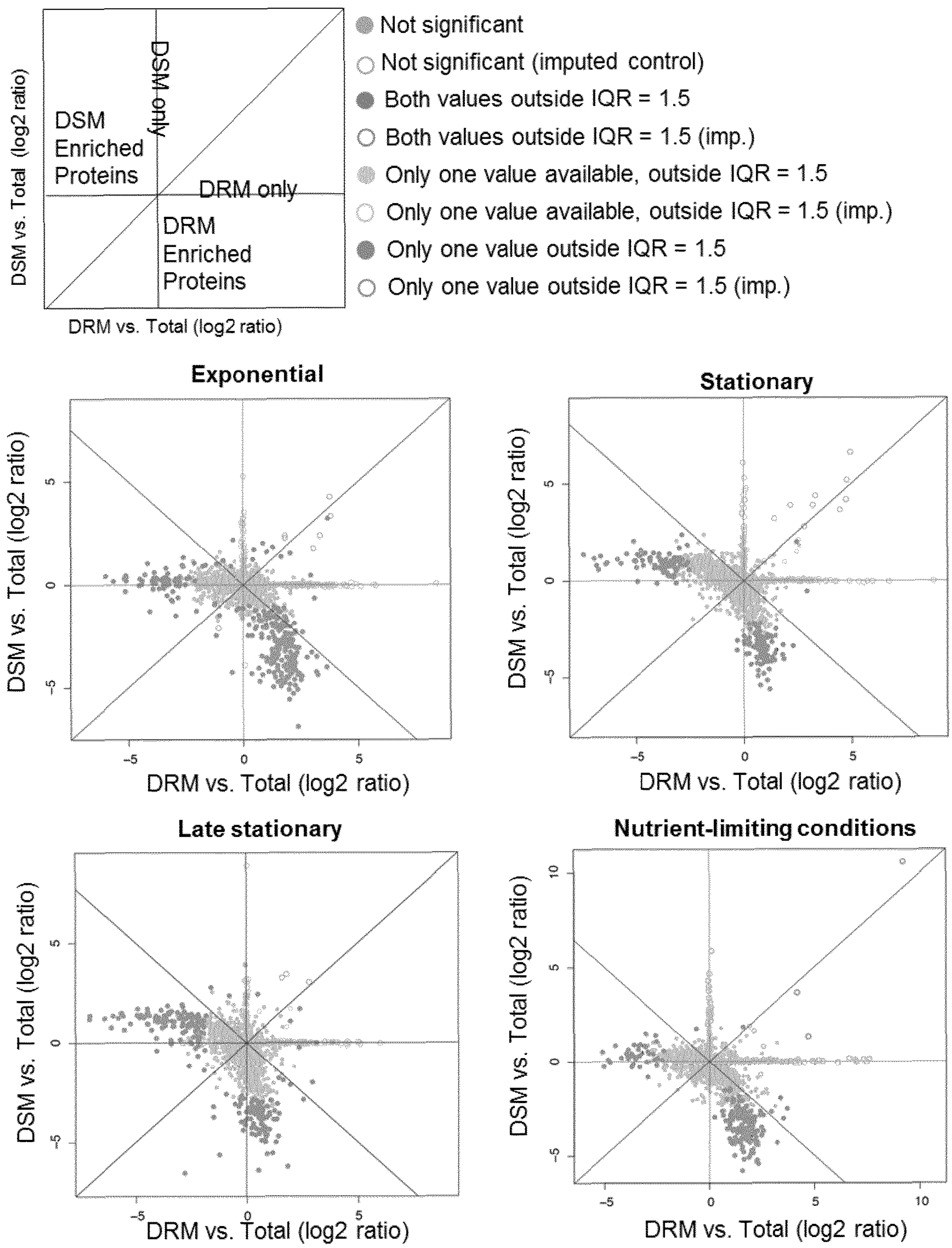
DSM vs. Total (log2 ratio)
DSM Enriched Proteins
DSM only
DRM only
DRM Enriched Proteins
DRM vs. Total (log2 ratio)
Not significant
Not significant (imputed control)
Both values outside IQR = 1.5
Both values outside IQR = 1.5 (imp.)
Only one value available, outside IQR = 1.5
Only one value available, outside IQR = 1.5 (imp.)
Only one value outside IQR = 1.5
Only one value outside IQR = 1.5 (imp.)
Exponential
Stationary
Late stationary
Nutrient-limiting conditions
DSM vs. Total (log2 ratio)
DRM vs. Total (log2 ratio)

Fig. 13

A

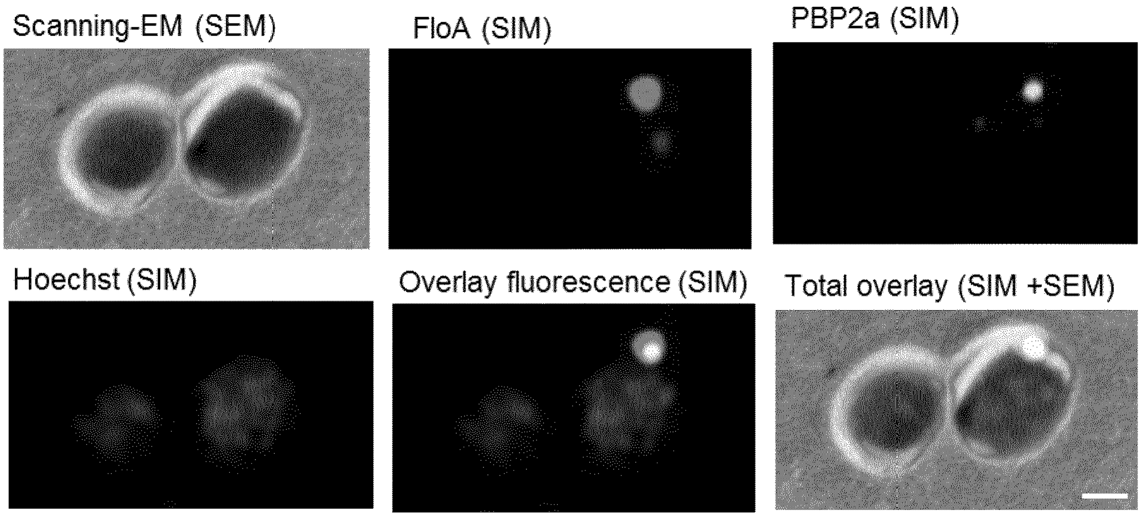
Scanning-EM (SEM)
FloA (SIM)
PBP2a (SIM)
Hoechst (SIM)
Overlay fluorescence (SIM)
Total overlay (SIM +SEM)

B

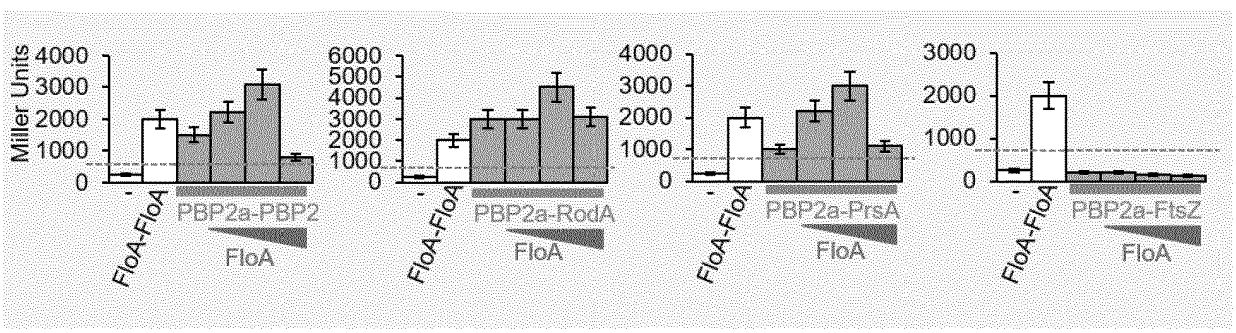
Miller Units
4000
3000
2000
1000
0
-
FloA-FloA
PBP2a-PBP2
FloA
6000
5000
4000
3000
2000
1000
0
PBP2a-RodA
PBP2a-PrsA
3000
PBP2a-FtsZ

**Fig. 13 (Cont.)**

## C

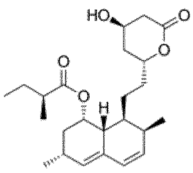

Lovastatin (L)

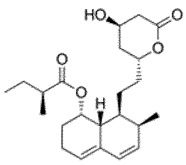

Mevastatin (M)

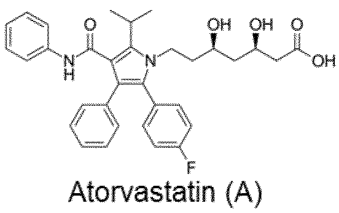

Atorvastatin (A)

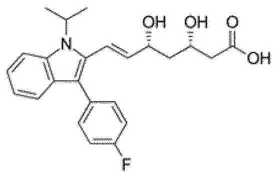

Fluvastatin (F)

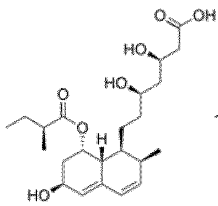

Pravastatin (P)

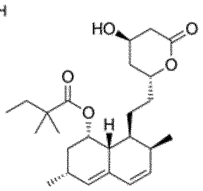

Simvastatin (S)

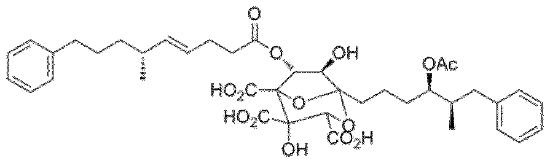

Squalestatin (Zaragozic acid, ZA)

## D

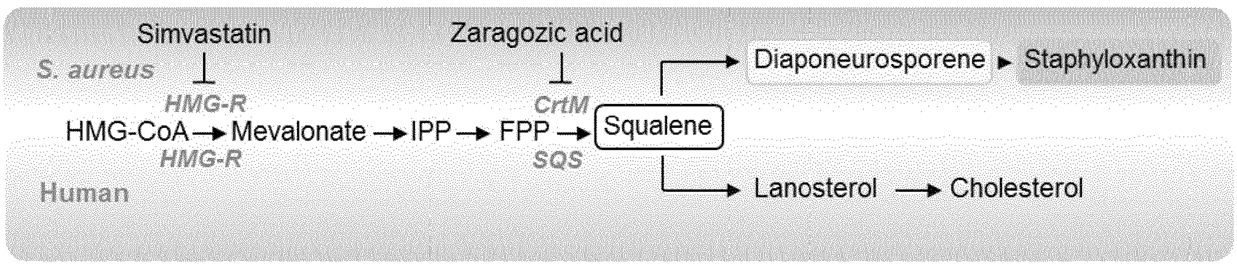

Fig. 14

A

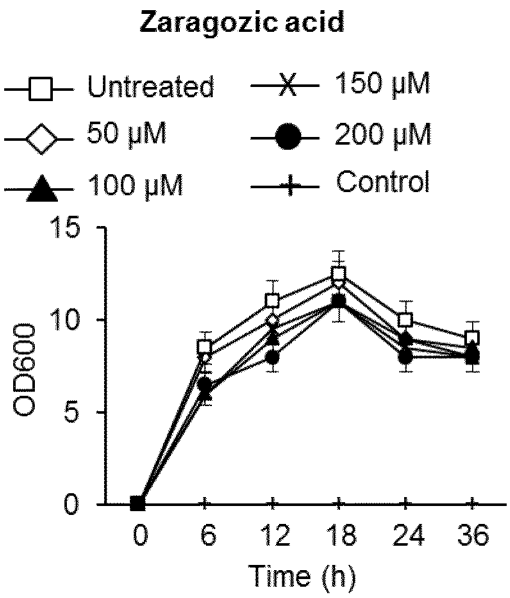
Zaragozic acid
Untreated
50 µM
100 µM
150 µM
200 µM
Control
OD600
15
10
5
0
0
6
12
18
24
36
Time (h)

B

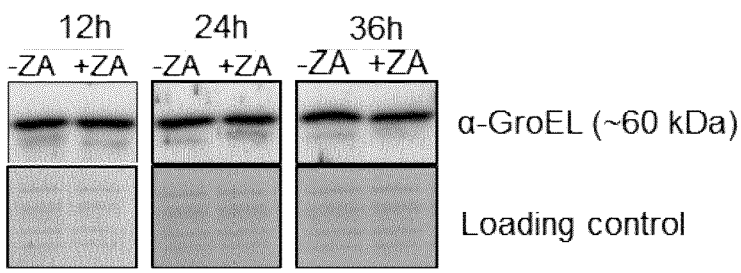
12h
24h
36h
-ZA
+ZA
-ZA
+ZA
-ZA
+ZA
α-GroEL (~60 kDa)
Loading control

Fig. 14 (Cont.)

C

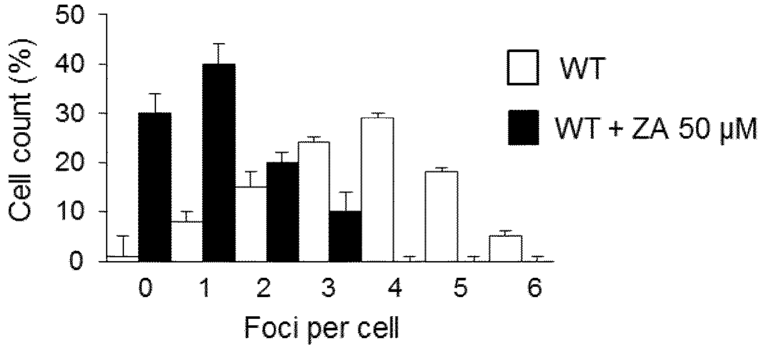


D

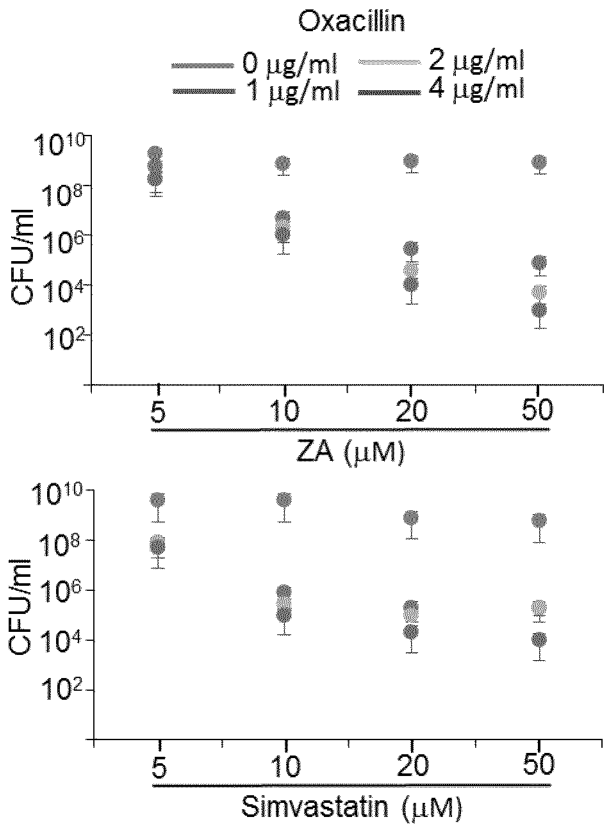

Fig. 14 (Cont.)

E

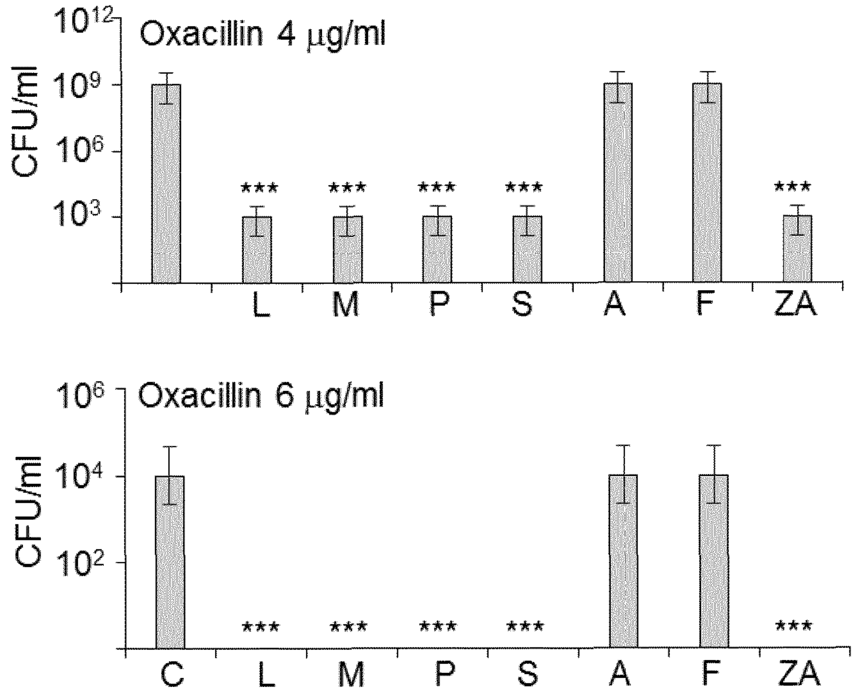


F

**MIC Oxacillin (Microdilution assay)**

| | |
|---|---|
| WT (USA300) | 32 - 48 μg/ml |
| MSSA (Newman) | 2,5 – 3,5 μg/ml |
| WT Δ*floA* (USA300) | 6,0 – 8,0 μg/ml |
| WT + ZA 50 μM (USA300) | 6,0 – 8,0 μg/ml |
| WT + Sim 50 μM (USA300) | 4,0 – 5,0 μg/ml |

Fig. 14 (Cont.)

G

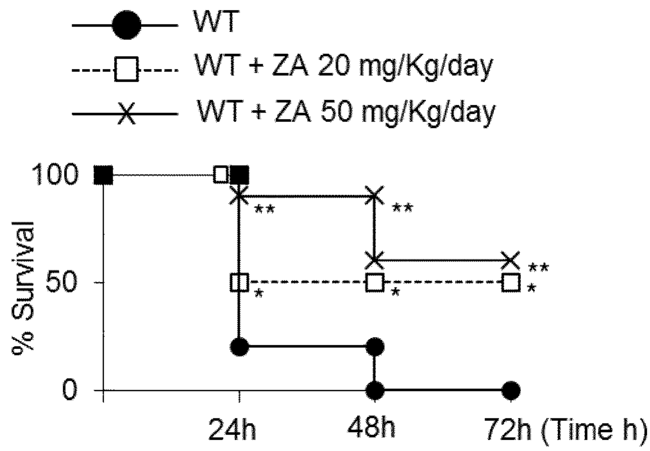
WT
WT + ZA 20 mg/Kg/day
WT + ZA 50 mg/Kg/day
100
50
0
% Survival
**
**
**
*
*
*
24h
48h
72h (Time h)

**Fig. 15**

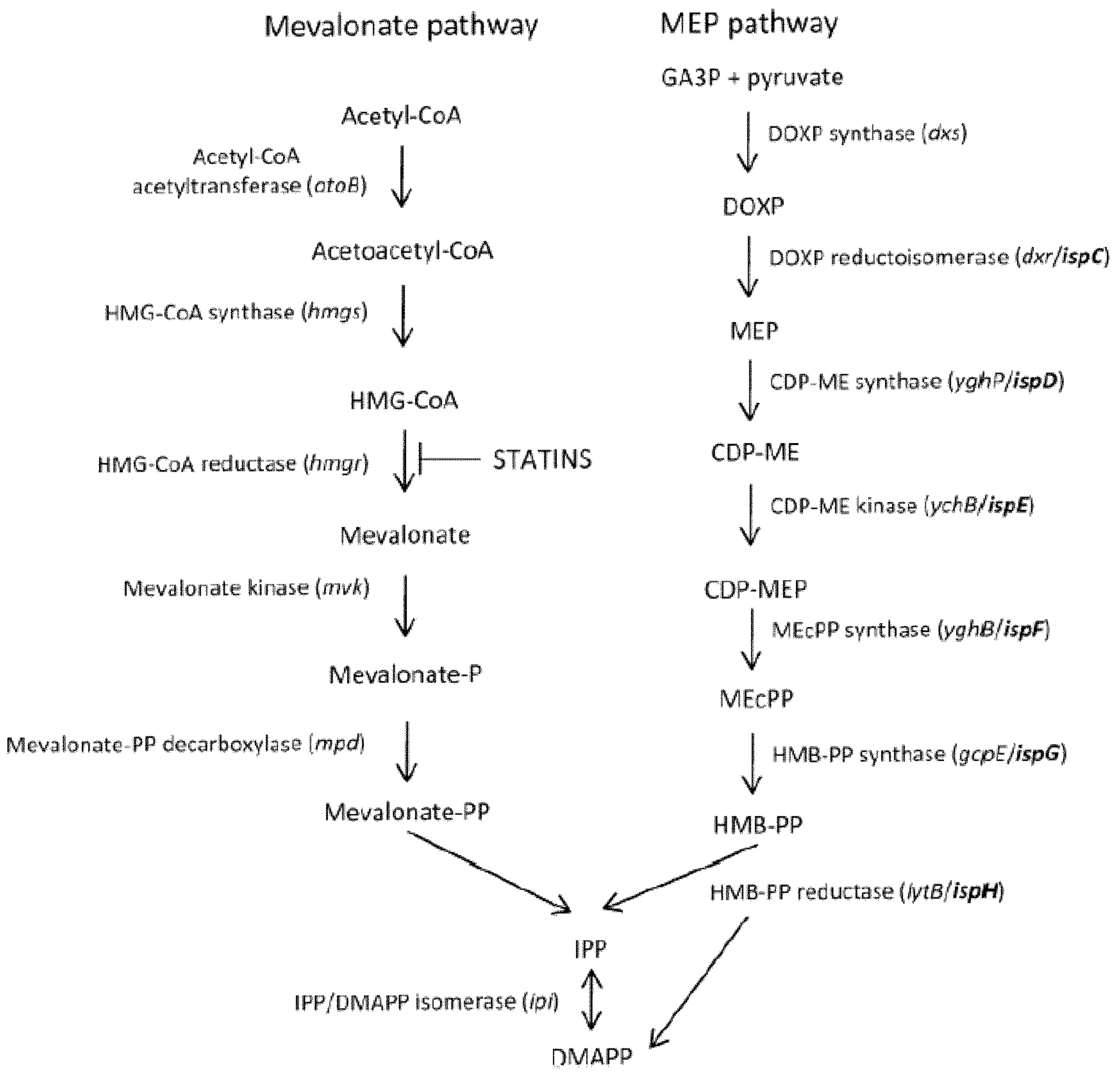
Mevalonate pathway
MEP pathway
GA3P + pyruvate
Acetyl-CoA
DOXP synthase (dxs)
Acetyl-CoA
acetyltransferase (atoB)
DOXP
Acetoacetyl-CoA
DOXP reductoisomerase (dxr/ispC)
HMG-CoA synthase (hmgs)
MEP
CDP-ME synthase (yghP/ispD)
HMG-CoA
HMG-CoA reductase (hmgr)
STATINS
CDP-ME
CDP-ME kinase (ychB/ispE)
Mevalonate
Mevalonate kinase (mvk)
CDP-MEP
MEcPP synthase (yghB/ispF)
Mevalonate-P
MEcPP
Mevalonate-PP decarboxylase (mpd)
HMB-PP synthase (gcpE/ispG)
Mevalonate-PP
HMB-PP
HMB-PP reductase (lytB/ispH)
IPP
IPP/DMAPP isomerase (ipi)
DMAPP

Fig. 16

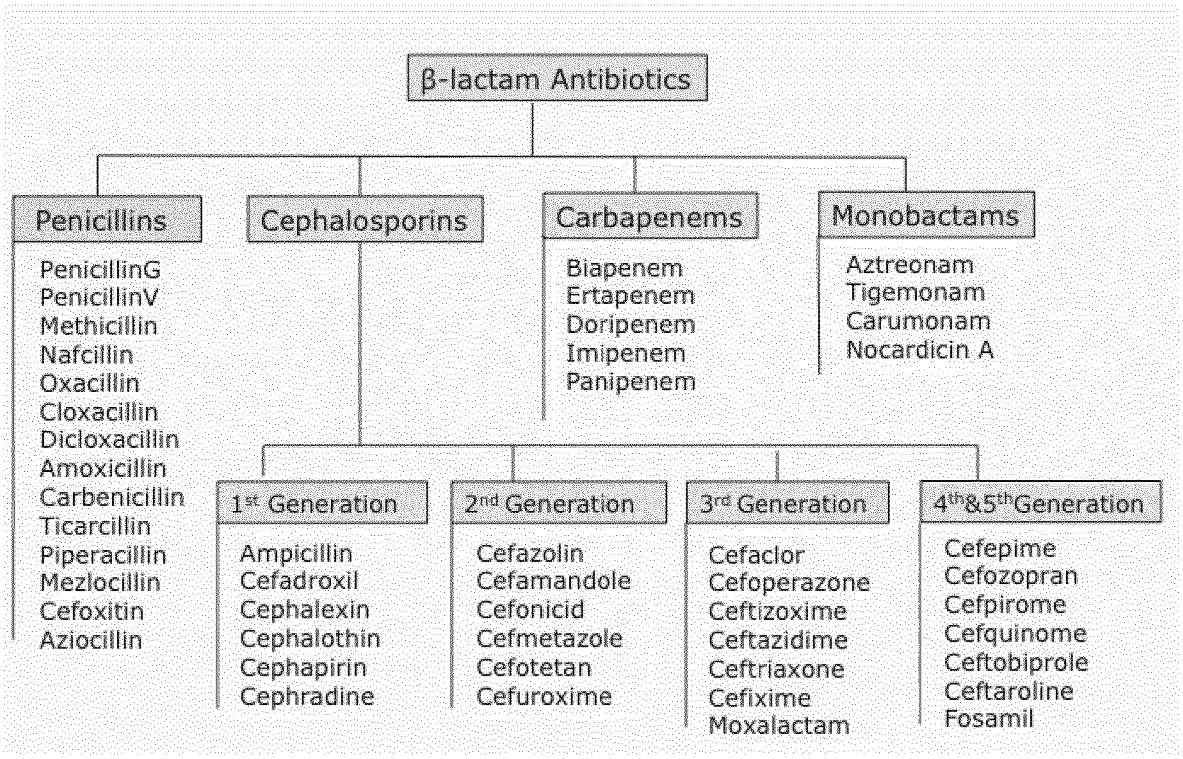
β-lactam Antibiotics
Penicillins
PenicillinG
PenicillinV
Methicillin
Nafcillin
Oxacillin
Cloxacillin
Dicloxacillin
Amoxicillin
Carbenicillin
Ticarcillin
Piperacillin
Mezlocillin
Cefoxitin
Aziocillin
Cephalosporins
Carbapenems
Biapenem
Ertapenem
Doripenem
Imipenem
Panipenem
Monobactams
Aztreonam
Tigemonam
Carumonam
Nocardicin A
1st Generation
Ampicillin
Cefadroxil
Cephalexin
Cephalothin
Cephapirin
Cephradine
2nd Generation
Cefazolin
Cefamandole
Cefonicid
Cefmetazole
Cefotetan
Cefuroxime
3rd Generation
Cefaclor
Cefoperazone
Ceftizoxime
Ceftazidime
Ceftriaxone
Cefixime
Moxalactam
4th&5thGeneration
Cefepime
Cefozopran
Cefpirome
Cefquinome
Ceftobiprole
Ceftaroline
Fosamil

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100173786 A
- WO 2012155019 A
- US 20110039287 A


### Non-patent literature cited in the description


- **SHANKAR THANGAMANI et al.** Exploring simvastatin, an antihyperlipidemic drug, as a potential topical antibacterial agent. *SCIENTIFIC REPORTS,* 10 November 2015, vol. 5 (1
- **AMBLER RP et al.** *Biochem J,* 1991, vol. 276, 269-70
- **BUSH K. ; JACOBY GA ; MEDEIROS AA.** *Antimicrob Agents Chemother,* 1995, vol. 39, 1211-33
- **DRAWZ S.M. ; BONOMO R.A.** *Clinical Microbiology Reviews,* 2010, vol. 23 (1), 160-201
- **M. RAUH.** *J Chromatogr B Analyt Technol Biomed Life Sci,* 01 February 2012, vol. 883-884, 59-67
- **LAIBLE G. ; HAKENBECK R.** *Journal of Bacteorology,* 1991, vol. 173 (21), 6986-6990
- **ZORZI et al.** *Journal of Bacteriology,* 1996, vol. 178 (16), 4948-4957
- **CHARLENE R. JACKSONETL.** *J. Clin. Microbiol.,* April 2013, vol. 51 (4), 1199-1207
- **SWENSON JM et al.** *J Clin Microbiol.,* 2009, vol. 47 (7), 2013-2017
- **JORGENSEN JH ; FERRARO MJ.** Antimicrobial susceptibility testing: a review of general principles and contemporary practices. *Clin Infect Dis,* 2009, vol. 49, 1749-55
- Encyclopedia of Pharmaceutical Technology. Marcel Dekker, Inc, 1988
- **ARNAUD, M. ; CHASTANET, A. ; DEBARBOUILLE, M.** New vector for efficient allelic replacement in naturally nontransformable, low-GC-content, gram-positive bacteria. *Appl Environ Microbiol,* 2004, vol. 70, 6887-6891
- **AYLIFFE, G.A.** Ampicillin inactivation and sensitivity of coliform bacilli. *J Gen Microbiol,* 1963, vol. 30, 339-348
- **BACH, J.N. ; BRAMKAMP, M.** Flotillins functionally organize the bacterial membrane. *Mol Microbiol,* 2013, vol. 88, 1205-1217
- **BACH, J.N. ; BRAMKAMP, M.** Dissecting the molecular properties of prokaryotic flotillins. *PLoS One,* 2015, vol. 10, e0116750
- **BERGMAN, P. ; LINDE, C. ; PUTSEP, K. ; POHANKA, A. ; NORMARK, S. ; HENRIQUES-NORMARK, B. ; ANDERSSON, J. ; BJORKHEM-BERGMAN, L.** Studies on the antibacterial effects of statins--in vitro and in vivo. *PLoS One,* 2011, vol. 6, e24394
- **BICKEL, P.E. ; SCHERER, P.E. ; SCHNITZER, J.E. ; OH, P. ; LISANTI, M.P. ; LODISH, H.F.** Flotillin and epidermal surface antigen define a new family of caveolae-associated integral membrane proteins. *J Biol Chem,* 1997, vol. 272, 13793-13802
- **BRAMKAMP, M. ; LOPEZ, D.** Exploring the Existence of Lipid Rafts in Bacteria. *Microbiol Mol Biol Rev,* 2015, vol. 79, 81-100
- Isolation and use of rafts. **BROWN, D.A.** Curr Protoc Immunol. 2002
- **BRYAN, S.J. ; BURROUGHS, N.J. ; EVERED, C. ; SACHARZ, J. ; NENNINGER, A. ; MULLINEAUX, C.W. ; SPENCE, E.M.** Loss of the SPHF homologue Slr1768 leads to a catastrophic failure in the maintenance of thylakoid membranes in Synechocystis sp. PCC 6803. *PLoS One,* 2011, vol. 6, e19625
- **CHO, H. ; WIVAGG, C.N. ; KAPOOR, M. ; BARRY, Z. ; ROHS, P.D. ; SUH, H. ; MARTO, J.A. ; GARNER, E.C. ; BERNHARDT, T.G.** Bacterial cell wall biogenesis is mediated by SEDS and PBP polymerase families functioning semi-autonomously. *Nat Microbiol,* 2016, 16172
- **DONOVAN, C. ; BRAMKAMP, M.** Characterization and subcellular localization of a bacterial flotillin homologue. *Microbiology,* 2009, vol. 155, 1786-1799
- **FALAGAS, M.E. ; MAKRIS, G.C. ; MATTHAIOU, D.K. ; RAFAILIDIS, P.I.** Statins for infection and sepsis: a systematic review of the clinical evidence. *J Antimicrob Chemother,* 2008, vol. 61, 774-785
- **FARMER, A. R. et al.** Effect of HMG-CoA reductase inhibitors on antimicrobial susceptibilities for Gram-Negative rods. *J. Basic Microbiol.,* 2013, vol. 53, 336-339

- **FENG, X. ; HU, Y. ; ZHENG, Y. ; ZHU, W. ; LI, K. ; HUANG, C.H. ; KO, T.P. ; REN, F. ; CHAN, H.C. ; NEGA, M. et al.** Structural and functional analysis of Bacillus subtilis YisP reveals a role of its product in biofilm production. *Chem Biol,* 2014, vol. 21, 1557-1563
- **FISHOVITZ, J. ; HERMOSO, J.A. ; CHANG, M. ; MOBASHERY, S.** Penicillin-binding protein 2a of methicillin-resistant Staphylococcus aureus. *IUBMB Life,* 2014, vol. 66, 572-577
- **GARCIA-LARA, J. ; WEIHS, F. ; MA, X. ; WALKER, L. ; CHAUDHURI, R.R. ; KASTURIARACHCHI, J. ; CROSSLEY, H. ; GOLESTANIAN, R. ; FOSTER, S.J.** Supramolecular structure in the membrane of Staphylococcus aureus. *Proc Natl Acad Sci U S A,* 2015, vol. 112, 15725-15730
- **GAUTAM, S. ; KIM, T. ; SPIEGEL, D.A.** Chemical probes reveal an extraseptal mode of cross-linking in Staphylococcus aureus. *J Am Chem Soc,* 2015, vol. 137, 7441-7447
- **GHODKE, R.M. ; TOUR, N. ; DEVI, K.** Effects of statins and cholesterol on memory functions in mice. *Metab Brain Dis,* 2012, vol. 27, 443-451
- **GOOD, M.C. ; ZALATAN, J.G. ; LIM, W.A.** Scaffold proteins: hubs for controlling the flow of cellular information. *Science,* 2011, vol. 332, 680-686
- **HAYAT, M.A.** Stains and cytochemical methods. Plenun Press, 1993
- **HEIMESAAT, M.M. ; LUGERT, R. ; FISCHER, A. ; ALUTIS, M. ; KUHL, A.A. ; ZAUTNER, A.E. ; TAREEN, A.M. ; HENNESSY, E. et al.** Is There Potential for Repurposing Statins as Novel Antimicrobials?. *Antimicrobial Agents and Chemotherapy,* 2016, vol. 60 (9), 5111-5121
- **HEUSTON, S. et al.** Isoprenoid biosynthesis in bacterial pathogens. *Microbiology,* 2012, vol. 158, 1389-1401
- **GOBEL, U.B. ; BERESWILL, S.** Impact of Campylobacter jejuni cj0268c knockout mutation on intestinal colonization, translocation, and induction of immunopathology in gnotobiotic IL-10 deficient mice. *PLoS One,* 2014, vol. 9, e90148
- **HELMPROBST, F. ; FRANK, M. ; STIGLOHER, C.** Presynaptic architecture of the larval zebrafish neuromuscular junction. *J Comp Neurol,* 2015, vol. 523, 1984-1997
- **HERTLEIN, T. ; STURM, V. ; LORENZ, U. ; SUMATHY, K. ; JAKOB, P. ; OHLSEN, K.** Bioluminescence and 19F magnetic resonance imaging visualize the efficacy of lysostaphin alone and in combination with oxacillin against Staphylococcus aureus in murine thigh and catheter-associated infection models. *Antimicrob Agents Chemother,* 2014, vol. 58, 1630-1638
- **HEUSTON, S. ; BEGLEY, M. ; GAHAN, C.G. ; HILL, C.** Isoprenoid biosynthesis in bacterial pathogens. *Microbiology,* 2012, vol. 158, 1389-1401
- **JOUSSELIN, A. ; MANZANO, C. ; BIETTE, A. ; REED, P. ; PINHO, M.G. ; ROSATO, A.E. ; KELLEY, W.L. ; RENZONI, A.** The Staphylococcus aureus Chaperone PrsA Is a New Auxiliary Factor of Oxacillin Resistance Affecting Penicillin-Binding Protein 2A. *Antimicrob Agents Chemother,* 2015, vol. 60, 1656-1666
- **JOUSSELIN, A. ; RENZONI, A. ; ANDREY, D.O. ; MONOD, A. ; LEW, D.P. ; KELLEY, W.L.** The post-translocational chaperone lipoprotein PrsA is involved in both glycopeptide and oxacillin resistance in Staphylococcus aureus. *Antimicrob Agents Chemother,* 2012, vol. 56, 3629-3640
- **KOCH, G. ; WERMSER, C. ; ACOSTA, I.C. ; KRICKS, L. ; STENGEL, S.T. ; YEPES, A. ; LOPEZ, D.** Attenuating Staphylococcus aureus Virulence by Targeting Flotillin Protein Scaffold Activity. *Cell Chem Biol,* 2017, vol. 24, 845-857
- **KRAFT, M.L.** Plasma membrane organization and function: moving past lipid rafts. *Mol Biol Cell,* 2013, vol. 24, 2765-2768
- **KREISWIRTH, B. ; KORNBLUM, J. ; ARBEIT, R.D. ; EISNER, W. ; MASLOW, J.N. ; MCGEER, A. ; LOW, D.E. ; NOVICK, R.P.** Evidence for a clonal origin of methicillin resistance in Staphylococcus aureus. *Science,* 1993, vol. 259, 227-230
- **KULLIK, I. ; GIACHINO, P. ; FUCHS, T.** Deletion of the alternative sigma factor sigmaB in Staphylococcus aureus reveals its function as a global regulator of virulence genes. *J Bacteriol,* 1998, vol. 180, 4814-4820
- **LAROCCA, T.J. ; PATHAK, P. ; CHIANTIA, S. ; TOLEDO, A. ; SILVIUS, J.R. ; BENACH, J.L. ; LONDON, E.** Proving lipid rafts exist: membrane domains in the prokaryote Borrelia burgdorferi have the same properties as eukaryotic lipid rafts. *PLoS Pathog,* 2013, vol. 9, e1003353
- **LESKI, T.A. ; TOMASZ, A.** Role of penicillin-binding protein 2 (PBP2) in the antibiotic susceptibility and cell wall cross-linking of Staphylococcus aureus: evidence for the cooperative functioning of PBP2, PBP4, and PBP2A. *J Bacteriol,* 2005, vol. 187, 1815-1824
- **LIAPPIS, A.P. ; KAN, V.L. ; ROCHESTER, C.G. ; SIMON, G.L.** The effect of statins on mortality in patients with bacteremia. *Clin Infect Dis,* 2001, vol. 33, 1352-1357
- **LIU, C.I. ; LIU, G.Y. ; SONG, Y. ; YIN, F. ; HENSLER, M.E. ; JENG, W.Y. ; NIZET, V. ; WANG, A.H. ; OLDFIELD, E.** A cholesterol biosynthesis inhibitor blocks Staphylococcus aureus virulence. *Science,* 2008, vol. 319, 1391-1394
- **LOPEZ, D. ; KOLTER, R.** Functional microdomains in bacterial membranes. *Genes Dev,* 2010, vol. 24, 1893-1902

- **LOPEZ-COLLAZO, E. ; JURADO, T. ; DE DIOS CABALLERO ; J., PEREZ-VAZQUEZ, M. ; VINDEL, A. ; HERNANDEZ-JIMENEZ, E. ; TAMAMES, J. ; CUBILLOS-ZAPATA, C. ; MANRIQUE, M. ; TOBES, R. et al.** In vivo attenuation and genetic evolution of a ST247-SCCmecI MRSA clone after 13 years of pathogenic bronchopulmonary colonization in a patient with cystic fibrosis: implications of the innate immune response. *Mucosal Immunol,* 2015, vol. 8, 362-371
- **LOPEZ-CORTES, L.E. ; GALVEZ-ACEBAL, J. ; DEL TORO, M.D. ; VELASCO, C. ; DE CUETO, M. ; CABALLERO, F.J. ; MUNIAIN, M.A. ; PASCUAL, A. ; RODRIGUEZ-BANO, J.** Effect of statin therapy in the outcome of bloodstream infections due to Staphylococcus aureus: a prospective cohort study. *PLoS One,* 2013, vol. 8, e82958
- **LORENT, J.H. ; LEVENTAL, I.** Structural determinants of protein partitioning into ordered membrane domains and lipid rafts. *Chem Phys Lipids,* 2015, vol. 192, 23-32
- **MARKERT, S.M. ; BRITZ, S. ; PROPPERT, S. ; LANG, M. ; WITVLIET, D. ; MULCAHY, B. ; SAUER, M. ; ZHEN, M. ; BESSEREAU, J.L. ; STIGLOHER, C.** Filling the gap: adding super-resolution to array tomography for correlated ultrastructural and molecular identification of electrical synapses at the C. elegans connectome. *Neurophotonics,* 2016, vol. 3, 041802
- **MARSHALL, J.H. ; WILMOTH, G.J.** Proposed pathway of triterpenoid carotenoid biosynthesis in Staphylococcus aureus: evidence from a study of mutants. *J Bacteriol,* 1981, vol. 147, 914-919
- **MARTOS, A. ; RASO, A. ; JIMENEZ, M. ; PETRASEK, Z. ; RIVAS, G. ; SCHWILLE, P.** FtsZ Polymers Tethered to the Membrane by ZipA Are Susceptible to Spatial Regulation by Min Waves. *Biophys J,* 2015, vol. 108, 2371-2383
- **MCDOUGAL, L.K. ; STEWARD, C.D. ; KILLGORE, G.E. ; CHAITRAM, J.M. ; MCALLISTER, S.K. ; TENOVER, F.C.** Pulsed-field gel electrophoresis typing of oxacillin-resistant Staphylococcus aureus isolates from the United States: establishing a national database. *J Clin Microbiol,* 2003, vol. 41, 5113-5120
- **MCQUILLEN, K.** Bacterial Ribosomes and Protein Synthesis. *J Gen Microbiol,* 1962, vol. 29, 53-57
- **MONTEIRO, J.M. ; FERNANDES, P.B. ; VAZ, F. ; PEREIRA, A.R. ; TAVARES, A.C. ; FERREIRA, M.T. ; PEREIRA, P.M. ; VEIGA, H. ; KURU, E. ; VANNIEUWENHZE, M.S. et al.** Cell shape dynamics during the staphylococcal cell cycle. *Nat Commun,* 2015, vol. 6, 8055
- **NICKELS, J.D. ; CHATTERJEE, S. ; STANLEY, C.B. ; QIAN, S. ; CHENG, X. ; MYLES, D.A.A. ; STANDAERT, R.F. ; ELKINS, J.G. ; KATSARAS, J.** The in vivo structure of biological membranes and evidence for lipid domains. *PLoS Biol,* 2017, vol. 15, e2002214
- **OTERO, L.H. ; ROJAS-ALTUVE, A. ; LLARRULL, L.I. ; CARRASCO-LOPEZ, C. ; KUMARASIRI, M. ; LASTOCHKIN, E. ; FISHOVITZ, J. ; DAWLEY, M. ; HESEK, D. ; LEE, M. et al.** How allosteric control of Staphylococcus aureus penicillin binding protein 2a enables methicillin resistance and physiological function. *Proc Natl Acad Sci U S A,* 2013, vol. 110, 16808-16813
- **PALADE, G.E.** A small particulate component of the cytoplasm. *J Biophys Biochem Cytol,* 1955, vol. 1, 59-68
- **PARIHAR, S.P. ; GULER, R. ; KHUTLANG, R. ; LANG, D.M. ; HURDAYAL, R. ; MHLANGA, M.M. ; SUZUKI, H. ; MARAIS, A.D. ; BROMBACHER, F.** Statin therapy reduces the Mycobacterium tuberculosis burden in human macrophages and in mice by enhancing autophagy and phagosome maturation. *J Infect Dis,* 2014, vol. 209, 754-763
- **PEACOCK, S.J. ; PATERSON, G.K.** Mechanisms of Methicillin Resistance in Staphylococcus aureus. *Annu Rev Biochem,* 2015, vol. 84, 577-601
- **PELZ, A. ; WIELAND, K.P. ; PUTZBACH, K. ; HENTSCHEL, P. ; ALBERT, K. ; GOTZ, F.** Structure and biosynthesis of staphyloxanthin from Staphylococcus aureus. *J Biol Chem,* 2005, vol. 280, 32493-32498
- **PINHO, M.G. ; DE LENCASTRE, H. ; TOMASZ, A.** An acquired and a native penicillin-binding protein cooperate in building the cell wall of drug-resistant staphylococci. *Proc Natl Acad Sci U S A,* 2001, vol. 98, 10886-10891
- **PINHO, M.G. ; FILIPE, S.R. ; DE LENCASTRE, H. ; TOMASZ, A.** Complementation of the essential peptidoglycan transpeptidase function of penicillin-binding protein 2 (PBP2) by the drug resistance protein PBP2A in Staphylococcus aureus. *J Bacteriol,* 2001, vol. 183, 6525-6531
- **ROCK, P. ; ALLIETTA, M. ; YOUNG, W.W., JR. ; THOMPSON, T.E. ; TILLACK, T.W.** Organization of glycosphingolipids in phosphatidylcholine bilayers: use of antibody molecules and Fab fragments as morphologic markers. *Biochemistry,* 1990, vol. 29, 8484-8490
- **SCHNEIDER, J. ; KLEIN, T. ; MIELICH-SUSS, B. ; KOCH, G. ; FRANKE, C. ; KUIPERS, O.P. ; KOVACS, A.T. ; SAUER, M. ; LOPEZ, D.** Spatio-temporal remodeling of functional membrane microdomains organizes the signaling networks of a bacterium. *PLoS Genet,* 2015, vol. 11, e1005140

- **SCHNEIDER, J. ; YEPES, A. ; GARCIA-BETANCUR, J.C. ; WESTEDT, I. ; MIELICH, B. ; LOPEZ, D.** Streptomycin-induced expression in Bacillus subtilis of YtnP, a lactonase-homologous protein that inhibits development and streptomycin production in Streptomyces griseus. *Appl Environ Microbiol,* 2012, vol. 78, 599-603
- **SILVA-ROCHA, R. ; MARTINEZ-GARCIA, E. ; CALLES, B. ; CHAVARRIA, M. ; ARCE-RODRIGUEZ, A. ; DE LAS HERAS, A. ; PAEZ-ESPINO, A.D. ; DURANTE-RODRIGUEZ, G. ; KIM, J. ; NIKEL, P.I. et al.** The Standard European Vector Architecture (SEVA): a coherent platform for the analysis and deployment of complex prokaryotic phenotypes. *Nucleic Acids Res,* 2013, vol. 41, D666-675
- **SIMONS, K. ; IKONEN, E.** Functional rafts in cell membranes. *Nature,* 1997, vol. 387, 569-572
- **SIMONS, K. ; TOOMRE, D.** Lipid rafts and signal transduction. *Nat Rev Mol Cell Biol,* 2000, vol. 1, 31-39
- **SINGER, S.J. ; NICOLSON, G.L.** The fluid mosaic model of the structure of cell membranes. *Science,* 1972, vol. 175, 720-731
- **SOMANI, V.K. ; AGGARWAL, S. ; SINGH, D. ; PRASAD, T. ; BHATNAGAR, R.** Identification of Novel Raft Marker Protein, FlotP in Bacillus anthracis. *Front Microbiol,* 2016, vol. 7, 169
- **TAREEN, A.M. ; LUDER, C.G. ; ZAUTNER, A.E. ; GROBETA, U. ; HEIMESAAT, M.M. ; BERESWILL, S. ; LUGERT, R.** The Campylobacter jejuni Cj0268c protein is required for adhesion and invasion in vitro. *PLoS One,* 2013, vol. 8, e81069
- **THANGAMANI , S. et al.** Exploring simvastatin, an antihyperlipidemic drug, as a potential topical antibacterial agent. *Scientific reports,* 2015, vol. 5, 16407
- **THOMPSON, T.E. ; TILLACK, T.W.** Organization of glycosphingolipids in bilayers and plasma membranes of mammalian cells. *Annu Rev Biophys Biophys Chem,* 1985, vol. 14, 361-386
- **TING-BEALL, H.P.** Interactions of uranyl ions with lipid bilayer membranes. *J Microsc,* 1980, vol. 118, 221-227
- **WAN, Y.D. ; SUN, T.W. ; KAN, Q.C. ; GUAN, F.X. ; ZHANG, S.G.** Effect of statin therapy on mortality from infection and sepsis: a meta-analysis of randomized and observational studies. *Crit Care,* 2014, vol. 18, R71
- **WIELAND, B. ; FEIL, C. ; GLORIA-MAERCKER, E. ; THUMM, G. ; LECHNER, M. ; BRAVO, J.M. ; PORALLA, K. ; GOTZ, F.** Genetic and biochemical analyses of the biosynthesis of the yellow carotenoid 4,4'-diaponeurosporene of Staphylococcus aureus. *J Bacteriol,* 1994, vol. 176, 7719-7726
- **WITTIG, I. ; BRAUN, H.P. ; SCHAGGER, H.** Blue native PAGE. *Nat Protoc,* 2006, vol. 1, 418-428
- **YEPES, A. ; KOCH, G. ; WALDVOGEL, A. ; GARCIA-BETANCUR, J.C. ; LOPEZ, D.** Reconstruction of mreB expression in Staphylococcus aureus via a collection of new integrative plasmids. *Appl Environ Microbiol,* 2014, vol. 80, 3868-3878
- **ZAPUN, A. ; CONTRERAS-MARTEL, C. ; VERNET, T.** Penicillin-binding proteins and betalactam resistance. *FEMS Microbiol Rev,* 2008, vol. 32, 361-385